# EUROPEAN PATENT SPECIFICATION

(11) **EP 0 535 250 B1**
(45) Date of publication and mention of the grant of the patent: **19.06.1996**
(21) Application number: 92908493.7
(22) Date of filing: 10.04.1992
(51) Int. Cl.: C07C 233/40, C07C 49/563, C07C 49/573, C07C 49/577, C07C 59/64, C07C 62/38, C07C 69/157, C07C 69/618, C07C 69/734, C07C 69/757, C07C 235/36

(54) **NOVEL COMPOUNDS AND USE THEREOF AS MEDICINE**
NEUE VERBINDUNGEN UND DEREN MEDIZINALE VERWENDUNG
NOUVEAUX COMPOSES ET LEUR UTILISATION MEDICINALE

(30) Priority: 10.04.1991 JP 103558/91; 27.02.1992 JP 75678/92
(43) Date of publication of application: 07.04.1993
(60) Divisional application: 95203104.5
(73) Proprietor: TSUMURA & CO., Chuo-ku, Tokyo 103 (JP)
(72) Inventor: KODA, Akihide, Gifu-shi Gifu-ken 502 (JP); WARAGAI, Koji, Inashiki-gun Ibaraki-ken 300-11 (JP); ONO, Yutaka, Inashiki-gun Ibaraki-ken 300-11 (JP); OZAWA, Hideyuki, Chiyoda-ku Tokyo 102 (JP); KAWAMURA, Hideki, Inashiki-gun Ibaraki-ken 300-11 (JP); MARUNO, Masao, Inashiki-gun Ibaraki-ken 300-11 (JP); WAKAMATSU, Takeshi, Inashiki-gun Ibaraki-ken 300-11 (JP)
(74) Representative: Wain, Christopher Paul
(86) International application number: PCT/JP92/00451
(87) International publication number: WO 92/18463

(56) References cited:
- EP-A- 0 495 998
- FR-A- 2 518 992
- JP-A- 3 086 853
- JP-A-55 130 949
- JP-A-60 214 766
- US-A- 2 487 179

## Description

### Technical Field

The present invention relates to a novel compound which has IV-type allergic reaction-suppressive function and which is useful as a medicine such as IV-type allergy suppressive drug.

### Background Art

It has been reported that IV-type allergic reaction is greatly responsible for the pathopoiesis of refractory diseases such as refractory asthma, chronic hepatitis and nephrotic syndrome. To cure these refractory diseases, immunosuppressive agents, a typical example of which is a steroid drug, are used. However, the use of the immunosuppressive agents is limited, inevitably because of their strong side effects.

### Disclosure of Invention

Therefore it has been demanded that a IV-type allergic reaction-suppressive agent be developed in place of the immunosuppressive agents hitherto known.

In order to meet the demand described above, the inventors hereof have been making research and study, in search for a substance which works against IV-type allergic reaction in its effector phase. They successfully synthesized the compounds represented by formulae I and II and examined these compounds for their pharmacological activities to find that these compounds have desired IV-type allergic reaction-suppressive function, thus completing the present invention.

The present invention provides the compounds represented by formulae I and II or pharmaceutically acceptable salts thereof, and IV-type allergic reaction-suppressive agents containing, as effective components, the compounds represented by formulae I and II, or pharmaceutically acceptable salts thereof.

Further compounds which have been found to have IV-type allergic reaction-suppressive function are described in European Patent Application No. 95203104.5, which has been divided from this application. 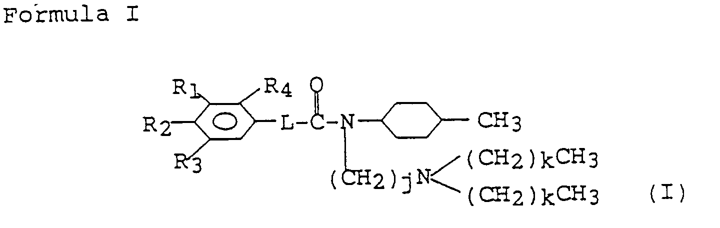
where L represents -(CH=CH)ₖ- or -(CH₂)ₖ-; j represents an integer of 1 to 6; k represents represents an integer of 0 to 5; R₁, R₂, R₃ and R₄ are either identical or different, and each represents a hydrogen atom, an amino group, an acylamino group (preferably, one in which the acyl moiety has 1 to 3 carbon atoms), a dialkylamino group in which each alkyl moiety has 1 to 5 carbon atoms, a halogen atom, a hydroxyl group, an acetoxy group, an alkoxy group having 1 to 3 carbon atoms, a trifluoromethyl group, a nitro group, tetrahydropyranyloxy group, or a benzyloxy group; and R₁ and R₂ may combine together to form a methylenedioxy group. 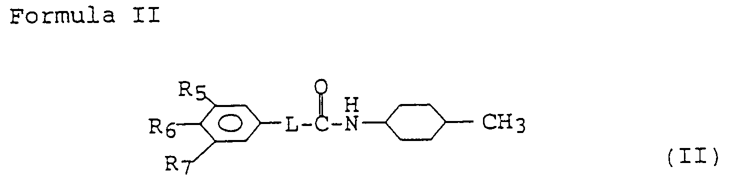
where L represents -(CH=CH)ₖ- or -(CH₂)ₖ-; each k represents an integer of 0 to 5; R₅, R₆ and R₇ are either identical or different, and each represents a hydrogen atom, a hydroxyl group, an acetoxy group, an amino acid ester group in which the amino moiety may be protected by a protective group, an alkoxy group having 1 to 3 carbon atoms, or -O-(CH₂)ⱼ-Y₁; R₅ and R₆ may combine together to form a methylenedioxy group. j represents an integer of 1 to 6, and Y₁ is a halogen atom, an amino group, an amino group substituted with 1 or 2 thioethanol groups, an amino group substituted with 1 or 2 allyl groups, a monoalkylamino group having 1 to 10 carbon atoms, a dialylamino group in which each alkyl moiety has 1 to 10 carbon atoms, an imidazole group substituted with 1 to 3 substituent groups (e.g., a nitro group, a cyano group, a phenyl group, an alkoxy group having 1 to 3 carbon atoms, a halogen atom, or an alkyl group having 1 to 3 carbon atoms), a benzimidazole group substituted with 1 to 3 substituent groups (e.g., a nitro group, a cyano group, a phenyl group, an alkoxy group having 1 to 3 carbon atoms, a halogen atom, or an alkyl group having 1 to 3 carbon atoms), a diazole group, a triazole group, a tetrazole group, a piperidine group, a piperidinopiperidine group, an isonipecotic acid group or an ester thereof with an alkyl group having 1 to 5 carbon atoms, a thiazolidine group, a pyrrolidine group, a morpholine group, a 4-methylpiperazine group, a carboxyl group (-COOH), a carboxylic acid alkylester group in which the alkyl moiety has 1 to 5 carbon atoms (-COO-C₁ to C₅ alkyl) or a carboxamide group.

In the formula (II), the amino acid ester groups represented by R₅, R₆, and/or R₇ are groups formed by removing hydrogen from the carboxyl group of an amino acid. Examples of the amino acid are glycine, alanine, leucine, and proline. Examples of the protective group for the amino group of the amino acid ester group are acetyl group, benzyloxycarbonyl group, and t-butoxycarbonyl group.

Also in the formula (II), the carboxylic carboxamide group represented by Y₁ is a group which is formed by reaction of a carboxylic acid group (-COOH) with a primary or secondary amine, HN(R')R'', and which can be represented by -COON(R')R'', where R' and R'' are either identical or different, and each represents, e.g., a hydrogen atom, a piperidinopiperidine group, or 4-methylpiperazine group.

Hereinafter, the compounds represented by formulae I and II shall be referred to as the "compounds of the present invention."

### Best Mode of Carrying Out the Invention

The compounds of the present invention can be obtained by performing the following reactions 1 to 9, in an appropriate combination, on the compounds known hitherto:
1. Alkylation
2. Reduction
3. Esterification and amidation
4. Halogenation
5. Hydrolysis
6. Conversion into imine or oxime
7. Knoevenagel condensation
8. Amination
9. Conversion into non-toxic salts
The reactions mentioned above may be carried out in ordinary manners. These reactions will be each explained below.
1. Alkylation is accomplished by using a base such as sodium carbonate, potassium carbonate, sodium hydride, lithium diisopropylamide (LDA), sodium hexamethyldisilazide, sodium hydroxide, or the like, in an amount equal to or greater than the equivalent amount of the substrate used, thereby generating anions, and by reacting the anions with one equivalent amount or more of alkyl halide or allyl halide.
   As a solvent, use may be made of at least one selected from a hydrocarbon, methylene chloride, chloroform, carbon tetrachloride, ether, tetrahydrofuran (THF), DMF, dimethylsulfoxide (DMSO), a lower alcohol, and water. The reaction may be performed at a temperature of -78°C to 150°C, in accordance with the reagent used.
2. Reduction may be achieved by: (i) a reduction by an alkali metal such as sodium or lithium, an alkaline earth metal such as calcium or magnesium, a metal such as aluminum, zinc, iron or copper, or a salt of any one of these metals is used as a reducing agent; (ii) a reduction by a metal hydride such as diisobutylaluminum hydride, organic tin hydride or hydrosilane, a metal hydrogen complex compound such as lithium aluminum hydride or sodium borohydride, or a complex thereof with borane or diborane; or (iii) a reduction in which a metal, such as nickel, cobalt, palladium, rhodium, copper or platinum, or an oxide, chloride or salt of any of these metals is used as a catalyst.
   In the reduction methods (i), (ii), and (iii), the reaction may be effected with the reducing agent in an amount equal to or greater than an equivalent amount of the substrate, or the metal or the oxide, chloride or salt thereof in a catalytic amount, in at least one solvent selected from water and an organic solvent such as a lower alcohol, acetic acid, hydrochloric acid, ammonia, a primary, secondary or tertiary amine, a hydrocarbon, methylene chloride, chloroform, ether, and THF, under air an inert gas or hydrogen gas, at an atmospheric pressure or an elevated pressure, and at a temperature of -78°C to 150°C in accordance with the reagent used.
   In the reduction method (iii), a salt of formic acid, for example, can be used as a hydrogen source.
3. Esterification and amidation may be performed by reacting sulfonic acid, carboxylic acid, or an acid chloride or acid anhydride thereof with a compound having a hydroxyl group, such as a phenol or an alcoholic compound, or having primary or secondary amine, at a temperature of -78°C to 150°C, using, as a solvent, a hydrocarbon, methylene chloride, chloroform, carbon tetrachloride, ether, tetrahydrofuran (THF), DMF, dimethylsulfoxide (DMSO), a lower alcohol, water, or an amine such as pyridine.
   If necessary, use can be made of a condensating agent such as diethyl chlorophosphate, diethyl cyanophosphate or dicyclohexylcarbodimide, and a catalytic amount of dimethylaminopyridine. Also, if necessary, use can be made of the dehydrating agent described above, the dehydrating apparatus, an acid catalyst, and a deacidifyer such as a tertiary amine.
4. Halogenation may be accomplished by reacting a sulfonic acid ester with a halogen or a salt thereof at -78°C to 150°C in the solvent specified above.
5. Hydrolysis may be performed by reacting an acetal, a lactone or an ester with an acid such as hydrochloric acid, sulfuric acid or p-toluenesulfonic acid, an alkali such as sodium hydroxide or potassium carbonate, or boron halide such as boron tribromide, in the above-mentioned solvent at a temperature of -78°C to 150°C.
6. Conversion into imine or oxime may be effected by reacting a carbonyl compound with a primary amine, a hydroxyamine or a salt thereof, in one of the above-mentioned ordinary solvents or a mixture of the ordinary solvents.
7. Knoevenagel condensation may be achieved by reacting a carbonyl compound with an equivalent amount or more of a reactive methylene compound such as malonic acid, cyanoacetic acid or an ester thereof, in one of the above-specified ordinary solvents, a mixture thereof or a tertiary amine at 0°C to 150°C. The reaction can be performed by using, if necessary, a catalytic amount of a primary or secondary amine, or the dehydrating agent specified above, or the apparatus for removing the water formed.
8. Amination may be performed by reacting an alkyl halide with ammonia or a primary or secondary amine such as dimethylamine or piperidine. If necessary, use may be made of a base such as sodium hydride.
   The ordinary solvents specified above or water may be used singly or in combination as a solvent. The reaction temperature may be 0°C to 150°C.
9. Conversion into non-toxic salts can be accomplished by reacting a compound having amine, alcohol, phenol or carboxylic acid, with an amino acid, potassium hydride, hydrogen chloride, hydrogen sulfide, phosphoric acid, maleic acid, fumaric acid, succinic acid, tartaric acid, salicylic acis, p-toluenesulfonic acid, citric acid, sodium methoxide, or a primary, secondary or tertiary amine such as ammonium, trishydroxymethylaminomethane or N-methyldiethanolamine, in one of the ordinary solvents specified above, water, an alcohol, or a mixture thereof, at a temperature of 0°C to 150°C

In this case, the salt formed must be pharmaceutically acceptable.

One skilled in the art will understand the application of these reactions and the details thereof, from the Examples of the invention which shall be described later.

Next, the experiments will be described to show that the compounds of the present invention has IV-type allergic reaction-suppressive function.

### Experiments

### 1) Procedure of the Experiments

Reaction in the sole of the foot, caused by sheep's red blood cells (hereinafter referred to as "SRBC") was used as model of IV-type allergic reaction. To be specific, SRBC was adjusted to 2.5 × 10⁶/mℓ with physiological saline, and 0.2 mℓ thereof was injected into the tail veins of BALB/c mice (7-week old, male) and thus sensitized. Four days later, SRBC of the same lot was adjusted to 4 × 10⁹/mℓ, and 0.025 mℓ thereof was injected into the skin of the right-foot soles, thereby provoking the reaction. The volume of the right-foot sole of each mouse, measured prior to the provocation of the reaction, was subtracted from the volume of the right-foot sole measured 24 hours after the provocation of the reaction, thus obtaining a difference. From this difference, the degree of the IV-type allergic reaction was determined.

The compounds obtained in the Examples were dissolved in purified water or distilled water, and each was orally administered to a group of mice twice, first immediately before the provocation of the reaction, and then 16 hours thereafter.

The administered amount of the compounds obtained in the Examples was 3.37 × 10⁻⁴ mol/kg in Experiments 1 to 8, and 3.46 × 10⁻⁴ mol/kg in Experiments 9 to 21.

The results are shown in the following tables, in terms of suppression rate (%) in comparison with a control.

**Table 1**

| | Suppression Rate (%) | Number of Mice |
|---|---|---|
| Control | | 7 |
| Sodium salt obtained in Example 4 | 40.0 | 7 |

**Table 2**

| | Suppression Rate (%) | Number of Mice |
|---|---|---|
| Control | | 7 |
| Sodium salt obtained in Example 4 | 46.2 | 7 |

**Table 3**

| | Suppression Rate (%) | Number of Mice |
|---|---|---|
| Control | | 10 |
| Sodium salt obtained in Example 41 | 69.6 | 9 |
| Sodium salt obtained in Example 65 | 67.5 | 8 |

**Table 4**

| | Suppression Rate (%) | Number of Mice |
|---|---|---|
| Control | | 9 |
| Sodium salt obtained in Example 41 | 36.2 | 9 |

**Table 5**

| | Suppression Rate (%) | Number of Mice |
|---|---|---|
| Control | | 8 |
| Hydrochloride salt obtained in Example 43 | 77.8 | 7 |
| Hydrochloride salt obtained in Example 73 | 60.0 | 8 |

**Table 6**

| | Suppression Rate (%) | Number of Mice |
|---|---|---|
| Control | | 8 |
| Hydrochloride salt obtained in Example 48 | 45.3 | 8 |
| Hydrochloride salt obtained in Example 16 | 47.6 | 8 |
| Hydrochloride salt obtained in Example 44 | 22.4 | 8 |
| Hydrochloride salt obtained in Example 91 | 69.6 | 8 |

**Table 7**

| | Suppression Rate (%) | Number of Mice |
|---|---|---|
| Control | | 8 |
| Hydrochloride salt obtained in Example 145 | 28.6 | 8 |
| Hydrochloride salt obtained in Example 48 | 74.9 | 8 |
| Hydrochloride salt obtained in Example 16 | 60.9 | 8 |
| Hydrochloride salt obtained in Example 44 | 77.8 | 7 |
| Hydrochloride salt obtained in Example 91 | 72.5 | 8 |

**Table 8**

| | Suppression Rate (%) | Number of Mice |
|---|---|---|
| Control | | 8 |
| Hydrochloride salt obtained in Example 43 | 65.3 | 8 |
| Hydrochloride salt obtained in Example 73 | 82.9 | 7 |
| Hydrochloride salt obtained in Example 48 | 80.3 | 8 |
| Hydrochloride salt obtained in Example 16 | 82.4 | 7 |
| Hydrochloride salt obtained in Example 44 | 68.7 | 7 |

**Table 9**

| | Suppression Rate (%) | Number of Mice |
|---|---|---|
| Control | | 7 |
| Hydrochloride salt obtained in Example 17 | 81.9 | 5 |

**Table 10**

| | Suppression Rate (%) | Number of Mice |
|---|---|---|
| Control | | 9 |
| Hydrochloride salt obtained in Example 17 | 97.9 | 4 |
| Hydrochloride salt obtained in Example 31 | 54.1 | 5 |

**Table 11**

| | Suppression Rate (%) | Number of Mice |
|---|---|---|
| Control | | 8 |
| Hydrochloride salt obtained in Example 22 | 31.9 | 7 |
| Hydrochloride salt obtained in Example 24 | 51.5 | 7 |
| Hydrochloride salt obtained in Example 23 | 27.8 | 8 |
| Hydrochloride salt obtained in Example 25 | 22.7 | 8 |
| Hydrochloride salt obtained in Example 26 | 36.7 | 7 |

**Table 12**

| | Suppression Rate (%) | Number of Mice |
|---|---|---|
| Control | | 8 |
| Hydrochloride salt obtained in Example 76 | 42.4 | 8 |
| Hydrochloride salt obtained in Example 77 | 52.5 | 8 |
| Hydrochloride salt obtained in Example 79 | 66.2 | 8 |
| Hydrochloride salt obtained in Example 80 | 72.8 | 8 |
| Hydrochloride salt obtained in Example 86 | 20.5 | 5 |
| Hydrochloride salt obtained in Example 88 | 55.8 | 7 |
| Hydrochloride salt obtained in Example 90 | 54.4 | 7 |

**Table 13**

| | Suppression Rate (%) | Number of Mice |
|---|---|---|
| Control | | 7 |
| Hydrochloride salt obtained in Example 78 | 82.7 | 7 |
| Hydrochloride salt obtained in Example 71 | 61.9 | 6 |
| Hydrochloride salt obtained in Example 70 | 54.0 | 7 |
| Hydrochloride salt obtained in Example 69 | 78.6 | 7 |
| Hydrochloride salt obtained in Example 89 | 47.9 | 7 |
| Hydrochloride salt obtained in Example 84 | 37.3 | 7 |
| Hydrochloride salt obtained in Example 83 | 75.7 | 7 |
| Hydrochloride salt obtained in Example 18 | 75.2 | 7 |

**Table 14**

| | Suppression Rate (%) | Number of Mice |
|---|---|---|
| Control | | 7 |
| Hydrochloride salt obtained in Example 11 | 53.3 | 7 |
| Hydrochloride salt obtained in Example 74 | 61.2 | 5 |
| Hydrochloride salt obtained in Example 85 | 66.2 | 7 |
| Hydrochloride salt obtained in Example 28 | 88.7 | 6 |
| Hydrochloride salt obtained in Example 29 | 41.8 | 6 |
| Hydrochloride salt obtained in Example 28 | 50.2 | 7 |

**Table 15**

| | Suppression Rate (%) | Number of Mice |
|---|---|---|
| Control | | 8 |
| Hydrochloride salt obtained in Example 36 | 58.4 | 8 |
| Hydrochloride salt obtained in Example 75 | 36.1 | 8 |
| Hydrochloride salt obtained in Example 33 | 100.0 | 6 |
| Hydrochloride salt obtained in Example 34 | 48.6 | 8 |
| Hydrochloride salt obtained in Example 50 | 92.0 | 6 |
| Hydrochloride salt obtained in Example 63 | 30.8 | 8 |
| Hydrochloride salt obtained in Example 54 | 63.6 | 7 |

**Table 16**

| | Suppression Rate (%) | Number of Mice |
|---|---|---|
| Control | | 7 |
| Hydrochloride salt obtained in Example 92 | 25.8 | 7 |
| Hydrochloride salt obtained in Example 96 | 59.6 | 7 |
| Hydrochloride salt obtained in Example 116 | 21.3 | 7 |
| Hydrochloride salt obtained in Example 118 | 11.2 | 7 |

**Table 17**

| | Suppression Rate (%) | Number of Mice |
|---|---|---|
| Control | | 7 |
| Sodium salt obtained in Example 106 | 10.9 | 7 |
| Trishydroxymethylaminomethane salt obtained in Example 106 | 14.1 | 7 |
| Sodium salt obtained in Example 107 | 13.5 | 7 |
| Hydrochloride salt obtained in Example 122 | 34.5 | 7 |
| Hydrochloride salt obtained in Example 124 | 11.3 | 7 |

From the results shown above, it was confirmed that the compounds of the present invention had excellent effect of suppressing IV-type allergic reaction in its effector phase.

Also, from results of the acute toxicity performed, the safety of the compounds of the present invention was confirmed.

Hence, the compounds of the present invention are useful as antiallergic drugs, effective against IV-type allergy and the like.

The dose amount and the preparation into formulations of the compounds of the invention will now be described.

The compounds of the invention can be administered to animal and man, directly or together with a vehicle commonly used. The dose form is not particularly limited, and is selected appropriately as needed on use, including oral drugs such as tablets, capsules, granules, grains and powder, or non-oral drugs such as injection and suppository.

In order to obtain the desired effect from the oral drugs, it is believed appropriate to administer the compound of the present invention to an ordinary adult in an amount of 1 mg to 600 mg per day, at several times, though the amount depends on the age and weight of the patient and the degree of the disease.

The oral drugs are prepared by ordinary methods, using starch, lactose, sucrose, mannitol, carboxymethylcellulose, corn starch, or inorganic salts.

In the drugs of this type, use can be made of a binder, a disintegrator, a surfactant, a lubricant, a fluidity-promoting agent, a flavor, a tinction, a perfume and the like, in addition to the vehicle mentioned above. Specific examples of some of these substances will be given below:

### [Binder]

Starch, dextrin, powdered acasia, gelatin, hydroxypropylstarch, methylcellulose, carboxymethylcellulose sodium, hydroxypropylcellulose, crystalline cellulose, ethylcellulose, polyvinylpyrrolidone, and macrogol.

### [Disintegrator]

Starch, hydroxypropylstarch, carboxymethylcellulose sodium, carboxymethylcellulose calcium, carboxymethylcellulose, and low-substituted hydroxypropylcellulose.

### [Surfactant]

Sodium laurylsulfate, soybean lecithin, succharose fatty acid ester, and polysorbate 80.

### [Lubricant]

Talc, waxes, hydrogenated vegetable oil, succharose fatty acid ester, magnesium stearate, calcium stearate, aluminum stearate, and polyethylene glycol.

### [Fluidity-Promoting Agent]

Light anhydrous silicic acid, dry aluminum hydroxide gel, synthetic aluminum silicate, and magnesium silicate.

The compounds of the present invention can also be administered in the form of suspension, emulsion, syrup or elixir. These forms of drugs may also contain flavor, perfume and tinction.

In order to obtain the desired effect from the non-oral drugs, it is believed appropriate to administer the compound of the present invention to an ordinary adult in an amount of 1 mg to 200 mg per day in the form of phleboclysis, intravenous drip, hypodermic injection, or intramuscular injection, though the amount depends on the age and weight of the patient, and the degree of the disease.

The non-oral drugs can be prepared by ordinary methods, and use may be made of an attenuant such as distilled water for injection, physiological saline, aqueous solution of glucose, vegetable oil for injection, sesame oil, peanut oil, soybean oil, corn oil, propylene glycol, or polyethylene glycol. If necessary, germicide, preservative, and stabilizer may be added. The non-oral drugs can be filled in a vial or the like and frozen, and then be removed of water by the ordinary freeze-dry technique. A liquid drug can be reformulated from the freeze-dried drug immediately before administration. Further, an isotonic, a stabilizer, a preservative, an antiseptic, a sedative, and the like may be added.

Examples of other non-oral drugs include a lotion, an ointment, and a suppository for intrarectal administration, which are prepared by ordinary methods.

The present invention will be described in greater detail, with reference to examples 3 to 139. The invention is not limited to these examples, nonetheless. Examples 1A, 1B and 2A-2D fall outside the scope of the present invention, but are included as they help to illustrate the present invention.

### Example 1A (Esterification and Amidation)

2 mℓ of piperidine was added to a solution of 5.09 g of 4-acetoxy-3-methoxycinnamoyl chloride in 100 mℓ of pyridine. The solution was reacted for 4 hours at room temperature. After reaction, 200 mℓ of 2N hydrochloric acid was added to the reaction solution. The solution was extracted three times with 100 mℓ of ethyl acetate. The organic layer obtained was washed three times with 2N hydrochloric acid, twice with an aqueous sodium hydrogencarbonate solution and twice with an aqueous sodium chloride solution, and then was dried over magnesium sulfate. Thereafter, the solvent was removed in vacuo, and the product obtained was recrystallized from methylene chloride/hexane, yielding 1.88 g of 1-(4-acetoxy-3-methoxycinnamoyl)piperidine (a compound of the present invention) as pale yellowish white crystal, which had the following physiochemical properties:
Melting point: 129.6 - 130.3°C
Proton nuclear magnetic resonance spectrum (δ ppm in CDCℓ₃):
1.6 - 1.8 (6H, br), 2.32 (3H, s),
3.6 - 3.8 (4H, br), 3.87 (3H, s),
6.80 (1H, d, J = 16Hz),
7.1 - 7.3 (3H, m),
7.55 (1H, d, J = 16Hz)
IRₘₐₓ (KBr, cm⁻¹): 2936, 1760, 1648
MS (M⁺): 303

| Elemental analysis (C₁₇H₂₁NO₄): | | | |
|---|---|---|---|
| Theoretical value; | C: 67.31, | H: 6.98, | N: 4.62 |
| Measured value; | C: 67.36, | H: 6.96, | N: 4.81 |

### Example 1B (Hydrolysis and Conversion into Non-toxic Salt)

5 g of potassium carbonate was added a solution of 1.88 g 1-(4-acetoxy-3-methoxycinnamoyl)piperidine (Example 1A) in 70 mℓ of methanol. The solution was vigorously stirred for 2.5 hours at room temperature.

After reaction, 2N hydrochloric acid was added to the reaction solution, acidifying the solution. The solution was extracted three times with 50 mℓ of ethyl acetate. The organic layer obtained was washed twice with an aqueous sodium chloride solution and dried over magnesium sulfate. Thereafter, the solvent was removed in vacuo, and the product obtained was recrystallized from ethyl acetate/hexane, yielding 1.51 g of 1-(4-hydroxy-3-methoxycinnamoyl)piperidine (a compound of the present invention) as pale yellow crystal, which had the following physiochemical properties:
Melting point: 136.6 - 137.1°C
Proton nuclear magnetic resonance spectrum (δ ppm in CDCℓ₃):
1.5 - 1.8 (6H, br), 3.5 - 3.8 (4H, br),
3.92 (3H, s), 6.27 (1H, s),
6.71 (1H, d, J = 16Hz),
6.89 (1H, d, J = 8Hz),
6.08 (1H, d, J = 2Hz),
7.06 (1H, dd, J = 2, 6Hz),
7.55 (1H, d, J = 16Hz)
IRₘₐₓ (KBr, cm⁻¹): 3268, 2936, 1640, 1620
MS (M⁺): 261

| Elemental analysis (C₁₅H₁₉NO₃): | | | |
|---|---|---|---|
| Theoretical value; | C: 68.94, | H: 7.33, | N: 5.36 |
| Measured value; | C: 68.77, | H: 7.34, | N: 5.50 |

The compound described above was reacted in the ordinary method, converting it into sodium 1-(3-methoxy-4-oxidocinnamoyl)piperidine, which had the following physiochemical properties:
Melting point: 270 - 271°C
Proton nuclear magnetic resonance spectrum (δ ppm in D₂O):
1.4 - 1.7 (6H, m), 3.5 - 3.7 (5H, m),
3.82 (3H, s), 6.63 (1H, d, J = 8Hz),
6.75 (1H, d, J = 16Hz),
7.09 (1H, d, J = 8Hz),
7.15 (1H, s), 7.40 (1H, d, J = 16Hz)
MS [(M+H)⁺]: 284

| HRMS (C₁₅H₁₉NO₃Na): | |
|---|---|
| Theoretical value: | 284.12629 |
| Measured value: | 284.12470 |

### Example 2A (Esterification and Amidation, and Hydrolysis and Conversion into Non-toxic Salt)

8.1 g of 4-acetoxy-3-methoxycinnamoyl chloride and 15 g of 4-piperidinecarboxylic acid were added to 200 mℓ of saturated aqueous potassium carbonate. The solution was stirred for 4 hours at room temperature. After reaction, 2N hydrochloric acid was added to the reaction solution, acidifying the solution. 50 mℓ of ethyl acetate was added to the solution, and the solution was stirred for 1 hour. The crystal precipitated was filtered out and recrystallized from ethyl acetate/hexane, yielding 5.4 g of 1-(4-hydroxy-3-methoxycinnamoyl)piperidine-4-carboxylic acid (a compound of the present invention) as white crystal, which had the following physiochemical properties:
Melting point: 182.2 - 182.8°C
Proton nuclear magnetic resonance spectrum (δ ppm in CD₃OD):
1.6 - 2.2 (4H, m), 2.6 - 3.5 (3H, m),
3.91 (3H, s), 4.1 - 4.6 (2H, m),
6.79 (1H, d, J = 8Hz),
6.90 (1H, d, J = 16Hz),
7.05 (1H, dd, 8, 2Hz),
7.19 (1H, d, J = 2Hz),
7.47 (1H, d, J = 16Hz)
IRₘₐₓ (KBr, cm⁻¹): 3260, 1730, 1640, 1604
MS (M⁺): 305

| Elemental analysis (C₁₆H₁₉NO₅): | | | |
|---|---|---|---|
| Theoretical value; | C: 62.94, | H: 6.27, | N: 4.59 |
| Measured value; | C: 62.85, | H: 6.20, | N: 4.87 |

The compound described above was reacted in the ordinary method, converting it into disodium 1-(3-oxido-4-methoxycinnamoyl)piperidine-4-carboxylate, which had the following physiochemical properties:
Melting point: 243.8 - 246.5°C
Proton nuclear magnetic resonance spectrum (δ ppm in D₂O):
1.4 - 3.4 (7H, m), 3.71 (1H, m),
3.84 (3H, s), 4.1 - 4.5 (2H, m),
6.68 (1H, d, J = 8Hz),
6.78 (1H, d, J = 16Hz),
7.10 (1H, d, J = 8Hz), 7.17 (1H, s),
7.42 (1H, d, J = 16Hz)
MS [(M+H)⁺]: 350

| HRMS (C₁₆H₁₈NO₅Na₂): | |
|---|---|
| Theoretical value: | 350.09810 |
| Measured value: | 350.10150 |

### Example 2B (Esterification and Amidation)

A solution of 6.9 g of 1-(4-hydroxy-3-methoxycinnamoyl)piperidine-4-carboxylic acid (Example 2A), 10 g of potassium carbonate and 6.5 mℓ of allyl bromide, mixed in 250 mℓ of DMF, was reacted for 7 hours at room temperature. After reaction, 400 mℓ of an aqueous sodium chloride solution was added to the reaction solution. The solution was extracted three times with 100 mℓ of ethyl acetate. The organic layer obtained was washed twice with an aqueous sodium chloride solution and dried over magnesium sulfate. Thereafter, the solvent was removed in vacuo. The oily substance obtained was subjected to column chromatography using 100 g of silica gel. From the fraction eluted with hexane and ethyl acetate = 3:2, the solvent was removed in vacuo, yielding 7.8 g of allyl 1-(4-allyloxy-3-methoxycinnamoyl)piperidine-4-carboxylate (a compound of the present invention) as a pale yellow oil, which had the following physiochemical properties:
Proton nuclear magnetic resonance spectrum (δ ppm in CDCℓ₃):
1.6 - 2.1 (6H, m), 2.5 - 3.3 (3H, m),
3.92 (3H, s), 4.6 - 4.7 (4H, m),
5.2 - 5.5 (4H, m), 5.8 - 6.2 (2H, m),
6.70 (1H, d, J = 16Hz),
6.84 (1H, d, J = 8Hz),
7.0 - 7.2 (2H, m),
7.56 (1H, d, J = 16Hz)
MS (M⁺): 385

| HRMS (C₂₂H₂₇NO₃): | |
|---|---|
| Theoretical value: | 385.18883 |
| Measured value: | 385.18883 |

### Example 2C (Alkylation)

50 mℓ of sodium hexamethyldisilazide (1M solution in THF) and 5.3 mℓ of methyl iodide were added to a solution of 7.8 g of allyl 1-(4-allyloxy-3-methoxycinnamoyl)-piperidine-4-carboxylate (Example 2B) in 200 mℓ of THF. The solution was reacted for 18 hours at room temperature. After reaction, 200 mℓ of an aqueous ammonium chloride solution was added to the reaction solution. The solution was extracted three times with 100 mℓ of ethyl acetate. The organic layer obtained was washed twice with an aqueous sodium chloride solution and dried over magnesium sulfate. Thereafter, the solvent was removed in vacuo. The oily substance obtained was subjected to column chromatography using 100 g of silica gel. From the fraction eluted with hexane and ethyl acetate = 3:2, the solvent was removed in vacuo, yielding 1.1 g of allyl 1-(4-allyloxy-3-methoxycinnamoyl)-4-methylpiperidine-4-carboxylate (a compound of the present invention) as a pale yellow oil, which had the following physiochemical properties:
Proton nuclear magnetic resonance spectrum (δ ppm in CDCℓ₃):
1.25 (3H, s), 1.3 - 1.8 (4H, m),
2.2 - 3.2 (3H, m), 3.8 - 4.2 (2H, m),
3.91 (3H, s), 5.7 (1H, m),
6.67 (1H, d, J = 16Hz),
6.87 (1H, d, J = 8 Hz), 7.05 (1H, s),
7.11 (1H, d, J = 8Hz),
7.68 (1H, d, J = 16Hz)
MS (M⁺): 399

### Example 2D (Reduction and Conversion into Non-toxic Salt)

0.4 g of bis(triphenylphosphine)palladium dichloride and 5 g of ammonium formate were added to a solution of 1.1 g of allyl 1-(4-allyloxy-3-methoxycinnamoyl)-4-methylpiperidine-4-carboxylate (Example 2C) in 100 mℓ of THF. Under an argon stream, the solution was reacted for 24 hours, while it was refluxed. After reaction, 150 mℓ of an aqueous sodium chloride solution was added to the reaction solution. The solution was extracted three times with 50 mℓ of ethyl acetate. The organic layer obtained was washed three times with an aqueous sodium chloride solution and dried over magnesium sulfate. Thereafter, the solvent was removed in vacuo, yielding 1.1 g of 1-(4-hydroxy-3-methoxycinnamoyl)-4-methylpiperidine-4-carboxylic acid (a compound of the present invention) as a pale yellow oil, which had the following physiochemical properties:
Proton nuclear magnetic resonance spectrum (δ ppm in CDCℓ₃):
1.26 (3H, s), 1.3 - 2.3 (4H, m),
2.9 - 3.4 (3H, m), 3.8 - 4.4 (2H, m),
3.93 (3H, s), 5.78 (1H, m),
6.68 (1H, d, J = 16Hz),
6.89 (1H, d, J = 8Hz),
6.99 (1H, d, J = 2Hz),
7.07 (1H, dd, J = 6, 2Hz)
MS (M⁺): 319

| HRMS (C₁₇H₂₁NO₅): | |
|---|---|
| Theoretical value: | 319.14191 |
| Measured value: | 319.14111 |

The compound described above was reacted in the ordinary method, converting it into disodium 1-(3-methoxy-4-oxidocinnamoyl)-4-methylpiperidine-4-carboxylate, which had the following physiochemical properties:
Proton nuclear magnetic resonance spectrum (δ ppm in D₂O):
1.0 - 2.2 (4H, m), 3.0 - 3.6 (2H, m),
3.70 (1H, m), 3.83 (3H, s),
3.8 - 4.1 (2H, m),
6.58 (1H, d, J = 8Hz),
6.75 (1H, d, J = 16Hz),
7.09 (1H, d, J = 8Hz), 7.29 (1H, s),
7.35 (1H, d, J = 16Hz)
MS [(M+H)⁺]: 364

| HRMS (C₁₇H₂₀NO₅Na₂): | |
|---|---|
| Theoretical value: | 364.11375 |
| Measured value: | 364.11150 |

### Example 3 (Esterification and Amidation)

Using 5.09 g of 4-acetoxy-3-methoxycinnamoyl chloride, 2.98 g of 4-methylcyclohexylamine hydrochloride, and 100 mℓ of pyridine, a reaction similar to that conducted in Example 1A was carried out. As a result, 4.31 g of N-(4-methylcyclohexyl)-4-acetoxy-3-methoxycinnamamide was obtained as yellowish white crystal, which had the following physiochemical properties:
Melting point: 193.5 - 194.3°C
Proton nuclear magnetic resonance spectrum (δ ppm in DMSO-d₆):
0.89 (12/5H, d, J = 6Hz),
0.93 (3/5H, d, J = 6Hz),
1.0 - 2.1 (9H, m), 2.32 (3H, s)
3.83 (12/5H, br), 3.85 (3/5H, s),
3.86 (12/5H, s), 4.12 (1/5H, br),
5.46 (4/5H, m), 5.69 (1/5H, m),
6.25 (4/5H, d, J = 16Hz),
6.30 (1/5H, d, J = 16Hz),
6.9 - 7.1 (3H, m),
7.51 (4/5H, d, J = 16Hz),
7.53 (1/5H, d, J = 16Hz)
IRₘₐₓ ( KBr, cm⁻¹): 3284, 2936, 1766 1656
MS (M⁺): 331

| Elemental analysis (C₁₉H₂₅NO₄): | | | |
|---|---|---|---|
| Theoretical value; | C: 68.86, | H: 7.60, | N: 4.23 |
| Measured value; | C: 68.81, | H: 7.41, | N: 4.25 |

### Example 4 (Hydrolysis and Conversion into Non-toxic Salt)

Using 4.31 g of N-(4-methylcyclohexyl)-4-acetoxy-3-methoxycinnamamide (Example 3), 100 mℓ of methanol, and 10 g of potassium carbonate, a reaction similar to that conducted in Example 1B was carried out. As a result, 3.14 g of N-(4-methylcyclohexyl)-4-hydroxy-3-methoxycinnamamide (a compound of the present invention) was obtained as pale yellowish white crystal, which had the following physiochemical properties:
Melting point: 205.6 - 206.2°C
Proton nuclear magnetic resonance spectrum (δ ppm in DMSO-d₆):
0.86 (12/5H, d, J = 8Hz),
0.90 (3/5H, d, J = 8Hz),
1.0 - 1.9 (9H, m), 3.56 (4/5H, m),
3.80 (12/5H, s), 3.82 (3/5H, s),
3.91 (1/5H, m),
6.37 (4/5H, d, J = 16Hz),
6.53 (1/5H, d, J = 16Hz),
6.7 - 7.1 (3H, m),
7.25 (4/5H, d, J = 16Hz),
7.26 (1/5H, d, J = 16Hz),
7.74 (1/5H, d, J = 8Hz),
7.75 (4/5H, d, J = 8Hz),
9.35 (1H, s)
IRₘₐₓ (KBr, cm⁻¹): 3252, 2924, 1650
MS (M⁺): 289

| Elemental analysis (C₁₇H₂₃NO₃): | | | |
|---|---|---|---|
| Theoretical value; | C: 70.56, | H: 8.01, | N: 4.84 |
| Measured value; | C: 70.60, | H: 7.96, | N: 5.04 |

The compound described above was reacted in the ordinary method, converting it into sodium N-(4-methylcyclohexyl)-3-methoxy-4-oxidocinnamamide, which had the following physiochemical properties:
Melting point: 233.6 - 235.8°C
Proton nuclear magnetic resonance spectrum (δ ppm in D₂O):
0.84 (12/5H, d, J = 6Hz),
0.86 (3/5H, d, J = 6Hz),
0.9 - 2.0 (9H, m), 3.58 (4/5H, m),
3.80 (12/5H, s), 3.81 (3/5H, s),
3.89 (1/5H, m),
6.23 (4/5H, d, J = 16Hz),
6.38 (1/5H, d, J = 16Hz),
6.6 - 7.1 (3H, m),
7.35 (4/5H, d, J = 16Hz),
7.42 (1/5H, d, J = 16Hz),
7.71 (1/5H, d, J = 8Hz),
7.73 (4/5H, d, J = 8Hz)
MS [(M+H)⁺]: 312

| HRMS (C₁₇H₂₃NO₃Na): | |
|---|---|
| Theoretical value: | 312.15759 |
| Measured value: | 312.15480 |

### Example 5 (Convertion into Imine)

A solution prepared by mixing 5.6 g of 4-methylcyclohexanone, 5.35 g of benzylamine, and 50 mℓ of benzene was reacted, while it was refluxed. The water formed during the reaction was removed from the system by using a Dean-Stark trap. The reaction was performed for 8 hours.

After reaction, the solvent was removed in vacuo, yielding 8.6 g of N-benzyl-4-methylcyclohexaneimine as a colorless oil, which had the following physiochemical properties:
Proton nuclear magnetic resonance spectrum (δ ppm in CDCℓ₃):
0.95 (3H, d, J = 6Hz),
1.0 - 2.9 (9H, m),
4.54 (2H, s), 7.1 - 7.5 (5H, m)
MS (M⁺): 201

| HRMS (C₁₄H₁₉N): | |
|---|---|
| Theoretical value: | 201.15175 |
| Measured value: | 201.14189 |

### Example 6 (Reduction and Hydrolysis)

2 g of sodium borohydride was added to a solution of 8.6 g of N-benzyl-4-methylcyclohexaneimine (Example 5) in 50 mℓ of methanol. The solution was reacted for 2 hours, while it was refluxed.

After reaction, 10 mℓ of 2N hydrochloric acid was added to the reaction solution under ice-cooling, and 10 mℓ of ammonia water and 100 mℓ of 2N aqueous sodium chloride solution were added. The solution was extracted three times with 30 mℓ of methylene chloride. The organic layer obtained was washed with an aqueous sodium chloride solution and dried over magnesium sulfate. Thereafter, the solvent was removed in vacuo, yielding 7.1 g of N-benzyl-4-methylcyclohexylamine which was a mixture of cis and trans = about 1:4 and which had the following physiochemical properties:
Proton nuclear magnetic resonance spectrum (δ ppm in CDCℓ₃):
0.85 (9/4H, d, J = 7Hz),
0;91 (3/4H, d, J = 7Hz),
1.0 - 2.8 (9H, m), 3.7 - 3.9 (1H, m),
3.86 (2H, s), 7.2 - 7.4 (5H, m)
MS (M⁺): 203

### Example 7 (Reduction)

0.1 g of 20% palladium hydroxide-carbon was added to a solution of 7.1 g of N-benzyl-4-methylcyclohexylamine (Example 6) in 50 mℓ of methanol. Under normal-pressure hydrogen gas, the solution was vigorously stirred for 18 hours. After reaction, the catalyst was filtered out, and hydrogen chloride gas was blown into the filtrate. Thereafter, the methanol was removed in vacuo, yielding 4.5 g of 4-methylcyclohexylammonium chloride which was a mixture of cis and trans = about 1:4 and which had the following physiochemical properties:
Proton nuclear magnetic resonance spectrum (δ ppm in CDCℓ₃):
0.83 (12/5H, d, J = 6Hz),
0.97 (3/5H, d, J = 6Hz),
0.9 - 2.2 (11H, m), 3.08 (4/5H, m),
3.39 (1/5H, m)
MS (M⁺): 149

### Example 8 (Refining)

4-methylcyclohexylammonium chloride (Example 7) was suspended in 4 mℓ of methylene chloride cooled. The crystal obtained was filtered out, yielding 3.2 g of trans-4-methylcyclohexylammonium chloride as white crystal, which had the following physiochemical properties:
Proton nuclear magnetic resonance spectrum (δ ppm in CDCℓ₃):
0.87 (3H, d, J = 6Hz),
0.9 - 2.2 (9H, m), 3.0 - 3.2 (1H, m),
8.22 (2H, br)
MS [(M-Cℓ)⁺]: 114

### Example 9 (Esterification and Amidation)

21 g of 4-dimethylaminobutyric acid hydrochloride was added, under ice-cooling, to a solution of 16 mℓ of diethyl chlorophosphate, 72 mℓ of triethylamine and 250 mℓ of methylene chloride. Then, the solution was restored to room temperature, and stirred for 1 hour. Thereafter, 22 g of trans-4-methylcyclohexylammonium chloride (Example 8), and 0.6 g of 4-dimethylaminopyridine were added to the solution.

After the addition, the solution was restored to room temperature, and was reacted for 6 hours. After reaction, the reaction solution was washed five times with 300 mℓ of an aqueous sodium hydrogencarbonate. The organic layer obtained was dried over magnesium sulfate. Thereafter, the solvent was removed in vacuo, yielding 13.1 g of N-(trans-4-methylcyclohexyl)-4-dimethylaminobutylamide as white crystal, which had the following physiochemical properties:
Proton nuclear magnetic resonance spectrum (δ ppm in CDCℓ₃):
0.87 (3H, d, J = 6Hz),
0.9 - 2.0 (13H, m), 2.21 (6H, s),
2.27 (2H, t, J = 7Hz), 3.68 (1H, m)
MS [(M+H)⁺]: 199

### Example 10 (Reduction)

13.1 g of N-(trans-4-methylcyclohexyl)-4-dimethylaminobutylamide (Example 9) was dissolved in 100 mℓ of THF. Under an argon stream, the solution was added dropwise to a solution of 7.5 g of lithium aluminum hydride suspended in 400 mℓ of THF under ice-cooling. After the addition, the solution was reacted for 8 hours, while it was refluxed. After reaction, the solution was allowed to cool to room temperature. Then, 22 g of sodium sulfate decahydrate was added to the solution under ice-cooling. The solution was stirred for 1 hour, and the precipitate was filtered out. The solvent was removed in vacuo, yielding an oily substance. This substance was subjected to vacuum distillation, yielding 5.7 g of N-(4-dimethylaminobutyl)-trans-4-methylcyclohexylamine as a colorless oil, which had the following physiochemical properties:
Boiling point: 78 - 79.5°C/0.5 mmHg
Proton nuclear magnetic resonance spectrum (δ ppm in CDCℓ₃):
0.86 (3H, d, J = 7Hz),
0.8 - 2.4 (16H, m), 2.21 (6H, s),
2.63 (2H, br)
MS [(M+H)⁺]: 213

### Example 11 (Esterification and Amidation, and Hydrolysis and Conversion into Non-toxic Salt)

A mixture solution of 2.8 mℓ of diethyl chlorophosphate, 2.7 mℓ of triethylamine, 2.3 g of 4-acetoxy-3-methoxycinnamic acid, and 100 mℓ of methylene chloride was stirred for 1 hour at room temperature, Next, 3.5 mℓ of N-(4-dimethylaminobutyl)-trans-4-methylcyclohexylamine (Example 10) and 0.5 g of 4-dimethylaminopyridine were added to the solution under ice-cooling. The solution was stirred further for 4 hours at room temperature. Then, 100 mℓ of methanol and 5 g of potassium carbonate were added to the solution, and the solution was stirred for 1.5 hours at room temperature. After stirring, the reaction solution was neutralized with 2N hydrochloric acid under ice-cooling. The solution was extracted three times with 50 mℓ of ethyl acetate. The organic layer obtained was washed twice with an aqueous sodium hydrogencarbonate solution and twice with aqueous sodium chloride solution, and was dried over magnesium sulfate. Thereafter, the solvent was removed in vacuo. The product obtained was recrystallized from methylene chloride/either, yielding 1.7 g of N-(4-dimethylaminobutyl)-N-(trans-4-methylcyclohexyl)-4-hydroxy-3-methoxycinnamamide (a compound of the present this invention) as a pale yellow oily oil, which had the following physiochemical properties:
Proton nuclear magnetic resonance spectrum (δ ppm in CDCℓ₃):
0.89 (3H, d, J = 6Hz),
0.9 - 1.9 (13H, m), 2.24 (6H, s),
2.28 (2H, t, J = 7Hz),
3.22 (2H, t, J = 7Hz), 3.66 (1H, m),
3.91 (3H, s),
6.71 (1H, d, J = 16Hz),
6.9 - 7.1 (3H, m),
7.58 (1H, d, J = 16Hz)
MS [(M+H)⁺]: 389

| HRMS (C₂₃H₃₇N₂O₃): | |
|---|---|
| Theoretical value: | 389.28042 |
| Measured value: | 389.28110 |

The compound described above was reacted in the ordinary method, converting it into N-(4-dimethylaminobutyl)-N-(trans-4-methylcyclohexyl)-4-hydroxy-3-methoxycinnamamide hydrochloride, which had the following physiochemical properties:
Proton nuclear magnetic resonance spectrum (δ ppm in D₂O):
0.89 (3H, d, J = 6Hz),
0.9 - 1.9 (13H, m),
2.51 (2H, t, J = 7Hz), 3.09 (6H, s),
3.61 (2H, t, J = 7Hz), 3.65 (1H, m),
3.90 (3H, s),
6.63 (1H, d, J = 16Hz),
6.8 - 7.1 (3H, m),
7.51 (1H, d, J = 16Hz)
MS [(M-Cℓ)⁺]: 389

| HRMS (C₂₃H₃₇N₂O₃): | |
|---|---|
| Theoretical value: | 389.28042 |
| Measured value: | 389.28057 |

### Example 12 (Reduction and Conversion into Non-toxic Salt)

0.05 g of 10% palladium-carbon was added to a solution of 1 g of N-(4-dimethylaminobutyl)-N-(trans-4-methylcyclohexyl)-4-hydroxy-3-methoxycinnamamide (Example 75) in 60 mℓ of methanol. Under normal-pressure hydrogen gas, the solution was reacted for 16 hours, while it was vigorously stirred. After reaction, the catalyst was filtered out, and the solvent was removed in vacuo from the filtrate, yielding 0.92 g of N-(4-dimethylaminobutyl)-N-(trans-4-methylcyclohexyl)-3-(4-hydroxy-3-methoxyphenyl)propionamide (a compound of the present invention) as a pale yellow oily oil, which had the following physiochemical properties:
Proton nuclear magnetic resonance spectrum (δ ppm in CDCℓ₃):
0.86 (3H, d, J = 6Hz),
0.9 - 1.9 (13H, m), 2.30 (6H, s),
2.42 (2H, t, J = 9Hz),
2.64 (2H, t, J = 7Hz),
2.80 (2H, t, J = 7Hz),
3.28 (2H, t, J = 7Hz), 3.62 (1H, m),
3.79 (3H, s), 6.8 - 7.1 (3H, m)
MS [(M+H)⁺]: 391

| HRMS (C₂₃H₃₉N₂O₃): | |
|---|---|
| Theoretical value: | 391.29603 |
| Measured value: | 391.29607 |

The compound described above was reacted in the ordinary method, converting it into N-(4-dimethylaminobutyl)-N-(trans-4-methylcyclohexyl)-3-(4-hydroxy-3-methoxyphenyl)propionamide hydrochloride, which had the following physiochemical properties:
Proton nuclear magnetic resonance spectrum (δ ppm in D₂O):
0.86 (3H, d, J = 6Hz),
0.9 - 1.9 (13H, m),
2.61 (2H, t, J = 7Hz),
2.98 (2H, t, J = 7Hz), 3.08 (6H, s),
3.26 (2H, t, J = 7Hz), 3.61 (1H, m),
3.76 (2H, t, J = 7Hz), 3.78 (3H, s),
6.7 - 7.1 (3H, m)
MS [(M-Cℓ)⁺]: 391

| HRMS (C₂₃H₃₉N₂O₃): | |
|---|---|
| Theoretical value: | 391.29607 |
| Measured value: | 391.29479 |

### Example 13 (Esterification and Amidation)

Under ice-cooling, 10.4 g of dimethylaminoglycine hydrochloride was added to a solution of 13 mℓ of diethyl chlorophosphate, 36 mℓ of triethylamine, and 100 mℓ of methylene chloride. Thereafter, the solution was restored to room temperature and stirred for 1 hour. After 1 hour, 18 g of trans-4-methylcyclohexylammonium chloride and 0.3 g of 4-dimethylaminopyridine were added to the solution.

After the addition, the solution was restored to room temperature, and the reaction was continued for 6 hours. After reaction, the reaction solution was washed five times with 200 mℓ of an aqueous sodium hydrogencarbonate. The organic layer obtained was dried over magnesium sulfate. The solvent was removed in vacuo, yielding 8.5 g of N-(trans-4-methylcyclohexyl)-2-dimethylaminoacetamide as white crystal, which had the following physiochemical properties:
Proton nuclear magnetic resonance spectrum (δ ppm in CDCℓ₃):
0.88 (3H, d, J = 6Hz),
1.0 - 2.0 (9H, m),
2.28 (3H, s), 2.92 (2H, s),
3.71 (1H, m)
MS [(M+H)⁺]: 199

| HRMS (C₁₁H₂₃N₂O): | |
|---|---|
| Theoretical value: | 199.18104 |
| Measured value: | 199.18206 |

### Example 14 (Reduction)

8.5 g of N-(trans-4-methylcyclohexyl)-2-dimethylaminoacetamide was dissolved in 100 mℓ of THF. Under an argon stream, the solution was added dropwise to a solution of 3.6 g of lithium aluminum hydride suspended in 200 mℓ of THF under ice-cooling. After the addition, the solution was reacted for 8 hours, while it was refluxed, After reaction, the solution was allowed to cool to room temperature. Then, 10 g of sodium sulfate decahydrate was added to the solution under ice-cooling. The solution was stirred for 1 hour, and the precipitate was filtered out. The solvent was removed in vacuo to obtaine an oily substance. This substance was refined by vacuum distillation, yielding 3.8 g of N-(2-dimethylaminoethyl)-trans-4-methylcyclohexylamine as a colorless oil, which had the following physiochemical properties:
Boiling point: 78.0 - 79.5°C/0.5 mmHg
Proton nuclear magnetic resonance spectrum (δ ppm in CDCℓ₃):
0.85 (3H, d, J = 6Hz),
0.9 - 2.0 (9H, m), 2.22 (6H, s),
2.36 (2H, t, J = 7Hz),
2.64 (2H, t, J = 7Hz),
MS [(M+H)⁺] 185

| HRMS (C₁₁H₂₅N₂): | |
|---|---|
| Theoretical value: | 185.20177 |
| Measured value: | 185.20212 |

### Example 15 (Esterification and Amidation, and Hydrolysis and Conversion into Non-toxic Salt)

Using 2.5 g of 4-acetoxy-3-methoxycinnamic acid, 2.8 mℓ of diethyl chlorophosphate, 2.7 mℓ of triethylamine, 100 mℓ of methylene chloride, 3 mℓ of N-(2-dimethylaminoethyl)-trans-4-methylcyclohexylamine (Example 14), 0.5 g of 4-dimethylaminopyridine, 100 mℓ of methanol, and 3 g of potassium carbonate, a reaction similar to that conducted in Example 9 was carried out. As a result, 1.8 g of N-(2-dimethylaminoethyl)-N-(trans-4-methylcyclohexyl)-4-hydroxy-3-methoxycinnamamide (a compound of the present invention) was obtained as white crystal, which had the following physiochemical properties:
Proton nuclear magnetic resonance spectrum (δ ppm in CDCℓ₃):
0.87 (3H, d, J = 6Hz),
0.9 - 1.8 (9H, m), 2.39 (6H, s),
2.49 (2H, t, J = 7Hz),
3.42 (2H, t, J = 7Hz), 3.72 (1H, m),
3.82 (3H, s),
6.81 (1H, d, J = 16Hz),
7.1 - 7.3 (3H, m),
7.37 (1H, d, J = 16Hz)
MS [(M+H)⁺] 361

| HRMS (C₂₁H₃₂N₂O₃): | |
|---|---|
| Theoretical value: | 361.24129 |
| Measured value: | 316.24910 |

The compound described above was reacted in the ordinary method, converting it into N-(2-dimethylaminoethyl)-N-(trans-4-methylcyclohexyl)-4-hydroxy-3-methoxycinnamamide hydrochloride, which had the following physiochemical properties:
Proton nuclear magnetic resonance spectrum (δ ppm in D₂O):
0.87 (3H, d, J = 6Hz),
0.9 - 1.9 (9H, m),
2.89 (2H, t, J = 7Hz), 3.04 (6H, s),
3.71 (1H, m),
3.76 (2H, t, J = 16Hz),
3.83 (3H, s),
6.77 (1H, d, J = 16Hz),
7.0 - 7.73 (3H, m),
7.34 (1H, d, J = 16Hz)
MS [(M-Cℓ)⁺]: 361

| HRMS (C₂₁H₃₃N₂O₃): | |
|---|---|
| Theoretical value: | 361.24912 |
| Measured value: | 361.24845 |

### Example 16 (Reduction and Conversion into Non-toxic Salt)

0.05 g of 10% palladium-carbon was added to a solution of 1 g of N-(2-dimethylaminoethyl)-N-(trans-4-methylcyclohexyl)-4-hydroxy-3-methoxycinnamamide (Example 15) in 60 mℓ of methanol. Under normal-pressure hydrogen gas, the solution was vigorously stirred. After 16 hours, the reaction was ceased, the catalyst was filtered out, and the solvent was removed in vacuo from the filtrate. The product obtained was recrystallized from methylene chloride/ether, yielding 0.94 g of N-(2-dimethylaminoethyl)-N-(trans-4-methylcyclohexyl)-3-(4-hydroxy-3-methoxyphenyl)propionamide (a compound of the present invention) as white crystal, which had the following physiochemical properties:
Proton nuclear magnetic resonance spectrum (δ ppm in CDCℓ₃):
0.87 (3H, d, J = 6Hz),
0.9 - 1.8 (9H, m),
2.38 (6H, s), 2.48 (2H, t, J = 7Hz),
2.58 (2H, t, J = 7Hz),
2.71 (2H, t, J = 7Hz),
3.43 (2H, t, J = 7Hz), 3.71 (1H, m),
3.81 (3H, s), 6.79 (1H, m)
7.0 - 7.3 (3H, m)
MS [(M+H)⁺]: 363

| HRMS (C₂₁H₃₅N₂O₃): | |
|---|---|
| Theoretical value: | 363.26477 |
| Measured value: | 363.26474 |

The compound described above was reacted in the ordinary method, converting it into N-(2-dimethylaminoethyl)-N-(trans-4-methylcyclohexyl)-3-(4-hydroxy-3-methoxyphenyl)propionamide hydrochloride, which had the following physiochemical properties:
Proton nuclear magnetic resonance spectrum (δ ppm in D₂O):
0.87 (3H, d, J = 6Hz),
0.9 - 1.9 (9H, m),
2.58 (2H, t, J = 7Hz),
2.99 (2H, t, J = 7Hz), 3.06 (6H, s),
3.51 (2H, t, J = 7Hz), 3.68 (1H, m),
3.82 (3H, s), 4.21 (2H, t, J = 7Hz),
6.9 - 7.3 (3H, m)
MS [(M-Cℓ)⁺]: 363

| HRMS (C₂₁H₃₅N₂O₃): | |
|---|---|
| Theoretical value: | 363.26477 |
| Measured value: | 363.26505 |

### Example 17 (Esterification and Amidation, and Conversion into Non-toxic Salt)

A solution of 2.5 g of 3,4-methylenedioxycinnamic acid, 2.8 mℓ of diethyl chlorophosphate and 2.7 mℓ of triethylamine, mixed in 100 mℓ of methylene chloride was stirred for 1 hour at room temperature. Next, under ice cooling, 3 mℓ of N-(2-dimethylaminoethyl)-trans-4-methylcyclohexylamine (Example 14) and 0.5 g of 4-dimethylaminopyridine were added to the solution, and the solution was further stirred for 4 hours at room temperature.

After reaction, the reaction solution was washed five times with 200 mℓ of an aqueous sodium hydrogencarbonate and dried over magnesium sulfate. Thereafter, the solvent was removed in vacuo. The product obtained was recrystallized from methylene chloride/ether, yielding 1.86 g of N-(2-dimethylaminoethyl)-N-(trans-4-methylcyclohexyl)-3,4-methylenedioxycinnamamide (a compound of the present invention) as white crystal, which had the following physiochemical properties:
Proton nuclear magnetic resonance spectrum (δ ppm in CDCℓ₃):
0.89 (3H, d, J = 6Hz),
0.9 - 1.8 (9H, m),
2.32 (6H, s), 2.44 (2H, t, J = 7Hz),
3.43 (2H, t, J = 7Hz), 3.69 (1H, m),
6.00 (2H, s),
6.66 (1H, d, J = 16Hz),
6.78 (1H, d, J = 8Hz),
6.98 (1H, d, J = 8Hz), 7.02 (1H, s),
7.56 (1H, d, J = 16Hz)
MS [(M+H)⁺]: 375

The compound described above was reacted in the ordinary method, converting it into N-(2-dimethylaminoethyl)-N-(trans-4-methylcyclohexyl)-3,4-methylenedioxycinnamamide hydrochloride, which had the following physiochemical properties:
Proton nuclear magnetic resonance spectrum (δ ppm in D₂O):
0.88 (3H, d, J = 6Hz),
0.9 - 1.9 (9H, m),
2.88 (2H, t, J = 7Hz), 3.02 (6H, s),
3.68 (1H, m), 3.81 (2H, t, J = 7Hz),
6.02 (1H, s),
6.63 (1H, d, J = 16Hz),
6.75 (1H, d, J = 8Hz),
6.9 - 7.1 (2H, m),
7.50 (1H, d, J = 16Hz)
MS [(M-Cℓ)⁺]: 359

| HRMS (C₂₁H₃₁N₂O₃): | |
|---|---|
| Theoretical value: | 359.23347 |
| Measured value: | 359.23256 |

### Example 18 (Esterification and Amidation, and Hydrolysis and Conversion into Non-toxic Salt)

Using 1 g of N-(2-dimethylaminoethyl)-N-(trans-4-methylcyclohexyl)-3,4-methylenedioxycinnamamide (Example 17), 60 mℓ of methanol, and 0.05 g of 10% palladium-carbon, a reaction similar to that conducted in Example 12 was carried out. As a result, 0.91 g of N-(2-dimethylaminoethyl)-N-(trans-4-methylcyclohexyl)-3-(3,4-methylenedioxyphenyl)propionamide (a compound of the present invention) was obtained as a colorless oil, which had the following physiochemical properties:
Proton nuclear magnetic resonance spectrum (δ ppm in CDCℓ₃):
0.86 (3H, d, J = 6Hz),
0.9 - 1.8 (9H, m),
2.30 (6H, s), 2.38 (2H, t, J = 7Hz),
2.46 (2H, t, J = 7Hz),
2.85 (2H, t, J = 7Hz),
3.39 (2H, t, J = 7Hz), 3,41 (1H, m),
6.70 (2H, s), 6.6 - 6.8 (3H, m)
MS [(M+H)⁺]: 361
The compound described above was reacted in the ordinary method, converting it into N-(2-dimethylaminoethyl)-N-(trans-4-methylcyclohexyl)-3-(3,4-methylenedioxyphenyl)propionamide hydrochloride which had the following physiochemical properties:
Proton nuclear magnetic resonance spectrum (δ ppm in D₂O):
0.86 (3H, d, J = 6Hz),
0.9 - 1.9 (9H, m),
2.44 (2H, t, J = 7Hz),
2.59 (2H, t, J = 7Hz),
2.99 (2H, t, J = 7Hz), 3.04 (6H, s),
3.43 (1H, m), 4.11 (2H, t, J = 7Hz),
6.92 (2H, s), 6.9 - 7.1 (3H, m)
MS [(M-Cℓ)⁺]: 361

| HRMS (C₂₁H₃₃N₂O₃): | |
|---|---|
| Theoretical value: | 361.24912 |
| Measured value: | 361.24707 |

### Example 19 (Esterification and Amidation, and Hydrolysis and Conversion into Non-toxic Salt)

Using 2.7 g of 4-acetoxycinnamic acid derived from 4-hydroxycinnamic acid by the acetylation thereof, 2.8 mℓ of diethyl chlorophosphate, 2.7 mℓ of triethylamine, 100 mℓ of methylene chloride, 3 mℓ of N-(2-dimethylaminoethyl)-trans-4-methylcyclohexylamine (Example 14), 0.5 g of 4-dimethylaminopyridine, 100 mℓ of methanol, and 3 g of potassium carbonate, a reaction similar to that conducted in Example 11 was carried out. As a result, 1.79 g of N-(2-dimethylaminoethyl)-N-(trans-4-methylcyclohexyl)-4-hydroxycinnamamide (a compound of the present invention) was as white crystal, which had the following physiochemical properties:
Proton nuclear magnetic resonance spectrum (δ ppm in CDCℓ₃):
0.88 (3H, d, J = 6Hz),
0.9 - 1.9 (9H, m),
2.37 (6H, s), 2.52 (2H, t, J = 7Hz),
4.42 (2H, t, J = 7Hz), 4.69 (1H, m),
6.65 (1H, d, J = 16Hz),
6.7 - 6.9 (2H, m),
7.27 (2H, d, J = 8Hz),
7.56 (2H, d, J = 7Hz),
MS [(M+H)⁺] 331

| HRMS (C₂₀H₃₁N₂O₂): | |
|---|---|
| Theoretical value: | 331.23855 |
| Measured value: | 331.23849 |

The compound described above was reacted in the ordinary method, converting it into N-(2-dimethylaminoethyl)-N-(trans-4-methylcyclohexyl)-4-hydroxycinnamamide hydrochloride, which had the following physiochemical properties:
Proton nuclear magnetic resonance spectrum (δ ppm in D₂O):
0.88 (3H, d, J = 6Hz),
0.9 - 1.9 (9H, m),
2.79 (2H, t, J = 7Hz), 3.06 (6H, s),
4.59 (2H, t, J = 7Hz), 4.70 (1H, m),
6.62 (1H, d, J = 16Hz),
6.7 - 6.9 (2H, m),
7.21 (2H, d, J = 8 Hz),
7.46 (1H, d, J = 16Hz)
MS [(M-Cℓ)⁺]: 331

| HRMS (C₂₀H₃₁N₂O₂): | |
|---|---|
| Theoretical value: | 331.23855 |
| Measured value: | 331.23989 |

### Example 20 (Esterification and Amidation, and Hydrolysis and Conversion into Non-toxic Salt)

Using 2.2g of 4-acetoxy-3-methoxybenzoic acid derived from vanillic acid by the acetylation thereof, 2.8 mℓ of diethyl chlorophosphate, 2.7 mℓ of triethylamine, 100 mℓ of methylene chloride, 3 mℓ of N-(2-dimethylaminoethyl)-trans-4-methylcyclohexylamine (Example 14), and 0.5 g of 4-dimethylaminopyridine, a reaction similar to that conducted in Example 11 was carried out. As a result, 0.85 g of N-(2-dimethylaminoethyl)-N-(trans-4-methylcyclohexyl)-vanilloylamide (a compound of the present invention) was obtained as a pale yellow oil, which had the following physiochemical properties:
Proton nuclear magnetic resonance spectrum (δ ppm in CDCℓ₃):
0.88 (3H, d, J = 6Hz),
0.9 - 1.9 (9H, m),
2.04 (6H, s), 2.26 (2H, t, J = 7Hz),
3.42 (2H, t, J = 7Hz), 3.59 (1H, m),
3.70 (3H, s), 6.8 - 7.1 (3H, m)
MS [(M+H)⁺]: 335

| HRMS (C₁₉H₃₁N₂O₃): | |
|---|---|
| Theoretical value: | 335.23347 |
| Measured value: | 335.23381 |

The compound described above was reacted in the ordinary method, converting it into N-(2-dimethylaminoethyl)-N-(trans-4-methylcyclohexyl)vanilloylamide hydrochloride, which had the following physiochemical properties:
Proton nuclear magnetic resonance spectrum (δ ppm in D₂O):
0.88 (3H, d, J = 6Hz),
0.9 - 1.9 (9H, m),
2.42 (2H, t, J = 7Hz), 3.05 (6H, s),
3.67 (2H, t, J = 7Hz), 3.58 (1H, m),
3.71 (3H, s), 6.9 - 7.1 (3H, m)
MS [(M-Cℓ)⁺]: 335

| HRMS (C₁₉H₃₁N₂O₃): | |
|---|---|
| Theoretical value: | 335.23347 |
| Measured value: | 335.23324 |

### Example 21 (Esterification and Amidation, and Hydrolysis and Conversion into Non-toxic Salt)

Using 2.9 g of 4-acetoxy-3,5-dimethyoxycinnamic acid derived from 3,5-dimethoxy-4-hydroxycinnamic acid by the acetylation thereof, 2.8 mℓ of diethyl chlorophosphate, 2.7 mℓ of triethylamine, 100 mℓ of methylene chloride, 3 mℓ of N-(2-dimethylaminoethyl)-trans-4-methylcyclohexylamine (Example 14), and 0.5 g of 4-dimethylaminopyridine, a reaction similar to that conducted in Example 11 was carried out. As a result, 1.95 g of N-(2-dimethylaminoethyl)-N-(trans-4-methylcyclohexyl)-3,5-dimethoxy-4-hydroxycinnamamide (a compound of the present invention) was obtained as a pale yellow oil, which had the following physiochemical properties:
Proton nuclear magnetic resonance spectrum (δ ppm in CDCℓ₃):
0.93 (3H, d, J = 6Hz),
0.9 - 1.9 (9H, m), 2.33 (6H, s),
2.42 (2H, t, J = 7Hz),
3.42 (2H, t, J = 7Hz), 3.49 (1H, m),
3.90 (6H, s),
6.64 (1H, d, J = 16Hz),
6.6 - 6.9 (2H, br),
7.56 (1H, d, J = 16Hz)
MS [(M+H)⁺]: 391

| HRMS (C₂₂H₃₅N₂O₄): | |
|---|---|
| Theoretical value: | 391.25968 |
| Measured value: | 391.25944 |

The compound described above was reacted in the ordinary method, converting it into N-(2-dimethylaminoethyl)-N-(trans-4-methylcyclohexyl)-3,5-dimethoxy-4-hydroxycinnamamide hydrochloride, which had the following physiochemical properties:
Proton nuclear magnetic resonance spectrum (δ ppm in D₂O):
0.91 (3H, d, J = 6Hz),
0.9 - 1.9 (9H, m),
2.56 (2H, t, J = 7Hz), 3.04 (6H, s),
3.61 (2H, t, J = 7Hz), 3.47 (1H, m),
3.89 (6H, s),
6.58 (1H, d, J = 16Hz),
6.5 - 6.9 (2H, m),
7.50 (2H, d, J = 16Hz),
MS [(M-Cℓ)⁺]: 391

| HRMS (C₂₂H₃₅N₂O₄): | |
|---|---|
| Theoretical value: | 391.25968 |
| Measured value: | 391.26010 |

### Example 22 (Esterification and Amidation, and Conversion into Non-toxic Salt)

Using 1.3 g of 3,4-dichlorocinammic acid, 1.4 mℓ of diethyl chlorophosphate, 1.4 mℓ of triethylamine, 60 mℓ of methylene chloride, 1.4 mℓ of N-(2-dimethylaminoethyl)-trans-4-methylcyclohexylamine (Example 14), and 0.25 g of 4-dimethylaminopyridine, a reaction similar to that conducted in Example 17 was carried out. As a result, 2.45 g of N-(2-dimethylaminoethyl)-N-(trans-4-methylcyclohexyl)-3,4-dichlorocinnamamide (a compound of the present invention) was obtained as white crystal, which had the following physiochemical properties:
Proton nuclear magnetic resonance spectrum (δ ppm in CDCℓ₃):
0.90 (3H, d, J = 6Hz),
1.0 - 1.9 (9H, m), 2.32 (6H, s),
2.42 (2H, t, J = 7Hz),
3.41 (2H, t, J = 7Hz), 3.69 (1H, m),
6.88 (1H, d, J = 16Hz),
7.5 - 7.8 (4H, m)
MS [(M+H)⁺]: 383

| HRMS (C₂₀H₂₉N₂OCℓ₂): | |
|---|---|
| Theoretical value: | 383.16569 |
| Measured value: | 383.16537 |

The compound described above was reacted in the ordinary method, converting it into N-(2-dimethylaminoethyl)-N-(trans-4-methylcyclohexyl)-3,4-dichlorocinnamamide hydrochloride, which had the following physiochemical properties:
Proton nuclear magnetic resonance spectrum (δ ppm in D₂O):
0.90 (3H, d, J = 6Hz),
1.0 - 1.9 (9H, m),
2.58 (2H, t, J = 7Hz), 3.08 (6H, s),
3.69 (1H, m), 3.91 (2H, t, J = 7Hz),
6.79 (1H, d, J = 16Hz),
7.3 - 7.6 (4H, m)
MS [(M-Cℓ)⁺]: 383

| HRMS (C₂₁H₃₀N₂OF₃): | |
|---|---|
| Theoretical value: | 383.16569 |
| Measured value: | 383.16537 |

### Example 23 (Esterification and Amidation, and Conversion into Non-toxic Salt)

Using 1.1 g of 3-chlorocinnamic acid, 1.4 mℓ of diethyl chlorophosphate, 1.4 mℓ of triethylamine, 60 mℓ of methylene chloride, 1.4 mℓ of N-(2-dimethylaminoethyl)-trans-4-methylcyclohexylamine (Example 14), and 0.25 g of 4-dimethylaminopyridine, a reaction similar to that conducted in Example 17 was carried out. As a result, 2.06 g of N-(2-dimethylaminoethyl)-N-(trans-4-methylcyclohexyl)-3-chlorocinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
0.9 - 1.9 (9H, m), 2.32 (6H, s),
2.44 (2H, t, J = 7Hz),
3.40 (2H, t, J = 7Hz), 3.71 (1H, m),
6.86 (1H, d, J = 16Hz),
7.3 - 7.5 (4H, m),
7.59 (1H, d, J = 16Hz)
MS [(M+H)⁺]: 349

| HRMS (C₂₀H₃₀N₂OCℓ): | |
|---|---|
| Theoretical value: | 349.20467 |
| Measured value: | 349.20501 |

The compound described above was reacted in the ordinary method, converting it into N-(2-dimethylaminoethyl)-N-(trans-4-methylcyclohexyl)-3-chlorocinnamamide hydrochloride, which had the following physiochemical properties:
Proton nuclear magnetic resonance spectrum (δ ppm in D₂O):
0.88 (3H, d, J = 6Hz),
0.9 - 1.9 (9H, m),
2.59 (2H, t, J = 7Hz), 3.06 (6H, s),
3.71 (2H, t, J = 7Hz), 3.70 (1H, m),
6.80 (1H, d, J = 16Hz),
7.1 - 7.4 (4H, m),
7.51 (1H, d, J = 16Hz)
MS [(M-Cℓ)⁺]: 349

| HRMS (C₂₀H₃₀N₂OCℓ): | |
|---|---|
| Theoretical value: | 349.20467 |
| Measured value: | 349.20435 |

### Example 24 (Esterification and Amidation, and Conversion into Non-toxic Salt)

Using 1.1 g of 2-chlorocinnamic acid, 1.4 mℓ of diethyl chlorophosphate, 1.4 mℓ of triethylamine, 60 mℓ of methylene chloride, 1.4 mℓ of N-(2-dimethylaminoethyl)-trans-4-methylcyclohexylamine (Example 14), and 0.25 g of 4-dimethylaminopyridine, a reaction similar to that conducted in Example 17 was carried out. As a result, 1.94 g of N-(2-dimethylaminoethyl)-N-(trans-4-methylcyclohexyl)-2-chlorocinnamamide (a compound of the present invention) was obtained as white crystal, which had the following physiochemical properties:
Proton nuclear magnetic resonance spectrum (δ ppm in CDCℓ₃):
0.90 (3H, d, J = 6Hz),
0.9 - 1.9 (9H, m), 2.32 (6H, s),
2.44 (2H, t, J = 7Hz),
3.44 (2H, t, J = 7Hz), 3.72 (1H, m),
6.85 (1H, d, J = 16Hz),
7.2 - 7.6 (4H, m),
7.88 (1H, d, J = 16Hz)
MS [(M+H)⁺]: 349

| HRMS (C₂₀H₃₀N₂OCℓ): | |
|---|---|
| Theoretical value: | 349.20467 |
| Measured value: | 349.20489 |

The compound described above was reacted in the ordinary method, converting it into N-(2-dimethylaminoethyl)-N-(trans-4-methylcyclohexyl)-2-chlorocinnamamide hydrochloride, which had the following physiochemical properties:
Proton nuclear magnetic resonance spectrum (δ ppm in D₂O):
0.90 (3H, d, J = 6Hz),
0.9 - 1.9 (9H, m),
2.58 (2H, t, J = 7Hz), 3.08 (6H, s),
3.71 (1H, m) 3.76 (2H, t, J = 7Hz),
6.79 (1H, d, J = 16Hz),
7.1 - 7.5 (4H, m),
7.79 (1H, d, J = 16Hz)
MS [(M-Cℓ)⁺]: 349

| HRMS (C₂₀H₃₀N₂OCℓ): | |
|---|---|
| Theoretical value: | 349.20467 |
| Measured value: | 349.20462 |

### Example 25 (Esterification and Amidation, and Conversion into Non-toxic Salt)

Using 1.0 g of 2-fluorocinnamic acid, 1.4 mℓ of diethyl chlorophosphate, 1.4 mℓ of triethylamine, 60 mℓ of methylene chloride, 1.4 mℓ of N-(2-dimethylaminoethyl)-trans-4-methylcyclohexylamine (Example 14), and 0.25 g of 4-dimethylaminopyridine, a reaction similar to that conducted in Example 17 was carried out. As a result, 1.69 g of N-(2-dimethylaminoethyl)-N-(trans-4-methylcyclohexyl)-2-fluorocinnamamide (a compound of the present invention) was obtained as white crystal, which had the following physiochemical properties:
Proton nuclear magnetic resonance spectrum (δ ppm in CDCℓ₃):
0.86 (3H, d, J = 6Hz)
0.9 - 1.9 (9H, m), 2.32 (6H, m),
2.45 (2H, t, J = 8Hz),
3.39 (2H, t, J = 8Hz), 3.71 (1H, m),
7.00 (1H, d, J = 16Hz),
7.0 - 7.6 (4H, m),
7.70 (1H, d, J = 16Hz)
MS [(M+H)⁺]: 333

| HRMS (C₂₀H₃₀N₂OF): | |
|---|---|
| Theoretical value: | 333.23442 |
| Measured value: | 333.23440 |

The compound described above was reacted in the ordinary method, converting it into N-(2-dimethylaminoethyl)-N-(trans-4-methylcyclohexyl)-2-fluorocinnamamide hydrochloride, which had the following physiochemical properties:
Proton nuclear magnetic resonance spectrum (δ ppm in D₂O):
0.86 (3H, d, J = 6Hz),
0.9 - 1.9 (9H, m),
2.63 (2H, t, J = 8Hz), 3.09 (6H, s),
3.62 (2H, t, J = 8Hz), 3.70 (1H, m),
6.96 (1H, d, J = 16Hz),
7.0 - 7.6 (4H, m),
7.63 (1H, d, J = 16Hz)
MS [(M-Cℓ)⁺]: 333

| HRMS (C₂₀H₃₀N₂OF): | |
|---|---|
| Theoretical value: | 333.23422 |
| Measured value: | 333.23435 |

### Example 26 (Esterification and Amidation, and Conversion into Non-toxic Salt)

Using 1.1 g of 3,4-difluorocinnamic acid, 1.4 mℓ of diethyl chlorophosphate, 1.4 mℓ of triethylamine, 60 mℓ of methylene chloride, 1.4 mℓ of N-(2-dimethylaminoethyl)-trans-4-methylcyclohexylamine (Example 14), and 0.25 g of 4-dimethylaminopyridine, a reaction similar to that conducted in Example 17 was carried out. As a result, 1.74 g of N-(2-dimethylaminoethyl)-N-(trans-4-methylcyclohexyl)-3,4-difluorocinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
0.9 - 1.9 (9H, m), 2.32 (6H, s),
2.40 (2H, t, J = 8Hz),
3.40 (2H, t, J = 8Hz), 3.72 (1H, m),
6.78 (1H, d, J = 16Hz),
7.1 - 7.4 (3H, m)
7.56 (1H, d, J = 16Hz)
MS [(M+H)⁺]: 351

| HRMS (C₂₀H₂₉N₂OF₂) | |
|---|---|
| Theoretical value: | 351.22488 |
| Measured value: | 351.22480 |

The compound described above was reacted in the ordinary method, converting it into N-(2-dimethylaminoethyl)-N-(trans-4-methylcyclohexyl)-3,4-difluorocinnamamide hydrochloride, which had the following physiochemical properties:
Proton nuclear magnetic resonance spectrum (δ ppm in D₂O):
0.88 (3H, d, J = 6Hz),
0.9 - 1.9 (9H, m),
2.59 (2H, t, J = 8Hz), 3.08 (6H, s),
3.68 (2H, t, J = 8Hz), 3.71 (1H, m),
6.71 (1H, d, J = 16Hz),
7.0 - 7.4 (3H, m),
7.50 (1H, d, J = 16Hz)
MS [(M-Cℓ)⁺]: 351

| HRMS (C₂₀H₂₉N₂OF₂): | |
|---|---|
| Theoretical value: | 351.22480 |
| Measured value: | 351.22534 |

### Example 27 (Esterification and Amidation, and Conversion into Non-toxic Salt)

Using 1.3 g of 3-trifluoromethylcinnamic acid, 1.4 mℓ of diethyl chlorophosphate, 1.4 mℓ of triethylamine, 60 mℓ of methylene chloride, 1.4 mℓ of N-(2-dimethylaminoethyl)-trans-4-methylcyclohexylamine (Example 14), and 0.25 g of 4-dimethylaminopyridine, a reaction similar to that conducted in Example 17 was carried out. As a result, 2.25 g of N-(2-dimethylaminoethyl)-N-(trans-4-methylcyclohexyl)-3-trifluoromethylcinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
0.9 - 1.9 (9H, m), 2.31 (6H, s),
2.40 (2H, t, J = 7Hz),
3.38 (2H, t, J = 7Hz), 3.72 (1H, m),
6.85 (1H, d, J = 16Hz),
7.3 - 7.7 (5H, m),
MS [(M+H)⁺]: 383

| HRMS (C₂₁H₃₀N₂OF₃): | |
|---|---|
| Theoretical value: | 383.23102 |
| Measured value: | 383.23148 |

The compound described above was reacted in the ordinary method, converting it into N-(2-dimethylaminoethyl)-N-(trans-4-methylcyclohexyl)-3-trifluoromethylcinnamamide hydrochloride, which had the following physiochemical properties:
Proton nuclear magnetic resonance spectrum (δ ppm in D₂O):
0.88 (3H, d, J = 6Hz),
0.9 - 1.9 (9H, m),
2.59 (2H, t, J = 7Hz), 3.09 (6H, s),
3.71 (2H, t, J = 7Hz), 3.68 (1H, m),
6.76 (1H, d, J = 16Hz),
7.2 - 7.6 (5H, m),
MS [(M-Cℓ)⁺]: 383

| HRMS (C₂₁H₃₀N₂OF₃): | |
|---|---|
| Theoretical value: | 383.23102 |
| Measured value: | 383.23144 |

### Example 28 (Esterification and Amidation, and Conversion into Non-toxic Salt)

Using 2.0 g of 3-nitrocinnamic acid, 1.9 mℓ of diethyl chlorophosphate, 3.5 mℓ of triethylamine, 60 mℓ of methylene chloride, 2.3 mℓ of N-(2-dimethylaminoethyl)-trans-4-methylcyclohexylamine (Example 14), and 0.04 g of 4-dimethylaminopyridine, a reaction similar to that conducted in Example 17 was conducted. As a result, 1.54 g of N-(2-dimethylaminoethyl)-N-(trans-4-methylcyclohexyl)-3-nitrocinnamamide (a compound of the present invention) was obtained as white crystal, which had the following physiochemical properties:
Proton nuclear magnetic resonance spectrum (δ ppm in CDCℓ₃):
0.90 (3H, d, J = 6Hz),
1.0 - 1.9 (9H, m), 2.34 (6H, s),
2.47 (2H, t, J = 8Hz),
3.42 (2H, t, J = 8Hz), 3.74 (1H, m),
7.06 (1H, d, J = 16Hz),
7.5 - 7.9 (5H, m),
MS [(M+H)⁺]: 360

The compound described above was reacted in the ordinary method, converting it into N-(2-dimethylaminoethyl)-N-(trans-4-methylcyclohexyl)-3-nitrocinnamamide hydrochloride, which had the following physiochemical properties:
Proton nuclear magnetic resonance spectrum (δ ppm in D₂O):
0.89 (3H, d, J = 6Hz),
1.0 - 1.9 (9H, m),
2.61 (2H, t, J = 8Hz), 3.08 (6H, s),
3.68 (2H, t, J = 8Hz), 3.72 (1H, m),
7.00 (1H, d, J = 16Hz),
7.4 - 7.9 (5H, m),
MS [(M-Cℓ)⁺]: 360

| HRMS (C₂₀H₃₀N₃O₃): | |
|---|---|
| Theoretical value: | 360.22872 |
| Measured value: | 360.22674 |

### Example 29 (Esterification and Amidation, and Conversion into Non-toxic Salt)

Using 2.3 g of 4-bromocinnammic acid, 1.9 mℓ of diethyl chlorophosphate, 3.5 mℓ of triethylamine, 60 mℓ of methylene chloride, 2.3 mℓ of N-(2-dimethylaminoethyl)-trans-4-methylcyclohexylamine (Example 14), and 0.04 g of 4-dimethylaminopyridine, a reaction similar to that conducted in Example 17 was carried out. As a resutl, 2.24 g of N-(2-dimethylaminoethyl)-N-(trans-4-methylcyclohexyl)-4-bromocinnamamide (a compound of the present invention) was obtained as white crystal, which had the following physiochemical properties:
Proton nuclear magnetic resonance spectrum (δ ppm in CDCℓ₃):
0.89 (3H, d, J = 7Hz),
0.9 - 1.9 (9H, m), 2.31 (6H, m),
2.39 (2H, t, J = 8Hz),
3.40 (2H, t, J = 8Hz), 3.72 (1H, m),
6.83 (1H, d, J = 16Hz),
7.35 (2H, d, J = 9Hz),
7.48 (2H, d, J = 8Hz),
7.58 (1H, d, J = 16Hz)
MS [(M+H)⁺]: 392
The compound described above was reacted in the ordinary method, converting it into N-(2-dimethylaminoethyl)-N-(trans-4-methylcyclohexyl)-4-bromocinnamamide hydrochloride, which had the following physiochemical properties:
Proton nuclear magnetic resonance spectrum (δ ppm in D₂O):
0.88 (3H, d, J = 6Hz),
0.9 - 1.9 (9H, m),
2.58 (2H, t, J = 8Hz), 3.09 (6H, s),
3.69 (2H, t, J = 8Hz), 3.71 (1H, m),
6.79 (1H, d, J = 16Hz),
7.29 (2H, d, J = 9Hz),
7.41 (2H, d, J = 8Hz),
7.51 (1H, d, J = 16Hz)
MS [(M-Cℓ)⁺]: 393

| HRMS (C₂₀H₃₀N₂OBr): | |
|---|---|
| Theoretical value: | 393.15415 |
| Measured value: | 393.15365 |

### Example 30 (Esterification and Amidation, and Conversion into Non-toxic Salt)

Using 2.0 g of 4-acetylaminocinnamic acid derived from 4-aminocinnamic acid by the acetylation thereof, 1.9 mℓ of diethyl chlorophosphate, 3.5 mℓ of triethylamine, 60 mℓ of methylene chloride, 2.3 mℓ of N-(2-dimethylaminoethyl)-trans-4-methylcyclohexylamine (Example 14), and 0,04 g of 4-dimethylaminopyridine, a reaction similar to that conducted in Example 17 was carried out. As a result, 2.08 g of N-(2-dimethylaminoethyl)-N-(trans-4-methylcyclohexyl)-4-acetylaminocinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
0.9 - 1.9 (9H, m), 2.18 (3H, s),
2.31 (6H, s),
2.45 (2H, t, J = 8Hz),
3.43 (2H, t, J = 7Hz), 3.74 (1H, m),
6.77 (1H, d, J = 16Hz),
7.40 (2H, d, J = 9Hz),
7.55 (2H, d, J = 9Hz),
7.60 (1H, d, J = 16Hz)
MS [(M+H)⁺]: 372

| HRMS (C₂₂H₃₄N₃O₂): | |
|---|---|
| Theoretical value: | 372.26510 |
| Measured value: | 372.26588 |

The compound described above was reacted in the ordinary method, converting it into N-(2-dimethylaminoethyl)-N-(trans-4-methylcyclohexyl)-4-acetylaminocinnamamide hydrochloride, which had the following physiochemical properties:
Proton nuclear magnetic resonance spectrum (δ ppm in D₂O):
0.88 (3H, d, J = 6Hz),
0.9 - 1.9 (9H, m), 2.15 (3H, s),
2.60 (2H, t, J = 8Hz),
3.09 (6H, s), 3.69 (2H, t, J = 7Hz),
3.73 (1H, m),
6.71 (1H, d, J = 16Hz),
7.31 (2H, d, J = 9Hz),
7.49 (2H, d, J = 9Hz),
7.52 (1H, d, J = 16Hz)
MS [(M-Cℓ)⁺]: 372

| HRMS (C₂₂H₃₄N₃O₂): | |
|---|---|
| Theoretical value: | 372.26510 |
| Measured value: | 372.26524 |

### Example 31 (Esterification and Amidation, and Conversion into Non-toxic Salt)

Using 2.7 g of 3,4-dimethoxycinnamic acid, 2.8 mℓ of diethyl chlorophosphate, 2.7 mℓ of triethylamine, 100 mℓ of methylene chloride, 3.0 mℓ of N-(2-dimethylaminoethyl)-trans-4-methylcyclohexylamine (Example 14), and 0.5 g of 4-dimethylaminopyridine, a reaction similar to that conducted in Example 17 was carried out. As a result, 1.53 g of N-(2-dimethylaminoethyl)-N-(trans-4-methylcyclohexyl)-3,4-dimethoxycinnamamide (a compound of the present invention) was obtained as white crystal, which had the following physiochemical properties:
Proton nuclear magnetic resonance spectrum (δ ppm in CDCℓ₃):
0.90 (3H, d, J = 6Hz),
1.0 - 1.8 (9H, m), 2.33 (6H, s),
2.46 (2H, t, J = 7Hz),
3.45 (2H, t, J = 7Hz), 3.72 (1H, m),
3.91 (6H, s),
6.72 (1H, d, J = 16Hz),
6.84 (1H, d, J = 8Hz),
7.0 - 7.2 (2H, m),
7.61 (1H, d, J = 16Hz)
MS [(M+H)⁺]: 375
The compound described above was reacted in the ordinary method, converting it into N-(2-dimethylaminoethyl)-N-(trans-4-methylcyclohexyl)-3,4-dimethoxycinnamamide hydrochloride, which had the following physiochemical properties:
Proton nuclear magnetic resonance spectrum (δ ppm in D₂O):
0.89 (3H, d, J = 6Hz),
0.9 - 1.9 (9H, m),
2.88 (2H, t, J = 7Hz), 3.05 (6H,s),
3.66 (2H, t, J = 7Hz), 3.71 (1H, m),
3.92 (6H, s),
6.69 (1H, d, J = 16Hz),
6.83 (1H, d, J = 8Hz),
6.9 - 7.2 (2H, m),
7.58 (1H, d, J = 16Hz)
MS [(M-Cℓ)⁺]: 375

| HRMS (C₂₂H₃₅N₂O₃): | |
|---|---|
| Theoretical value: | 375.26477 |
| Measured value: | 375.26414 |

### Example 32 (Esterification and Amidation, and Hydrolysis and Conversion into Non-toxic Salt)

Using 3.4 g of 3,4-diacetoxycinnamic acid derived from caffeic acid by the acetylation thereof, 2.8 mℓ of diethyl chlorophosphate, 2.7 mℓ of triethylamine, 100 mℓ of methylene chloride, 3.0 mℓ of N-(2-dimethylaminoethyl)-trans-4-methylcyclohexylamine (Example 14), and 0.5 g of 4-dimethylaminopyridine, a reaction similar to that conducted in Example 17 was carried out. As a result, 0.68 g of N-(2-dimethylaminoethyl)-N-(trans-4-methylcyclohexyl)-3,4-diacetoxycinnamamide (a compound of the present invention) was obtained as pale yellow crystal, which had the following physiochemical properties:
Proton nuclear magnetic resonance spectrum (δ ppm in CDCℓ₃):
0.90 (3H, d, J = 6Hz),
0.9 - 1.8 (9H, m), 2.30 (6H, s),
2.32 (6H, s),
2.41 (2H, t, J = 7Hz),
3.38 (2H, t, J = 7Hz), 3.71 (1H, m),
6.71 (1H, d, J = 16Hz),
7.1 - 7.4 (3H, m),
7.52 (1H, d, J = 16Hz)
MS [(M+H)⁺]: 431

| HRMS (C₂₄H₃₅N₂O₅): | |
|---|---|
| Theoretical value: | 431.25460 |
| Measured value: | 431.25496 |

The compound described above was reacted in the ordinary method, converting it into N-(2-dimethylaminoethyl)-N-(trans-4-methylcyclohexyl)-3,4-dihydroxycinnamamide hydrochloride, which had the following physiochemical properties:
Proton nuclear magnetic resonance spectrum (δ ppm in D₂O):
0.88 (3H, d, J = 6Hz),
0.9 - 1.8 (9H, m),
2.59 (2H, t, J = 7Hz), 3.08 (6H, s),
3.62 (2H, t, J = 7Hz), 3.71 (1H, m),
6.69 (1H, d, J = 16Hz),
7.0 - 7.4 (3H, m),
7.46 (1H, d, J = 16Hz)
MS [(M-Cℓ)⁺]: 347

| HRMS (C₂₀H₃₁N₂O₃): | |
|---|---|
| Theoretical value: | 347.23347 |
| Measured value: | 347.23169 |

### Example 33 (Esterification and Amidation and Conversion into Non-toxic Salt)

Using 2.0 g of 4-dimethylaminocinnamic acid, 1.9 mℓ of diethyl chlorophosphate, 3.5 mℓ of triethylamine, 60 mℓ of methylene chloride, 2.3 mℓ of N-(2-dimethylaminoethyl)-trans-4-methylcyclohexylamine (Example 14), and 0.04 g of 4-dimethylaminopyridine, a reaction similar to that conducted in Example 17 was carried out. As a result, 2.1 g of N-(2-dimethylaminoethyl)-N-(trans-4-methylcyclohexyl)-4-dimethylaminocinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
0.9 - 1.9 (9H, m), 2.30 (6H, s),
2.32 (6H, s),
2.41 (2H, t, J = 7Hz),
3.39 (2H, t, J = 7Hz), 3.71 (1H, m),
6.84 (1H, d, J = 16Hz),
7.41 (2H, d, J = 9Hz),
7.53 (2H, d, J = 9Hz),
7.66 (1H, d, J = 16Hz), 6.38 (1H, m)
MS [(M+H)⁺]: 358
The compound described above was reacted in the ordinary method, converting it into N-(2-dimethylaminoethyl)-N-(trans-4-methylcyclohexyl)-4-dimethylaminocinnamamide dihydrochloride, which had the following physiochemical properties:
Proton nuclear magnetic resonance spectrum (δ ppm in D₂O):
0.89 (3H, d, J = 6Hz),
0.9 - 1.9 (9H, m), 2.31 (6H, s),
2.59 (2H, t, J = 7Hz),
3.08 (6H, s), 3.61 (2H, t, J = 7Hz),
3.70 (1H, m),
6.79 (1H, d, J = 16Hz),
7.36 (2H, d, J = 9Hz),
7.42 (2H, d, J = 9Hz),
7.59 (1H, d, J = 16Hz)
MS [(M+H-2HCℓ)⁺]: 358

| HRMS (C₂₂H₃₆N₃O): | |
|---|---|
| Theoretical value: | 358.28584 |
| Measured value: | 358.28564 |

### Example 34 (Esterification and Amidation, and Hydrolysis and Conversion into Non-toxic Salt)

Using 2.3 g of (4-acetoxy-3-methoxyphenyl)acetic acid derived from homovanillic acid by the acetylation thereof, 2.8 mℓ of diethyl chlorophosphate, 2.7 mℓ of triethylamine, 100 mℓ of methylene chloride, 3 mℓ of N-(2-dimethylaminoethyl)-trans-4-methylcyclohexylamine (Example 14), 0.5 g of 4-dimethylaminopyridine, 100 mℓ of methanol, and 3 g of potassium carbonate, a reaction similar to that conducted in Example 11 was carried out. As a result, 1.44 g of N-(2-dimethylaminoethyl)-N-(trans-4-methylcyclohexyl)-2-(4-hydroxy-3-methoxyphenyl)acetamide (a compound of the present invention) was obtained as pale yellow crystal, which had the following physiochemical properties:
Proton nuclear magnetic resonance spectrum (δ ppm in CDCℓ₃):
0.87 (3H, d, J = 6Hz),
0.9 - 2.0 (9H, m), 2.25 (6H, s),
2.48 (2H, t, J = 7Hz),
2.78 (2H, t, J = 7Hz), 3.31 (1H, m),
3.64 (2H, s), 3.87 (3H, s),
6.6 - 6.9 (3H, m)
MS [(M+H)⁺]: 349
The compound described above was reacted in the ordinary method, converting it into N-(2-dimethylaminoethyl)-N-(trans-4-methylcyclohexyl)-2-(4-hydroxy-3-methoxyphenyl)acetamide hydrochloride, which had the following physiochemical properties:
0.86 (3H, d, J = 6Hz),
0.9 - 2.0 (9H, m),
2.61 (2H, t, J = 7Hz),
2.99 (2H, t, J = 7Hz), 3.06 (6H, s),
3.32 (1H, m), 3.72 (2H, s),
3.86 (3H, s), 6.6 - 7.0 (3H, m)
MS [(M-Cℓ)⁺]: 349

| HRMS (C₂₀H₃₃N₂O₃): | |
|---|---|
| Theoretical value: | 349.24912 |
| Measured value: | 349.24792 |

### Example 35 (Knoevenagel Condensation)

3.1 g of 2-fluoro-p-anisaldehyde and 4.2 g of malonic acid were dissolved in 80 mℓ of pyridine. Then, 0.5 mℓ of piperidine was added to the solution, and the solution was reacted at 80°C for 4 hours, After reaction, the reaction solution was poured into 500 mℓ of ice-water, and 200 moℓ of 2N hydrochloric acid was added to the solution. The solution was extracted three times with 100 mℓ of ethyl acetate. The organic layer obtained was washed three times with 2N hydrochloric acid and twice with an aqueous sodium chloride solution, and was dried over magnesium sulfate. Thereafter, the solvent was removed in vacuo. The product obtained was recrystallized from methylene chloride/ether, yielding 2.7 g of 3-fluoro-4-methoxycinnamic acid as white crystal, which had the following physiochemical properties:
Proton nuclear magnetic resonance spectrum (δ ppm in DMSO-d₆):
3.88 (3H, s),
6.39 (1H, d, J = 16Hz),
7.13 (1H, t, J = 9Hz),
7.4 - 7.5 (2H, m),
7.59 (1H, d, J = 16Hz)
MS (M⁺): 196

### Example 36 (Esterification and Amidation, and Conversion into Non-toxic Salt)

Using 2.0 g of 3-fluoro-4-methoxycinnamic acid (Example 35), 2.8 mℓ of diethyl chlorophosphate, 2.7 mℓ of triethylamine, 100 mℓ of methylene chloride, 3 mℓ of N-(2-dimethylaminoethyl)-trans-4-methylcyclohexylamine (Example 14), and 0.5 g of 4-dimethylaminopyridine, a reaction similar to that performed in Example 17 was carried out. As a result, 2.21 g of N-(2-dimethylaminoethyl)-N-(trans-4-methylcyclohexyl)-3-fluoro-4-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
0.9 - 1.9 (9H, m), 2.32 (6H, s),
2.44 (2H, t, J = 8Hz),
3.39 (2H, t, J = 8Hz), 3.72 (1H, m),
3.91 (3H, s),
6.70 (1H, d, J = 16Hz),
6.90 (1H, t, J = 9Hz),
7.2 - 7.4 (2H, m),
7.57 (1H, d, J = 16Hz)
MS [(M+H)⁺]: 363
The compound described above was reacted in the ordinary method, converting it into N-(2-dimethylaminoethyl)-N-(trans-4-methylcyclohexyl)-3-fluoro-4-methoxycinnamamide hydrochloride, which had the following physiochemical properties:
Proton nuclear magnetic resonance spectrum (δ ppm in D₂O):
0.89 (3H, d, J = 6Hz),
0.9 - 1.9 (9H, m),
2.63 (2H, t, J = 8Hz), 3.09 (6H, s),
3.71 (2H, t, J = 8Hz), 3.72 (1H, m),
3.90 (3H, s),
6.62 (1H, d, J = 16Hz),
6.82 (1H, t, J = 9Hz),
7.1 - 7.3 (2H, m),
7.50 (1H, d, J = 16Hz)
MS [(M-Cℓ)⁺]: 363

| HRMS (C₂₁H₃₂N₂O₂F): | |
|---|---|
| Theoretical value: | 363.24478 |
| Measured value: | 363.24352 |

### Example 37 (Knoevenagel Condensation)

Using 4.8 g of 2-chloro-4-(2-tetrahydropyranyloxy)benzaldehyde derived from 2-chloro-4-hydroxybenzaldehyde by the introduction of tetrahydropyranyl group thereinto, 4.2 g of malonic acid, 80 mℓ of pyridine, and 0.5 mℓ of piperidine, a reaction similar to that performed in Example 35 was carried out. As a result, 3.1 g of 2-chloro-4-(2-tetrahydropyranyloxy)cinnamic acid was obtained as crystal, which had the following physiochemical properties:
Proton nuclear magnetic resonance spectrum (δ ppm in CDCℓ₃):
1.5 - 2.0 (6H, m), 3.5 - 3.9 (2H, m),
5.47 (1H, m),
6.32 (1H, d, J = 16Hz),
6.95 (1H, dd, J = 6, 3Hz),
7.14 (1H, d, J = 2Hz),
7.58 (1H, d, J = 9Hz),
8.12 (1H, d, J = 16Hz)
MS [(M+H)⁺]: 283

| HRMS (C₁₄H₁₆N₄O): | |
|---|---|
| Theoretical value: | 283.07371 |
| Measured value: | 283.07440 |

### Example 38 (Esterification and Amidation, and Conversion into Non-toxic Salt)

Using 1.7 g of 2-chloro-4-(2-tetrahydropyranyloxy)cinnamic acid, 1.4 mℓ of diethyl chlorophosphate, 1.4 mℓ of triethylamine, 60 mℓ of methylene chloride, 1.4 mℓ of N-(2-dimethylaminoethyl)-trans-4-methylcyclohexylamine (Example 14), and 0.25 g of 4-dimethylaminopyridine, a reaction similar to that performed in Example 17 was carried out. As a result, 3.01 g of N-(2-dimethylaminoethyl)-N-(trans-4-methylcyclohexyl)-2-chloro-4-(2-tetrahydropyranyloxy)cinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
0.9 - 2.0 (15H, m), 2.31 (6H, s),
2.41 (2H, t, J = 7Hz),
3.41 (2H, t, J = 7Hz),
3.5 - 3.9 (3H, m),
6.98 (1H, d, J = 16Hz),
7.01 (1H, dd, J = 6, 3Hz),
7.12 (1H, d, J = 2Hz),
7.60 (1H, d, J = 9Hz),
8.10 (1H, d, J = 16Hz)
MS [(M+H)⁺]: 437
The compound described above was reacted in the ordinary method, converting it into N-(2-dimethylaminoethyl)-N-(trans-4-methylcyclohexyl)-2-chloro-4-hydroxycinnamamide hydrochloride, which had the following physiochemical properties:
Proton nuclear magnetic resonance spectrum (δ ppm in D₂O):
0.88 (3H, d, J = 6Hz),
0.9 - 2.0 (9H, m),
2.59 (2H, t, J = 7Hz), 3.08 (6H, s),
3.62 (2H, t, J = 7Hz), 3.61 (1H, m),
6.91 (1H, d, J = 16Hz),
7.0 - 7.2 (2H, m),
7.56 (1H, d, J = 9Hz),
8.01 (1H, d, J = 16Hz)
MS [(M-Cℓ)⁺]: 365

| HRMS (C₂₀H₃₀N₂O₂Cℓ): | |
|---|---|
| Theoretical value: | 365.19958 |
| Measured value: | 365.19868 |

### Example 39 (Reduction)

0.1 g of 20% palladium hydroxide-carbon was added to a solution of 3g of N-(1-phenylethyl)-4-methyl-1-cyclohexylamine disclosed in the literature [G. Knupp, and A.W. Frahm, J. Chem, Research, 164-165 (1981)] in 50 mℓ of methanol. The solution was vigorously stirred for 18 hours at room temperature under normal-pressure hydrogen gas. After reaction, the catalyst was filtered out, and the solvent was removed in vacuo from the filtrate, yielding 1.5 g of cis-4-methyl-1-cyclohexylamine as a colorless oil, which had the following physiochemical properties:
Proton nuclear magnetic resonance spectrum (δ ppm in CDCℓ₃):
0.90 (3H, d, J = 8Hz),
1.3 - 2.1 (9H, m), 3.49 (1H, m)
MS (M⁺): 113

| HRMS (C₇H₁₅N): | |
|---|---|
| Theoretical value: | 113.12042 |
| Measured value: | 113.12002 |

### Example 40 (Esterification and Amidation)

A solution prepared by mixing 2.5 g of 4-hydroxy-3-methoxycinnamic acid, 2.8 mℓ of diethyl chlorophosphate, 2.7 mℓ of triethyleneamine, and 100 mℓ of methylene chloride was stirred for 1 hour at room temperature. Then, 1.8 mℓ of cis-4-methyl-1-cyclohexylamine (Example 39) and 0.5 g of 4-dimethylaminopyridine were added to the solution, and the solution was further stirred for 4 hours at room temperature.

After reaction, the reaction solution was washed five times with 200 mℓ of an aqueous sodium hydrogencarbonate solution and dried over magnesium sulfate. Thereafter, the solvent was removed in vacuo. The product obtained was recrystallized from methylene chloride/hexane, yielding 2.8 g of N-(cis-4-methylcyclohexyl)-4-acetoxy-3-methoxycinnamamide (a compound of the present invention) as white crystal, which had the following physiochemical properties:
Proton nuclear magnetic resonance spectrum (δ ppm in CDCℓ₃):
0.93 (3H, d, J = 7Hz),
1.0 - 1.8 (9H, m),
2.32 (3H, s), 3.86 (3H, s),
4.12 (1H, br), 5.69 (1H, br),
6.30 (1H, d, J = 16Hz),
7.0 - 7.2 (3H, m),
7.53 (1H, d, J = 16Hz)
IRₘₐₓ (KBr, cm⁻¹): 3284, 2938, 1765, 1655
MS (M⁺): 331

| Elemental analysis (C₁₉H₂₅NO₄): | | | |
|---|---|---|---|
| Theoretical value; | C: 68.86, | H: 7.60, | N: 4.23 |
| Measured value; | C: 68.83, | H: 7.49, | N: 4.24 |

### Example 41 (Hydrolysis and Conversion into Non-toxic Salt)

5 g of sodium carbonate was added to a solution of 2.8 g of N-(cis-4-methylcyclohexyl)-4-acetoxy-3-methoxycinnamamide (Example 40) in 100 mℓ of methanol. The solution was stirred for 2 hours at room temperature. After reaction, 2N hydrochloric acid was added to the reaction solution, acidifying the solution. The solution was extracted three times with 90 mℓ of ethyl acetate. The organic layer obtained was washed twice with an aqueous sodium chloride solution and dried over magnesium sulfate. Thereafter, the solvent was removed in vacuo. The product obtained was recrystallized from ethyl acetate/hexane, yielding 2.2 g of N-(cis-4-methylcyclohexyl)-4-hydroxy-3-methoxycinnamamide (a compound of the present invention) as white crystal, which had the following physiochemical properties:
Melting point: 135.8 - 136.9°C
Proton nuclear magnetic resonance spectrum (δ ppm in DMSO-d₆):
0.90 (3H, d, J = 6Hz),
1.2 - 1.7 (9H, m), 3.82 (3H, s),
3.91 (1H, br), 5.73 (1H, s),
6.53 (1H, d, J = 16Hz),
6.76 (1H, d, J = 8Hz),
6.95 (1H, dd, J = 6, 2Hz),
7.10 (1H, d, J = 2Hz),
7.26 (1H, d, J = 16Hz),
7.74 (1H, d, J = 8Hz), 9.35 (1H, s)
IRₘₐₓ (KBr, cm⁻¹): 3254, 2954, 1668
MS (M⁺): 289

| Elemental analysis (C₁₇H₂₃NO₃): | | | |
|---|---|---|---|
| Theoretical value; | C: 70.56, | H: 8.01, | N: 4.84 |
| Measured value; | C: 70.61, | H: 7.97, | N: 5.01 |

The compound described above was reacted in the ordinary method, converting it into sodium N-(cis-4-methylcyclohexyl)-3-methoxy-4-oxidocinnamamide, which had the following physiochemical properties:
Proton nuclear magnetic resonance spectrum (δ ppm in D₂O):
0.86 (3H, d, J = 6Hz),
1.0 - 1.9 (9H, m),
3.81 (3H, s), 3.89 (1H, m),
6.51 (1H, d, J = 16Hz),
6.38 (1H, d, J = 16Hz),
6.64 (1H, d, J = 8Hz),
6.91 (1H, d, J = 8Hz), 7.05 (1H, s),
7.42 (1H, d, J - 16Hz)
MS [(M+Na)⁺]: 334, [(M+H)⁺]: 312

| HRMS (C₁₇H₂₂NO₃Na₂): | |
|---|---|
| Theoretical value: | 334.13957 |
| Measured value: | 334.13870 |

### Example 42 (Alkylation)

6.2 g of potassium carbonate and 4.2 mℓ of 1-bromo-2-chloroethane were added to a solution of 8.67 g of N-(cis-4-methylcyclohexyl)-4-hydroxy-3-methoxycinnamamide (Example 41) in 200 mℓ of methyl-isobutylketone. The solution was reacted for 10 hours, while it was refluxed. After reaction, the reaction solution was allowed to cool, added with 200 mℓ of methylene chloride and 200 mℓ of an aqueous sodium chloride solution, and subjected to extraction. The aqueous layer was extracted twice with 100 mℓ of methylene chloride. The organic layer obtained was washed twice with an aqueous sodium chloride solution and dried over magnesium sulfate. Thereafter, the solvent was removed in vacuo. The product obtained was recrystallized from methylene chloride/ether, yielding 8.76 g of N-(cis-4-methylcyclohexyl)-4-(2-chloroethoxy)-3-methoxycinnamamide (a compound of the present invention) as white crystal, which had the following physiochemical properties:
Proton nuclear magnetic resonance spectrum (δ ppm in CDCℓ₃):
0.88 (3H, d, J = 6Hz),
1.0 - 1.8 (9H, m),
3.81 (2H, t, J = 7Hz), 3.87 (3H, s),
4.06 (1H, m), 4.25 (2H, t, J = 7Hz),
5.49 (1H, m),
6.26 (1H, d, J = 16Hz),
6.81 (1H, d, J = 8Hz),
7.0 - 7.2 (2H, m),
7.47 (1H, d, J=16Hz)
MS [(M+H)⁺]: 352

### Example 43 (Amination and Conversion into Non-toxic Salt)

40 mℓ of a 50% aqueous dimethylamine solution was added to a solution of 2 g of N-(cis-4-methylcyclohexyl)-4-(2-chloroethoxy)-3-methoxycinnamamide (Example 42) in 40 mℓ of methylisobutylketone. The solution was reacted for 18 hours by heating heated to 110°C, in a closed system. After reaction, the reaction solution was added with 200 mℓ of an aqueous sodium chloride solution and 100 mℓ of methylene chloride, and subjected to extraction. The aqueous layer was extracted twice with 100 mℓ of methylene chloride. The organic layer obtained was washed twice with an aqueous sodium chloride solution and dried over magnesium sulfate. Thereafter, the solvent was removed in vacuo. The product obtained was recrystallized from methylene chloride/ether, yielding 1.6 g of N-(cis-4-methylcyclohexyl)-4-(2-dimethylaminoethoxy)-3-methoxycinnamamide (a compound of the present invention) as white crystal, which had the following physiochemical properties:
Proton nuclear magnetic resonance spectrum (δ ppm in CDCℓ₃):
0.91 (3H, d, J = 6Hz),
1.0 - 1.8 (9H, m), 2.36 (6H, s),
2.73 (2H, t, J = 7Hz), 3.88 (3H, s),
4.09 (2H, t, J = 7Hz), 4.10 (1H, m),
5.74 (1H, m),
6.28 (1H, d, J = 16Hz),
6.83 (1H, d, J = 8Hz),
7.0 - 7.2 (2H, m),
7.49 (1H, d, J = 16Hz)
MS [(M+H)⁺]: 361

| HRMS (C₂₁H₃₃N₂O₃): | |
|---|---|
| Theoretical value: | 361.24943 |
| Measured value: | 361.24912 |

The compound described above was reacted in the ordinary method, converting it into N-(cis-4-methylcyclohexyl)-4-(2-dimethylaminoethoxy)-3-methoxycinnamamide hydrochloride, which had the following physiochemical properties:
Proton nuclear magnetic resonance spectrum (δ ppm in D₂O):
1.11 (3H, d, J = 6Hz),
1.3 - 2.1 (9H, m), 3.22 (6H, s),
3.78 (2H, t, J = 7Hz),
3.88 (3H, s), 4.21 (1H, br),
4.45 (2H, t, J = 7Hz)
6.92 (1H, d, J = 16Hz),
7.04 (1H, d, J = 8Hz), 7.19 (1H, s),
7.23 (1H, d, J = 8Hz),
7.61 (1H, d, J = 16Hz)
MS [(M-Cℓ)⁺]: 361

| HRMS (C₂₁H₃₃N₂O₃): | |
|---|---|
| Theoretical value: | 361.24912 |
| Measured value: | 361.24868 |

### Example 44 (Reduction and Conversion into Non-toxic Salt)

0.05 g of 10% palladium-carbon was added to a solution of 1 g of N-(cis-4-methylcyclohexyl)-4-(2-dimethylaminoethoxy)-3-methoxycinnamamide (Example 43) in 50 mℓ of methanol. The solution was vigorously stirred for 16 hours under normal-pressure hydrogen gas. After reaction, the catalyst was filtered out, and the solvent was removed in vacuo from the filtrate. The product obtained was recrystallized from methylene chloride/ether, yielding 0.93 g of N-(cis-4-methylcyclohexyl)-3-[4-(2-dimethylaminoethoxy)-3-methoxyphenyl]propionamide (a compound of the present invention) as white crystal, which had the following physiochemical properties:
Proton nuclear magnetic resonance spectrum (δ ppm in CDCℓ₃):
0.85 (3H, d, J = 6Hz),
1.2 - 1.6 (9H, m), 2.34 (6H, s),
2.39 (2H, t, J = 7Hz),
2.74 (2H, t, J = 7Hz),
2.87 (2H, t, J = 7Hz), 3.83 (3H, s),
3.98 (1H, m), 4.05 (2H, t, J = 7Hz),
5.41 (1H, m), 6.7 - 6.9 (3H, m)
MS [(M+H)⁺]: 363

| HRMS (C₂₁H₃₅N₂O₃): | |
|---|---|
| Theoretical value: | 363.26462 |
| Measured value: | 363.26477 |

The compound described above was reacted in the ordinary method, converting it into N-(cis-4-methylcyclohexyl)-3-[4-(2-dimethylaminoethoxy)-3-methoxyphenyl]propionamide hydrochloride, which had the following physiochemical properties:
Proton nuclear magnetic resonance spectrum (δ ppm in D₂O):
1.04 (3H, d, J = 6Hz),
1.3 - 2.1 (9H, m),
2.51 (2H, t, J = 7Hz),
2.90 (2H, t, J = 7Hz), 3.18 (6H, s),
3.69 (2H, t, J = 7Hz), 3.88 (3H, s),
4.42 (2H, t, J = 7Hz),
7.2 - 7.4 (3H, m)
MS [(M-Cℓ)⁺]: 363

| HRMS (C₂₁H₃₅N₂O₃): | |
|---|---|
| Theoretical value: | 363.26477 |
| Measured value: | 363.26427 |

### Example 45 (Esterification and Amidation)

Using 6.5 mℓ of diethyl chlorophosphate, 18 mℓ of triethylamine, 5.2 g of dimethylaminoglycine hydrochloride, 5 g of cis-4-methylcyclohexylammonium chloride, and 0.15 g of 4-dimethylaminopyridine, a reaction similar to that performed in Example 13 was carried out. As a result, 4.8 g of N-(cis-4-methylcyclohexyl)-2-dimethylaminoacetamide was obtained as a pale yellow oil, which had the following physiochemical properties:
Proton nuclear magnetic resonance spectrum (δ ppm in CDCℓ₃):
0.92 (3H, d, J = 6Hz),
1.1 - 1.7 (9H, m), 2.30 (6H, s),
2.93 (2H, s), 4.0 (1H, m)
MS [(M+H)⁺]: 199

| HRMS (C₁₁H₂₃N₂O): | |
|---|---|
| Theoretical value: | 199.18104 |
| Measured value: | 199.18272 |

### Example 46

Using 4.8 g of N-(cis-4-methylcyclohexyl)-2-dimethylaminoacetamide (Example 45), 1.8 g of lithium aluminum hydride, 150 mℓ of THF, and 5 g of sodium sulfate decahydrate, a reaction similar to that performed in Example 14 was carried out. As a result, 2.9 g of N-(2-dimethylaminoethyl)-cis-4-methylcyclohexylamine was obtained as a colorless oil, which had the following physiochemical properties:
Proton nuclear magnetic resonance spectrum (δ ppm in CDCℓ₃):
0.89 (3H, d, J = 6Hz),
1.2 - 1.8 (9H, m), 2.24 (6H, s),
2.38 (2H, t, J = 7Hz), 2.59 (1H, m),
2.64 (2H, t, J = 7Hz)
MS [(M+H)⁺]: 185

| HRMS (C₁₁H₂₅N₂): | |
|---|---|
| Theoretical value: | 185.20177 |
| Measured value: | 185.20242 |

### Example 47 (Esterification and Amidation, and Hydrolysis and Conversion into Non-toxic Salt)

Using 2.5 g of 4-acetoxy-3-methoxycinnamic acid, 2.8 mℓ of diethyl chlorophosphate, 2.7 mℓ of triethylamine, 100 mℓ of methylene chloride, 3 mℓ of N-(2-dimethylaminoethyl)-cis-4-methylcyclohexylamine (Example 110), and 0.5 g of 4-dimethylaminopyridine, a reaction similar to that conducted in Example 11 was carried out. As a result, 1.6 g of N-(2-dimethylaminoethyl)-N-(cis-4-methylcyclohexyl)-4-hydroxy-3-methoxycinnamamide (a compound of the present invention) was obtained as white crystal, which had the following physiochemical properties:
Proton nuclear magnetic resonance spectrum (δ ppm in CDCℓ₃):
0.98 (3H, d, J = 7Hz),
1.0 - 2.0 (9H, m), 2.31 (6H, s),
2.42 (2H, t, J = 7Hz),
3.43 (2H, t, J = 7Hz), 3.82 (3H, s),
4.01 (1H, m),
6.79 (1H, d, J = 16 Hz),
7.0 - 7.4 (3H, m),
7.36 (1H, d, J = 16Hz)
MS [(M+H)⁺]: 361

| HRMS (C₂₁H₃₂N₂O₃): | |
|---|---|
| Theoretical value: | 361.24912 |
| Measured value: | 361.24910 |

The compound described above was reacted in the ordinary method, converting it into N-(2-dimethylaminoethyl)-N-(cis-4-methylcyclohexyl)-4-hydroxy-3-methoxycinnamamide hydrochloride, which had the following physiochemical properties:
Proton nuclear magnetic resonance spectrum (δ ppm in D₂O):
1.00 (3H, d, J = 7Hz),
1.0 - 2.0 (9H, m),
2.93 (2H, t, J = 7Hz), 3.05 (6H, s),
3.82 (3H, s), 3.91 (2H, t, J = 7Hz),
4.02 (1H, m).
6.75 (1H, d, J = 16Hz),
6.9 - 7.3 (3H, m),
7.31 (1H, d, J = 16Hz)
MS [(M-Cℓ)⁺]: 361

| HRMS (C₂₁H₃₃N₂O₃): | |
|---|---|
| Theoretical value: | 361.24912 |
| Measured value: | 361.24969 |

### Example 48 (Reduction and Conversion into Non-toxic Salt)

Using a solution of 1 g of N-(2-dimethylaminoethyl)-N-(cis-4-methylcyclohexyl)-4-hydroxy-3-methoxycinnamamide (Example 47) in 60 mℓ of methanol, and 0.05 g of 10% palladium-carbon, a reaction similar to that conducted in Example 12 was carried out. As a result, 0.91 g of N-(2-dimethylaminoethyl)-N-(cis-4-methylcyclohexyl)-3-(4-hydroxy-3-methoxyphenyl)propionamide (a compound of the present invention) was obtained as a colorless oil, which had the following physiochemical properties:
Proton nuclear magnetic resonance spectrum (δ ppm in CDCℓ₃):
0.98 (3H, d, J = 6Hz),
1.0 - 2.0 (9H, m), 2.32 (6H, s),
2.39 (2H, t, J = 7Hz), 2.59 (2H, t, J = 7Hz),
2.72 (2H, t, J = 7Hz),
3.45 (2H, t, J = 7Hz), 3.83 (3H, s),
4.01 (1H, m), 7.0 - 7.4 (3H, m)
MS [(M+H)⁺]: 363

| HRMS (C₂₁H₃₅N₂O₃): | |
|---|---|
| Theoretical value: | 363.26477 |
| Measured value: | 363.26462 |

The compound described above was reacted in the ordinary method, converting it into N-(2-dimethylaminoethyl)-N-(cis-4-methylcyclohexyl)-3-(4-hydroxy-3-methoxyphenyl)propionamide hydrochloride, which had the following physiochemical properties:
Proton nuclear magnetic resonance spectrum (δ ppm in D₂O):
0.99 (3H, d, J = 6Hz),
1.0 - 2.0 (9H, m),
2.53 (2H, t, J = 7Hz),
2.89 (2H, t, J = 7Hz), 3.09 (6H, s),
3.51 (2H, t, J = 7Hz), 3.82 (3H, s),
4.02 (1H, m), 4.41 (2H, t, J = 7Hz),
7.0 - 7.4 (3H, m)
MS [(M-Cℓ)⁺]: 363

| HRMS (C₂₁H₃₅N₂O₃): | |
|---|---|
| Theoretical value: | 363.26477 |
| Measured value: | 363.26444 |

### Example 49 (Esterification and Amidation)

Using 4g of 4-(4-benzyloxy-3-methoxyphenyl)-1,3-butadiene-1-carboxylic acid disclosed in the literature [R. Bäckström, E. Honkanen, A. Pippuri, P. Kairisalo, J. Pystynen, K. Heinola, E. Nissinen, I. Linden, P.T. Männistö, S. Kaakkola, and P. Pohto, J. Med. Chem., 32, 841-848 (1989)], 3 mℓ of diethyl chlorophosphate, 3.5 mℓ of triethylamine, 150 mℓ of methylene chloride, 4 mℓ of N-(2-dimethylaminoethyl)-trans-4-methylcyclohexylamine (Example 14), and 0.5 g of 4-dimethylaminopyridine, a reaction similar to that conducted in Example 17 was carried out. As a result, 3.81 g of N-(2-dimethylaminoethyl)-N-(trans-4-methylcyclohexyl)-4-(4-benzyloxy-3-methoxyphenyl)-1,3-butadiene-1-carboxamide (a compound of the present invention) was obtained as a pale yellow oil, which had following physiochemical properties:
Proton nuclear magnetic resonance spectrum (δ ppm in CDCℓ₃):
0.87 (3H, d, J = 6Hz),
0.9 - 1.9 (9H, m),
2.29 (6H, s), 2.42 (2H, t, J = 7Hz),
3.68 (1H, m), 3.88 (3H, s),
5.23 (2H, s),
6.23 (1H, d, J = 16 Hz),
6.7 - 7.3 (5H, m),
7.43 (1H, d, J = 16Hz)
MS [(M+H)⁺]: 477

| HRMS (C₃₀H₄₁N₂O₃): | |
|---|---|
| Theoretical value: | 477.31172 |
| Measured value: | 477.31212 |

### Example 50 (Reduction and Conversion into Non-toxic Salt)

3.81 g of N-(2-dimethylaminoethyl)-N-(trans-4-methylcyclohexyl)-4-(4-benzyloxy-3-methoxyphenyl)-1,3-butadiene-1-carboxamide (Example 49) was dissolved in 100 mℓ of methanol. 0.2 g of 10% palladium-carbon was added to the solution. The solution was vigorously stirred at room temperature under normal-pressure hydrogen gas. After 18 hours, the reaction was ceased, and the catalyst was filtered out. The solvent was removed in vacuo from the filtrate, yielding 2.1 g of N-(2-dimethylaminoethyl)-N-(trans-4-methylcyclohexyl)-5-(4-hydroxy-3-methoxyphenyl)valeramide (a compound of the present invention) as a pale yellow oil, which had the following physiochemical properties:
Proton nuclear magnetic resonance spectrum (δ ppm in CDCℓ₃):
0.87 (3H, d, J = 6Hz),
0.9 - 2.2 (13H, m), 2.28 (6H, s),
2.39 (2H, t, J = 7Hz),
2.44 (2H, t, J = 7Hz),
2.68 (2H, T, J = 7Hz),
2.99 (2H, t, J = 7Hz),
3.48 (2H, t, J = 7Hz), 3.68 (1H, m),
3.86 (3H, s), 6.6 - 6.9 (3H, m)
MS [(M+H)⁺]: 391

| HRMS (C₂₃H₃₉N₂O₃): | |
|---|---|
| Theoretical value: | 391.29607 |
| Measured value: | 391.29576 |

The compound described above was reacted in the ordinary method, converting it into N-(2-dimethylaminoethyl)-N-(trans-4-methylcyclohexyl)-5-(4-hydroxy-3-methoxyphenyl)valeramide hydrochloride, which had the following physiochemical properties:
Proton nuclear magnetic resonance spectrum (δ ppm in D₂O):
0.87 (3H, d, J = 6Hz),
0.9 - 2.2 (13H, m),
2.51 (2H, t, J = 7Hz),
2.99 (2H, t, J = 7Hz), 3.08 (6H, s),
3.41 (2H, t, J = 7Hz), 3.68 (1H, m),
3.71 (2H, t, J = 7Hz), 3.85 (3H, s),
6.5 - 6.9 (3H, m)
MS [(M-Cℓ)⁺]: 391

| HRMS (C₂₃H₃₉N₂O₃): | |
|---|---|
| Theoretical value: | 391.29607 |
| Measured value: | 391.29606 |

### Examples 51 (Esterification and Amidation)

Using 4g of 4-(4-benzyloxy-3-methoxyphenyl)-1,3-butadiene-1-carboxylic acid, 3 mℓ of diethyl chlorophosphate, 3.5 mℓ of triethylamine, 150 mℓ of methylene chloride, 2.2 g of trans-4-methylcyclohexylammonium chloride, and 0.5 g of 4-dimethylaminopyridine, a reaction similar to that conducted in Example 17 was carried out. As a result, 3.62 g of N-(trans-4-methylcyclohexyl)-4-(4-benzyloxy-3-methoxyphenyl)-1,3-butadiene-1-carboxamide (a compound of the present invention) was obtained as a pale yellowhish brown oil, which had the following physiochemical properties:
Proton nuclear magnetic resonance spectrum (δ ppm in CDCℓ₃):
0.85 (3H, d, J = 6Hz),
0.9 - 2.0 (9H, m), 3.64 (1H, m),
3.86 (3H, s), 5.24 (2H, s),
5.44 (1H, m),
6.21 (1H, d, J = 16Hz),
6.7 - 7.2 (10H, m),
7.47 (1H, d, J =16Hz)
MS [(M+H)⁺]: 406

| HRMS (C₂₆H₃₂NO₃): | |
|---|---|
| Theoretical value: | 406.23822 |
| Measured value: | 406.23851 |

### Example 52 (Reduction)

3.62 g of N-(trans-4-methylcyclohexyl)-4-(4-benzyloxy-3-methoxyphenyl)-1,3-butadiene-1-carboxamide (Example 51) was dissolved in 150 mℓ of methanol. 0.2 g of 10% palladium-carbon was added to the solution. The solution was stirred for 16 hours under normal-pressure hydrogen gas. After reaction, the catalyst was filtered out, and the solvent was removed in vacuo from the filtrate, yielding 2.3 g of N-(trans-4-methylcyclohexyl)-5-(4-hydroxy-3-methoxyphenyl)valeramide (a compound of the present invention) as a pale yellow oil, which had the following physiochemical properties:
Proton nuclear magnetic resonance spectrum (δ ppm in CDCℓ₃):
0.87 (3H, d, J = 6Hz),
0.9 - 2.0 (13H, m),
2.08 (2H, t, J = 7Hz),
2.49 (2H, t, J = 7Hz), 3.62 (1H, m),
3.87 (3H, s), 5.21 (1H, m),
6.6 - 6.9 (3H, m)
MS [(M+H)⁺]: 320

| HRMS (C₁₉H₃₀NO₃): | |
|---|---|
| Theoretical value: | 320.22257 |
| Measured value: | 320.22214 |

### Example 53 (Alkylation)

Using 2.3 g of N-(trans-4-methylcyclohexyl)-5-(4-hydroxy-3-methoxyphenyl)valeramide (Example 118), 100 mℓ of methylisobutylketone, 5 g of potassium carbonate, and 4 mℓ of 1-bromo-2-chloroethane, a reaction similar to that conducted in Example 42 was carried out. As a result, 2.1 g of N-(trans-4-methylcyclohexyl)-5-[4-(2-chloroethoxy)-3-methoxyphenyl]valeramide (a compound of the present invention) was obtained as a pale yellow oil, which had the following physiochemical properties:
Proton nuclear magnetic resonance spectrum (δ ppm in CDCℓ₃):
0.87 (3H, d, J = 6Hz),
0.9 - 2.0 (11H, m),
2.07 (2H, t, J = 7Hz),
2.50 (2H, t, J = 7Hz), 3.66 (1H, m),
3.81 (2H t, J = 7Hz), 3.85 (3H, s),
4.06 (2H, t, J = 7Hz), 5.24 (1H, m),
6.6 - 6.9 (3H, m)
MS [(M+H)⁺]: 382

| HRMS (C₂₁H₃₃NO₃Cℓ): | |
|---|---|
| Theoretical value: | 382.21490 |
| Measured value: | 382.21480 |

### Example 54 (Amination and Conversion into Non-toxic Salt)

40 mℓ of a 50% aqueous dimethylamine solution was added to a solution of 2.1 g of N-(trans-4-methylcyclohexyl)-5-[4-(2-chloroethoxy)-3-methoxyphenyl]valeramide (Example 53) in 50 mℓ of methylisobutylketone. The solution was reacted for 18 hours by heating to 110°C in a closed system. After reaction, 200 mℓ of an aqueous sodium chloride solution and 100 mℓ of methylene chloride were added to the reaction solution, which was then subjected to extraction. The aqueous layer was extracted twice with 100 mℓ of methylene chloride. The organic layer obtained was washed twice with an aqueous sodium chloride solution and dried over magnesium sulfate. Thereafter, the solvent was removed in vacuo, yielding 1.54 g of N-(trans-4-methylcyclohexyl)-5-[4-(2-dimethylaminoethoxy)-3-methoxyphenyl]valeramide (a compound of the present invention) as a pale yellow oil, which had the following physiochemical properties:
Proton nuclear magnetic resonance spectrum (δ ppm in CDCℓ₃):
0.86 (3H, d, J = 6Hz),
0.9 - 2.0 (11H, m),
2.08 (2H, t, J = 7Hz), 2.34 (6H, s),
2.37 (2H, t, J = 7Hz),
2.48 (2H, t, J = 7Hz), 3.68 (1H, m),
3.86 (3H, s), 4.05 (2H, t, J = 7Hz),
5.24 (1H, m), 6.6 - 6.9 (3H, m)
MS [(M+H)⁺]: 391

| HRMS (C₂₃H₃₉N₂O₃): | |
|---|---|
| Theoretical value: | 391.29607 |
| Measured value: | 391.29669 |

The compound described above was reacted in the ordinary method, converting it into N-(trans-4-methylcyclohexyl)-5-[4-(2-dimethylaminoethoxy)-3-methoxyphenyl]valeramide hydrochloride, which had the following physiochemical properties:
Proton nuclear magnetic resonance spectrum (δ ppm in D₂O):
0.84 (3H, d, J = 6Hz),
0.9 - 2.0 (11H, m),
2.06 (2H, t, J = 7Hz),
2.36 (2H, t, J = 7Hz), 3.66 (1H, m),
3.72 (2H, t, J = 7Hz), 3.84 (3H, s),
4.03 (2H, t, J = 7Hz),
4.19 (2H, t, J = 7Hz),
6.5 - 6.9 (3H, m)
MS [(M-Cℓ)⁺]: 391

| HRMS (C₂₃H₃₉N₂O₃): | |
|---|---|
| Theoretical value: | 391.29607 |
| Measured value: | 391.29563 |

### Example 55 (Esterification and Amidation, and Hydrolysis)

A solution of 2.9 g of 4-acetoxy-3,5-dimethoxycin-namic acid derived from 3,5-dimethoxy-4-hydroxycinnamic acid by the acetylation thereof, 2.8 mℓ of diethyl chlorophosphate and 3.9 mℓ of triethylamine, mixed in 60 mℓ of methylene chloride, was stirred for 1 hours at room temperature. Next, 2 g of trans-4-methylcyclohexylammonium chloride and 0.6 g of 4-dimethylaminopiridine were added under ice-cooling to the solution. The solution was reacted for 4 hours at room temperature. Then, the solvent was removed in vacuo, and 100 mℓ of methanol and 5 g of potassium carbonate were added to the residue, and the solution was reacted for 2 hours.

After reaction, 2N hydrochloric acid was added to the reaction solution, acidifying the solution. The solution was extracted three times with 100 mℓ of ethyl acetate. The organic layer obtained was washed twice with an aqueous sodium hydrogencarbonate solution and twice with an aqueous sodium chloride solution, and was dried over magnesium sulfate. Then, the solvent was removed in vacuo, yielding 3.48 g of N-(trans-4-methylcyclohexyl)-3,5-dimethoxy-4-hydroxycinnamamide (a compound of the present invention) as a pale yellow oil, which had the following physiochemical properties:
Proton nuclear magnetic resonance spectrum (δ ppm in DMSO-d₆):
0.86 (3H, d, J = 6Hz)
0.9 - 1.9 (9H, m), 3.53 (1H, m),
3.79 (6H, s),
6.41 (1H, d, J = 16Hz),
6.83 (2H, s),
7.25 (1H, d, J = 16Hz),
7.77 (1H, d, J = 8Hz)
MS (M⁺): 319

### Example 56 (Esterification and Amidation, and Hydrolysis)

Using 2.7 g of 4-acetoxy-3-methoxybenzoic acid derived from vanillic acid by the acetylatin thereof, 2.8 mℓ of diethyl chlorophosphate, 3.9 mℓ of triethylamine, 2 g of trans-4-methylcyclohexylammmonium chloride, 60 mℓ of methylene chloride, 0.6 g of 4-dimethylaminopyridine, 100 mℓ of methanol, and 5 g of potassium carbonate, a reaction similar to that conducted in Example 55 was carried out. As a result, 3.04 g of N-(trans-4-methylcyclohexyl)vanilloylamide (a compound of the present invention) as a pale yellow oil, which had the following physiochemical properties:
Proton nuclear magnetic resonance spectrum (δ ppm in CDCℓ₃):
0.90 (3H, d, J = 6Hz),
0.9 - 2.1 (9H, m), 3.86 (1H, m),
3.93 (3H, s), 5.86 (1H, m),
6.01 (1H, br),
6.87 (1H, d, J = 8Hz),
7.13 (1H, dd, J = 6, 2Hz),
7.43 (1H, d, J = 2Hz)
MS (M⁺): 263

### Example 57 (Esterification and Amidation, and Hydrolysis)

2.7 g of 4-acetoxycinnamic acid derived from p-coumaric acid by the acetylation thereof, 2.8 mℓ of diethyl chlorophosphate, 3.9 mℓ of triethylamine, 2 g of trans-4-methylcyclohexylammmonium chloride, 60 mℓ of methylene chloride, 0.6 g of 4-dimethylaminopyridine, 100 mℓ of methanol, and 5 g of potassium carbonate, a reaction similar to that conducted in Example 121 was carried out. The product obtained was recrystallized from methylene chloride/hexane, yielding 2.83 g of N-(trans-4-methylcyclohexyl)-4-hydroxycinnamamide (a compound of the present invention) as white crystal, which had the following physiochemical properties:
Proton nuclear magnetic resonance spectrum (δ ppm in DMSO-d₆):
0.86 (3H, d, J = 6Hz),
0.9 - 1.9 (9H, m), 4.58 (1H, m),
6.34 (1H, d, J = 16Hz),
6.34 (1H, d, J = 9Hz),
6.76 (2H, d, J=9Hz),
7.26 (1H, d, J = 16Hz),
7.34 (2H, d, J = 9Hz),
7.77 (1H, d, J = 8Hz)
MS (M⁺): 259

### Example 58 (Esterification and Amidation)

Using 2.5 g of 3,4-methylenedioxycinnamic acid, 2.8 mℓ of diethyl chlorophosphate, 3.9 mℓ of triethylamine, 60 mℓ of methylene chloride, 2 g of trans-4-methylcyclohexylammmonium chloride, and 0.6 g of 4-dimethylaminopyridine, a reaction similar to that conducted in Example 17 was carried out. The product obtained was recrystallized from methylene chloride/hexane, yielding 3.11 g of N-(trans-4-methylcyclohexyl)-3,4-methylenedioxycinnamamide (a compound of the present invention) as white crystal, which had the following physiochemical properties:
Proton nuclear magnetic resonance spectrum (δ ppm in DMSO-d₆):
0.86 (3H, d, J = 6Hz),
0.9 - 1.9 (9H, m), 3.52 (1H, m),
6.05 (2H, s), 6.39 (1H, d, J = 16Hz),
6.90 (1H, d, J = 8Hz),
7.01 (1H, d, J = 2Hz),
7.06 (1H, dd, J = 6, 2Hz),
7.26 (1H, d, J = 16Hz)
MS (M⁺): 287

### Example 59 (Esterification and Amidation)

Using 2.7 g of 3,4-dimethoxycinnamic acid, 2.8 mℓ of diethyl chlorophosphate, 3.9 mℓ of triethylamine, 2 g of trans-4-methylcyclohexylammmonium chloride, 60 mℓ of methylene chloride, and 0.6 g of 4-dimethylaminopyridine, a reaction similar to that conducted in Example 17 was carried out. The product obtained was recrystallized from methylene chloride/hexane, yielding 2.83 g of N-(trans-4-methylcyclohexyl)-3,4-dimethoxycinnamamide (a compound of this invention) as white crystal, which had the following physiochemical properties:
Proton nuclear magnetic resonance spectrum (δ ppm in DMSO-d₆):
0.86 (3H, d, J = 6Hz),
0.9 - 1.9 (9H, m),
3.58 (1H, m), 3.78 (3H, s),
3.79 (3H, s),
6.44 (1H, d, J = 16Hz),
6.95 (1H, d, J = 8Hz),
7.11 (1H, dd, J = 6, 2Hz),
7.13 (1H, s),
7.29 (1H, d, J = 16Hz)
MS (M⁺): 303

### Example 60 (Esterification and Amidation)

Using 3.4 g of 3,4-diacetoxycinnamic acid derived from caffeic acid by the acetylation thereof, 2.8 mℓ of diethyl chlorophosphate, 3.9 mℓ of triethylamine, 2 g of trans-4-methylcyclohexylammmonium chloride, 60 mℓ of methylene chloride, and 0.6 g of 4-dimethylaminopyridine, a reaction similar to that conducted in Example 81 was carried out. The product obtained was recrystallized from methylene chloride/hexane, yielding 3.19 g of N-(trans-4-methylcyclohexyl)-3,4-diacetoxycinnamamide (a compound of the present invention) as white crystal, which had the following physiochemical properties:
Proton nuclear magnetic resonance spectrum (δ ppm in CDCℓ₃):
0.85 (3H, d, J = 6Hz),
0.9 - 1.8 (9H, m), 2.30 (6H, s),
3.60 (1H, m), 5.41 (1H, m),
6.80 (1H, d, J = 16Hz),
6.88 (1H, d, J = 8Hz),
7.0 - 7.2 (2H, m),
7.40 (1H, d, J = 16Hz)
MS (M⁺): 359

### Example 61 (Esterification and Amidation, and Hydrolysis)

Using 2.9 g of (4-acetoxy-3-methoxyphenyl)acetic acid derived from homovanillic acid by the acetylation thereof, 2.8 mℓ of diethyl chlorophosphate, 3.9 mℓ of triethylamine, 60 mℓ of methylene chloride, 2 g of trans-4-methylcyclohexylammmonium chloride, 0.6 g of 4-dimethylaminopyridine, 100 mℓ of methanol, and 5 g of potassium carbonate, a reaction similar to that conducted in Example 55 was carried out. The product obtained was recrystallized from methylene chloride/hexane, yielding 1.5 g of N-(trans-4-methylcyclohexyl)-2-(4-hydroxy-3-methoxyphenyl)acetamide (a compound of the present invention) as pale yellowish white crystal, which had the following physiochemical properties:
Proton nuclear magnetic resonance spectrum (δ ppm in CDCℓ₃):
0.84 (3H, d, J = 6Hz),
0.9 - 2.0 (9H, m), 3.46 (2H, s),
3.68 (1H, m), 3.88 (3H, s),
5.19 (1H, m), 6.7 - 6.9 (3H, m)
MS [(M+H)⁺]: 278

| HRMS (C₁₆H₂₄NO₃): | |
|---|---|
| Theoretical value: | 278.17562 |
| Measured value: | 278.17614 |

### Example 62 (Alkylation)

Using 1.5 g of N-(trans-4-methylcyclohexyl)-2-(4-hydroxy-3-methoxyphenyl)acetamide (Example 61), 100 mℓ of methylisobutylketone, 5 g of potassium carbonate, and 4 mℓ of 1-bromo-2-chloroethane, a reaction similar to that conducted in Example 42 was carried out. As a result, 1.38 g of N-(trans-4-methylcyclohexyl)-2-[4-(2-chloroethoxy)-3-methoxyphenyl]acetamide (a compound of the present invention) was obtained as a pale yellowish white crystal, which had the following physiochemical properties:
Proton nuclear magnetic resonance spectrum (δ ppm in CDCℓ₃):
0.85 (3H, d, J = 6Hz),
0.9 - 2.0 (9H, m), 3.48 (2H, s),
3.54 (1H, m), 3.80 (2H, t, J = 7Hz),
3.86 (3H, s), 4.24 (2H, t, J = 7Hz),
5.16 (1H, m), 6.7 - 6.9 (3H, m)
MS [(M+H)⁺]: 340

| HRMS (C₁₈H₂₇NO₃Cℓ): | |
|---|---|
| Theoretical value: | 340.16795 |
| Measured value: | 340.16765 |

### Example 63 (Amidation and Conversion into Non-Toxic) Salt

Using 1.38 g of N-(trans-4-methylcyclohexyl)-2-[4-(2-chloroethoxy)-3-methoxyphenyl]acetamide (Example 128), 50 mℓ of methylisobutylketone, and 40 mℓ of 50% aqueous dimethylamine solution, a reaction similar to that conducted in Example 43 was carried out. As a result, 1.08 g of N-(trans-4-methylcyclohexyl)-2-[4-(2-dimethylaminoethoxy)-3-methoxyphenyl]acetamide (a compound of the present invention) was obtained as pale yellowish white crystal, which had the following physiochemical properties:
Proton nuclear magnetic resonance spectrum (δ ppm in CDCℓ₃):
0.85 (3H, d, J = 6Hz),
0.9 - 1.9 (9H, m), 2.37 (6H, s),
2.41 (2H, t, J = 7Hz), 3.47 (2H, s),
3.64 (1H, m), 3.84 (3H, s),
4.09 (2H, t, J = 7Hz), 5.18 (1H, m),
6.7 - 6.9 (3H, m)
MS [(M+H)⁺]: 349

| HRMS (C₂₀H₃₃N₃O₃): | |
|---|---|
| Theoretical value: | 349.24912 |
| Measured value: | 349.24920 |

The compound described above was reacted in the ordinary method, converting it into N-(trans-4-methylcyclohexyl)-2-[4-(2-dimethylaminoethoxy)-3-methoxyphenyl]acetamide hydrochloride, which had the following physiochemical properties:
Proton nuclear magnetic resonance spectrum (δ ppm in D₂O):
0.86 (3H, d, J = 6Hz),
0.9 - 1.9 (9H, m),
2.91 (2H, t, J = 7Hz), 3.01 (6H, s),
3.61 (2H, s), 3.63 (1H, m),
3.83 (3H, s), 4.21 (2H, t, J = 7Hz),
6.6 - 6.9 (3H, m)
MS [(M-Cℓ)⁺]: 349

| HRMS (C₂₀H₃₃N₂O₃): | |
|---|---|
| Theoretical value: | 349.24912 |
| Measured value: | 349.24922 |

### Example 64 (Esterification and Amidation)

Using 3.1 g of 4-acetoxy-3-methoxycinnamic acid, 2.8 mℓ of diethyl chlorophosphate, 3.9 mℓ of triethylamine, 100 mℓ of methylene chloride, 2 g of trans-4-methylcyclohexylammmonium chloride, and 0.6 g of 4-dimethylaminopyridine, a reaction similar to that conducted in Example 40 was carried out. As a result, 3.7 g of N-(trans-4-methylcyclohexyl)-4-acetoxy-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (9H, m),
2.31 (3H, s), 3.83 (1H, m),
3.84 (3H, s), 5.46 (1H, m),
6.25 (1H, d, J = 16Hz),
6.7 - 7.2 (3H, m),
7.51 (1H, d, J = 16Hz)
IRₘₐₓ (KBr, cm⁻¹): 3286, 2940, 1766, 1653
MS (M⁺): 331

| Elemental analysis (C₁₉H₂₅NO₄): | | | |
|---|---|---|---|
| Theoretical value; | C: 68.86, | H: 7.60, | N: 4.23 |
| Measured value; | C: 68.81, | H: 7.50, | N: 4.26 |

### Example 65 (Hydrolysis and Conversion into Non-toxic Salt)

Using 3.7 g of N-(trans-4-methylcyclohexyl)-4-acetoxy-3-methoxycinnamamide (Example 130), 100 mℓ of methanol, and 5 g of potassium carbonate, a reaction similar to that conducted in Example 41 was carried out. As a result, 2.75 g of N-(trans-4-methylcyclohexyl)-4-hydroxy-3-methoxycinnamamide (a compound of the present invention) was obtained as white crystal, which had the following physiochemical properties:
Melting point: 208.5 - 209.2°C
Proton nuclear magnetic resonance spectrum (δ ppm in DMSO-d₆):
0.86 (3H, d, J = 6Hz),
0.9 - 1.9 (9H, m), 3.56 (1H, m),
3.80 (3H, s),
6.37 (1H, d, J = 16Hz),
6.76 (1H, d, J = 8Hz),
6.94 (1H, dd, J = 6, 2Hz),
7.09 (1H, d, J = 2Hz),
7.25 (1H, d, J = 16Hz),
7.75 (1H, d, J = 8Hz), 9.35 (1H, s)
IRₘₐₓ (KBr, cm⁻¹): 3256, 2953, 1669
MS (M⁺): 289

| Elemental analysis (C₁₇H₂₃NO₃): | | | |
|---|---|---|---|
| Theoretical value; | C: 70.56, | H: 8.01, | N: 4.84 |
| Measured value; | C: 70.66, | H: 7.99, | N: 5.00 |

The compound described above was reacted in the ordinary method, converting it into sodium N-(trans-4-methylcyclohexyl)-3-methoxy-4-oxidocinnamamide, which had the following physiochemical properties:
Proton nuclear magnetic resonance spectrum (δ ppm in D₂O):
0.84 (3H, d, J = 6Hz),
0.9 - 2.0 (9H, m), 3.58 (1H, m),
3.80 (3H, s),
6.23 (1H, d, J = 16Hz),
6.59 (1H, d, J = 8Hz),
7.06 (1H, d, J = 8Hz), 7.10 (1H, s),
7.35 (1H, d, J = 16Hz)
MS [(M+Na)⁺]: 334, [(M+H)⁺]: 312

| HRMS (C₁₇H₂₂NO₃Na₂): | |
|---|---|
| Theoretical value: | 334.13957 |
| Measured value: | 334.13870 |

### Example 66 (Alkylation)

Using 5 g of N-(trans-4-methylcyclohexyl)-4-hydroxy-3-methoxycinnamamide (Example 131), 120 mℓ of methylisobutylketone, 3.5 g of potassium carbonate, and 3.2 mℓ of 1-bromo-5-chloropentane, a reaction similar to that conducted in Example 42 was carried out. As a result, 5.86 g of N-(trans-4-methylcyclohexyl)-4-(5-chloropentyloxy)-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (15H, m),
3.53 (2H, t, J = 7Hz), 3.85 (1H, m),
3.87 (3H, s), 4.01 (2H, t, J = 7Hz),
5.40 (1H, m),
6.19 (1H, d, J = 16Hz),
6.81 (1H, d, J = 8Hz),
7.0 - 7.1 (2H, m),
7.49 (1H, d, J = 16Hz)
MS [(M+H)⁺]: 394

### Example 67 (Alkylation)

Using 5 g of of N-(trans-4-methylcyclohexyl)-4-hydroxy-3-methoxycinnamamide (Example 65), 120 mℓ of methylisobutylketone, 3.5 g of potassium carbonate, and 2.9 mℓ of 1-bromo-4-chlorobutane, a reaction similar to that conducted in Example 42 was carried out. As a result, 5.79 g of N-(trans-4-methylcyclohexyl)-4-(4-chlorobutoxy)-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (13H, m),
3.60 (2H, t, J = 7Hz), 3.86 (1H, m),
3.87 (3H, s), 4.02 (2H, t, J = 7Hz),
5.42 (1H, m),
6.20 (1H, d, J = 16Hz),
6.81 (1H, d, J = 8Hz),
7.0 - 7.1 (2H, m),
7.49 (1H, d, J = 16Hz)
MS [(M+H)⁺]: 380

### Example 68 (Alkylation)

Using 5 g of of N-(trans-4-methylcyclohexyl)-4-hydroxy-3-methoxycinnamamide (Example 65), 120 mℓ of methylisobutylketone, 3.5 g of potassium carbonate, and 2.4 mℓ of 1-bromo-3-chloropropane, a reaction similar to that conducted in Example 42 was carried out. As a result, 5.63 g of N-(trans-4-methylcyclohexyl)-4-(3-chloropropoxy)-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (9H, m),
3.73 (2H, t, J = 7Hz), 3.86 (1H, m),
3.87 (3H, s), 4.16 (2H, t, J = 7Hz),
5.42 (1H, m),
6.20 (1H, d, J = 16Hz),
6.85 (1H, d, J = 8Hz),
7.0 - 7.1 (2H, m),
7.49 (1H, d, J = 16Hz)
MS [(M+H)⁺]: 366

### Example 69 (Amidation and Conversion into Non-toxic Salt)

Using 5 g of N-(trans-4-methylcyclohexyl)-4-(5-chloropentyloxy)-3-methoxycinnamamide (Example 66), 100 mℓ of methylisobutylketone, and 150 mℓ of 50% aqueous dimethylamine solution, a reaction similar to that conducted in Example 43 was carried out. As a result, 3.17 g of N-(trans-4-methylcyclohexyl)-4-(5-dimethylaminopentyloxy)-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (15H, m), 2.22 (6H, s),
2.23 (2H, t, J = 7Hz), 3.81 (1H, m),
3.87 (3H, s), 4.00 (2H, t, J = 7Hz),
5.41 (1H, m),
6.19 (1H, d, J = 16Hz),
6.81 (1H, d, J = 8Hz),
7.0 - 7.1 (2H, m),
7.49 (1H, d, J = 16Hz)
MS [(M+H)⁺]: 403
The compound described above was reacted in the ordinary method, converting it into sodium N-(trans-4-methylcyclohexyl)-4-(5-dimethylaminopentyloxy)-3-methoxycinnamamide hydrochloride, which had the following physiochemical properties:
Proton nuclear magnetic resonance spectrum (δ ppm in D₂O):
0.88 (3H, d, J = 6Hz),
0.9 - 2.1 (15H, m),
2.88 (2H, t, J = 7Hz), 3.00 (6H, s),
3.82 (1H, m), 3.88 (3H, s),
4.19 (2H, t, J = 7Hz),
6.16 (1H, d, J = 16Hz),
6.79 (1H, d, J = 8Hz),
6.9 - 7.1 (2H, m),
7.41 (1H, d, J = 16Hz)
MS [(M-Cℓ)⁺]: 403

| HRMS (C₂₄H₃₉N₂O₃): | |
|---|---|
| Theoretical value: | 403.29607 |
| Measured value: | 403.29792 |

### Example 70 (Amidation and Conversion into Non-toxic Salt)

Using 5 g of N-(trans-4-methylcyclohexyl)-4-(4-chlorobutoxy)-3-methoxycinnamamide (Example 67), 100 mℓ of methylisobutylketone, and 150 mℓ of 50% aqueous dimethylamine solution, a reaction similar to that conducted in Example 43 was carried out. As a result, 2.01 g of N-(trans-4-methylcyclohexyl)-4-(4-dimethylaminobutoxy)-3-methoxycinnamamide (a compound of the present invention) was obtained as light-greenish white crystal, which had the following physiochemical properties:
Proton nuclear magnetic resonance spectrum (δ ppm in CDCℓ₃):
0.89 (3H, d, J = 6Hz),
1.0 - 2.2 (13H, m), 2.29 (6H, s),
2.32 (2H, t, J = 7Hz), 3.84 (1H, m),
3.86 (3H, s), 4.04 (2H, t, J = 9Hz),
5.41 (1H, m),
6.19 (1H, d, J = 16Hz),
6.80 (1H, d, J = 8Hz),
7.0 - 7.1 (2H, m),
7.49 (1H, d, J = 16Hz)
MS [(M+H)⁺]: 389

| HRMS (C₂₃H₃₇N₂O₃): | |
|---|---|
| Theoretical value: | 389.28042 |
| Measured value: | 389.28028 |

The compound described above was reacted in the ordinary method, converting it into N-(trans-4-methylcyclohexyl)-4-(4-dimethylaminobutoxy)-3-methoxycinnamamide hydrochloride, which had the following physiochemical properties:
Proton nuclear magnetic resonance spectrum (δ ppm in D₂O):
0.88 (3H, d, J = 6Hz),
0.9 - 2.3 (13H, m),
2.86 (2H, t, J = 7Hz), 2.99 (6H, s),
3.83 (1H, m), 3.88 (3H, s),
4.22 (2H, t, J = 7Hz),
6.16 (1H, d, J = 16Hz),
6.9 - 7.1 (3H, m),
7.42 (1H, d, J = 16Hz)
MS [(M-Cℓ)⁺]: 389

| HRMS (C₂₃H₃₇N₂O₃): | |
|---|---|
| Theoretical value: | 389.28042 |
| Measured value: | 389.27942 |

### Example 71 (Reduction and Conversion into Non-toxic Salt)

Using 5 g of N-(trans-4-methylcyclohexyl)-4-(3-chloropropoxy)-3-methoxycinnamamide (Example 68), 100 mℓ of methylisobutylketone, and 150 mℓ of a 50% aqueous dimethylamine solution, a reaction similar to that conducted in Example 43 was carried out. As a result, 4.61 g of N-(trans-4-methylcyclohexyl)-4-(3-dimethylaminopropoxy)-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (11H, m), 2.27 (6H, s),
2.45 (2H, t, J = 7Hz), 3.86 (1H, m),
3.87 (3H, s), 4.06 (2H, t, J = 7Hz),
5.41 (1H, m),
6.18 (1H, d, J = 16Hz),
6.84 (1H, d, J = 8Hz),
7.0 - 7.1 (2H, m),
7.49 (1H, d, J = 16Hz)
MS [(M+H)⁺]: 375
The compound described above was reacted in the ordinary method, converting it into N-(trans-4-methylcyclohexyl)-4-(3-dimethylaminopropoxy)-3-methoxycinnamamide hydrochloride, which had the following physiochemical properties:
Proton nuclear magnetic resonance spectrum (δ ppm in D₂O):
0.88 (3H, d, J = 6Hz),
0.9 - 2.1 (11H, m),
3.00 (6H, s), 3.87 (1H, m),
3.91 (2H, t, J = 7Hz), 3.88 (3H, s),
4.20 (2H, t, J = 7Hz),
6.16 (1H, d, J = 16Hz),
6.9 - 7.1 (3H, m),
7.41 (1H, d, J = 16Hz)
MS [(M-Cℓ)⁺]: 375

| HRMS (C₂₂H₃₅N₂O₃): | |
|---|---|
| Theoretical value: | 375.26477 |
| Measured value: | 375.26475 |

### Example 72 (Alkylation)

Using 5 g of N-(tans-4-methylcyclohexyl)-4-hydroxy-3-methoxycinnamamide (Example 65), 120 mℓ of methylisobutylketone, 3.5 g of potassium carbonate, and 2.1 mℓ of 1-bromo-2-chloroethane, a reaction similar to that conducted in Example 42 was carried out. As a result, 5.48 g of N-(trans-4-methylcyclohexyl)-4-(2-chloroethoxy)-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (9H, m),
3.81 (2H, t, J = 7Hz), 3.88 (3H, s),
4.26 (2H, t, J = 7Hz),
5.41 (1H, d, J = 8Hz),
6.21 (1H, d, J = 16Hz),
6.85 (1H, d, J = 8Hz),
7.0 - 7.1 (2H, m),
7.49 (1H, d, J = 16Hz)
MS [(M+H)⁺]: 352

### Example 73 (Animation and Conversion into Non-toxic Salts)

Using 5 g of N-(trans-4-methylcyclohexyl)-4-(2-chloroethoxy)-3-methoxycinnamamide (Example 72), 100 mℓ of methylisobutylketone, and 150 mℓ of a 50% aqueous dimethylamine solution, a reaction similar to that conducted in Example 43 was carried out. As a result, 3.59 g of N-(trans-4-methylcyclohexyl)-4-(2-dimethylaminoethoxy)-3-methoxycinnamamide (a compound of the present invention) was obtained as white crystal, which had the following physiochemical properties:
Proton nuclear magnetic resonance spectrum (δ ppm in DMSO-d₆):
0.86 (3H, d, J = 6Hz),
0.9 - 1.9 (9H, m), 2.22 (6H, s),
2.50 (2H, t, J = 7Hz), 3.52 (1H, m),
3.79 (3H, s), 4.02 (2H, t, J = 7Hz),
6.43 (1H, d, J = 16Hz),
6.9 - 7.2 (3H, m),
7.28 (1H, d, J = 16Hz),
7.81 (1H, d, J = 8Hz)
MS [(M+H)⁺]: 361
The compound described above was reacted in the ordinary method, converting it into N-(trans-4-methylcyclohexyl)-4-(2-dimethylaminoethoxy)-3-methoxycinnamamide hydrochloride, which had the following physiochemical properties:
Proton nuclear magnetic resonance spectrum (δ ppm in D₂O):
0.86 (3H, d, J = 6Hz),
0.9 - 2.0 (9H, m),
2.98 (6H, s), 3.58 (1H, m),
3.60 (2H, t, J = 7Hz), 3.76 (3H, s),
4.27 (2H, t, J = 7Hz),
6.49 (1H, d, J = 16Hz),
6.8 - 7.2 (3H, m),
7.35 (1H, d, J = 16Hz)
MS [(M-Cℓ)⁺]: 361

| HRMS (C₂₁H₃₃N₂O₃): | |
|---|---|
| Theoretical value: | 361.24912 |
| Measured value: | 361.24904 |

The compound described above was reacted in the ordinary method, converting it into N-(trans-4methylcyclohexyl)-4-(2-dimethylaminoethoxy)-3-methoxycinnamamide maleic acid salt, which had the following physiochemical properties:
Proton nuclear magnetic resonance spectrum (δ ppm in CDCℓ₃):
0.88 (3H, d, J = 6Hz),
0.9 - 2.1 (9H, m), 2.98 (6H, s), 3.49 (2H, t, J = 5Hz),
3.7 - 3.9 (1H, m), 3.84 (3H, s),
4.37 (2H, t, J = 7Hz),
5.73 (1H, d, J = 8Hz),
6.26 (2H, d, J = 1Hz),
6.28 (1H, d, J = 16Hz),
6.83 (1H, d, J = 8Hz), 7.02 (1H, s),
7.02 (1H, dd, J = 2, 8Hz),
7.47 (1H, d, J = 16Hz)
The compound described above was reacted in the ordinary method, converting it into N-(trans-4-methylcyclohexyl)-4-(2-dimethylaminoethoxy)-3-methoxycinnamamide d-tartaric acid salt, which had the following physiochemical properties:
Proton nuclear magnetic resonance spectrum (δ ppm in CD₃OD):
0.90 (3H, d, J = 6Hz),
0.9 - 2.0 (9H, m),
2.89 (6H, s), 3.31 (2H, t, J = 5Hz),
3.6 - 3.8 (1H, m), 3.89 (3H, s),
4.31 (2H, t, J = 7Hz), 4.85 (2H, s),
6.44 (1H, d, J = 16Hz),
7.00 (1H, d, J = 8Hz),
7.11 (1H, dd, J = 2, 8Hz),
7.16 (1H, s),
7.41 (1H, d, J = 16Hz)
The compound described above was reacted in the ordinary method, converting it into N-(trans-4-methylcyclohexyl)-4-(2-dimethylaminoethoxy)-3-methoxycinnamamide p-toluenesulfonic acid salt, which had the following physiochemical properties:
Proton nuclear magnetic resonance spectrum (δ ppm in CD₃OD):
0.87 (3H, d, J = 6Hz),
0.9 - 2.0 (9H, m),
2.31 (3H, s), 2.95 (6H, d, J = 4Hz),
2.4 - 2.5 (2H, m), 3.7 - 3.9 (1H, m),
3.74 (3H, s), 4.2 - 4.3 (2H, m),
6.49 (1H, d, J = 16Hz),
6.67 (1H, d, J = 8Hz),
6.96 (1H, d, J = 2, 8Hz), 7.13 (1H, s),
7.45 (1H, d, J = 16Hz),
7.69 (2H, d, J = 8Hz)
The compound described above was reacted in the ordinary method, converting it into N-(trans-4-methylcyclohexyl)-4-(2-dimethylaminoethoxy)-3-methoxycinnamamide salicylic acid salt, which had the following physiochemical properties:
Proton nuclear magnetic resonance spectrum (δ ppm in CD₃OD):
0.89 (3H, d, J = 6Hz),
0.9 - 2.1 (9H, m),
3.7 - 3.9 (1H, m), 3.84 (3H, s),
5.46 (1H, d, J = 8Hz),
6.21 (1H, d, J = 16Hz),
7.00 (1H, d, J = 2Hz),
7.03 (1H, d, J = 8Hz),
7.3 - 7.4 (2H, m),
7.49 (1H, d, J = 16Hz),
7.85 (1H, dd, J = 2, 8Hz)

### Example 74 (Amination and Conversion into Non-toxic Salt)

Using 5 g of N-(trans-4-methylcyclohexyl)-4-(2-chloroethoxy)-3-methoxycinnamamide (Example 72), 100 mℓ of methylisobutylketone, and 50 mℓ of diethylamine, a reaction similar to that conducted in Example 43 was carried out. As a result, 4.75 g of N-(trans-4-methylcyclohexyl)-4-(2-diethylaminoethoxy)-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (9H, m),
1.04 (3H, d, J = 7Hz),
2.60 (2H, q, J = 7Hz),
2.91 (2H, t, J = 7Hz), 3.80 (1H, m),
3.87 (3H, s), 4.09 (2H, t, J = 7Hz),
5.38 (1H, m),
6.19 (1H, d, J = 16 Hz),
6.84 (1H, d, J = 8Hz),
7.0 - 7.1 (2H, m),
7.49 (1H, d, J = 16Hz)
MS[(M+H)⁺]: 389
The compound described above was reacted in the ordinary method, converting it into N-(trans-4-methylcyclohexyl)-4-(2-dimethylaminoethoxy)-3-methoxycinnamamide hydrochloride, which had the following physiochemical properties:
Proton nuclear magnetic resonance spectrum (δ ppm in D₂O):
0.88 (3H, d, J = 6Hz),
0.9 - 2.1 (9H, m),
1.99 (3H, t, J = 7Hz),
3.22 (2H, t, J = 7Hz),
3.59 (q, 2H, J = 7Hz), 3.79 (1H, m),
3.86 (3H, s), 4.24 (2H, t, J = 7Hz),
6.15 (1H, d, J = 16Hz),
6.79 (1H, d, J = 8Hz),
6.9 - 7.1 (2H, m),
7.46 (1H, d, J = 16Hz)
MS [(M-Cℓ)⁺]: 389

| HRMS (C₂₃H₃₇N₂O₃): | |
|---|---|
| Theoretical value: | 389.28042 |
| Measured value: | 389.28018 |

### Example 75 (Amination)

Using 5 g of N-(trans-4-methylcyclohexyl)-4-(2-chloroethoxy)-3-methoxycinnamamide (Example 72), 100 mℓ of methylisobutylketone, and 200 mℓ of 28% ammonia water, a reaction similar to that conducted in Example 43 was carried out. The crystal procipitated was filtered out, yielding 0.9 g of N-(trans-4-methylcyclohexyl)-4-(2-aminoethoxy)-3-methoxycinnamamide (a compound of the present invention) as white crystal, which had the following physiochemical properties:
Proton nuclear magnetic resonance spectrum (δ ppm in DMSO-d₆):
0.86 (3H, d, J = 6Hz),
0.9 - 1.9 (11H, m),
2.90 (2H, t, J = 7Hz), 3.54 (1H, m),
3.80 (3H, s), 4.03 (2H, t, J = 7Hz),
6.45 (1H, d, J = 16Hz),
6.9 - 7.2 (3H, m),
7.28 (1H, d, J = 16Hz),
7.83 (1H, d, J = 8Hz)
MS [(M+H)⁺]: 333

| HRMS (C₁₉H₂₉N₂O₃): | |
|---|---|
| Theoretical value: | 333.21782 |
| Measured value: | 333.21761 |

### Example 76 (Amination and Conversion into Non-toxic Salt)

22 mℓ of piperidine was added to a solution of 3.9 g of N-(trans-4-methylcyclohexyl)-4-(2-chloroethoxy)-3-methoxycinnamamide (Example 72) in 100 mℓ of methylisobutylketone. The solution was reacted for 18 hours, while it was refluxed.

After reaction, 200 mℓ of an aqueous sodium chloride solution and 200 mℓ of methylene chloride were added to the reaction solution, which was then subjected to extraction. Further, the aqueous layer was extracted twice with 100 mℓ of methylene chloride. The organic layer obtained was washed twice with an aqueous sodium chloride solution and was dried over magnesium sulfate. Thereafter, the solvent was removed in vacuo. The product obtained was recrystallized from methylene chloride/ether, yielding 3.0 g of N-(trans-4-methylcyclohexyl)-4-(2-piperidinoethoxy)-3-methoxycinnamamide (a compound of the present invention) as white crystal, which had the following physiochemical properties:
Proton nuclear magnetic resonance spectrum (δ ppm in CDCℓ₃):
0.88 (3H, d, J = 6Hz),
1.0 - 2.1 (15H, m), 2.50 (4H, m),
2.80 (2H, t, J = 7Hz), 3.86 (1H, m),
3.87 (3H, s), 4.14 (3H, t, J = 6Hz),
5.39 (1H, m),
6.19 (1H, d, J = 16Hz),
6.84 (1H, d, J = 8Hz),
7.0 - 7.1 (2H, m),
7.48 (1H, d, J = 16Hz)
MS [(M+H)⁺]: 401
The compound described above was reacted in the ordinary method, converting it into N-(trans-4-methylcyclohexyl)-4-(2-piperidinoethoxy)-3-methoxycinnamamide hydrochloride, which had the following physiochemical properties:
Proton nuclear magnetic resonance spectrum (δ ppm in D₂O):
0.87 (3H, d, J = 6Hz),
1.0 - 2.4 (15H, m),
3.42 (4H, m), 3.69 (2H, t, J = 7Hz),
3.85 (1H, m), 3.89 (3H, s),
4.31 (2H, t, J = 7Hz),
6.21 (1H, d, J = 16Hz),
6.79 (1H, d, J = 8Hz),
6.9 - 7.1 (2H, m),
7.41 (1H, d, J = 16Hz)
MS [(M-Cℓ)⁺]: 401

| HRMS (C₂₄H₃₇N₂O₃): | |
|---|---|
| Theoretical value: | 401.28377 |
| Measured value: | 401.28051 |

### Example 77 (Amination and Conversion into Non-toxic Salt)

Using 3.9 g of N-(trans-4-methylcyclohexyl)-4-(2-chloroethoxy)-3-methoxycinnamamide (Example 72) and 18 mℓ of pyrrolidine, a reaction similar to that conducted in Example 76 was carried out. As a result, 2.68 g of N-(trans-4-methylcyclohexyl)-4-[2-(1-pyrrolidinyl)ethoxy]-3-methoxycinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
0.9 - 2.1 (6H, m), 2.60 (2H, m),
2.92 (2H, t, J = 6Hz), 3.87 (3H, s),
4.14 (2H, t, J = 6Hz), 5.42 (1H, m),
6.19 (1H, d, J = 16Hz),
6.84 (1H, d, J = 8Hz),
7.0 - 7.1 (2H, m),
7.49 (1H, d, J = 16Hz)
MS [(M+H)⁺]: 387
The compound described above was reacted in the ordinary method, converting it into N-(trans-4-methylcyclohexyl)-4-[2-(1-pyrrolidinyl)ethoxy]-3-methoxycinnamamide hydrochloride, which had the following physiochemical properties:
Proton nuclear magnetic resonance spectrum (δ ppm in D₂O):
0.87 (3H, d, J = 6Hz),
0.9 - 2.1 (6H, m),
3.01 (2H, t, J = 7Hz), 3.40 (2H, m),
3.88 (3H, s), 4.20 (2H, t, J = 7Hz),
6.23 (1H, d, J = 16Hz),
6.88 (1H, d, J = 8Hz),
6.9 - 7.1 (2H, m),
7.41 (1H, d, J = 16Hz)
MS [(M-Cℓ)⁺]: 387

| HRMS (C₂₃H₃₅N₂O₃): | |
|---|---|
| Theoretical value: | 387.26477 |
| Measured value: | 387.26451 |

### Example 78 (Amination and Conversion into Non-toxic Salt)

Using 3.9 g of N-(trans-4-methylcyclohexyl)-4-(2-chloroethoxy)-3-methoxycinnamamide (Example 72) and 19 mℓ of morpholine, a reaction similar to that conducted in Example 76 was carried out. As a result, 3.19 g of N-(trans-4-methylcyclohexyl)-4-(2-morpholinoethoxy)-3-methoxycinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
1.0 - 2.1 (9H, m),
2.56 (4H, t, J = 5Hz),
2.81 (2H, t, J = 6Hz),
3.70 (4H, t, J = 5Hz), 3.86 (3H, s),
4.14 (2H, t, J = 6Hz), 5.44 (1H, m),
6.20 (1H, d, J = 16Hz),
6.83 (1H, d, J = 8Hz),
7.0 - 7.1 (2H, m),
7.49 (1H, d, J = 16Hz)
MS [(M+H)⁺]: 403
The compound described above was reacted in the ordinary method, converting it into N-(trans-4-methylcyclohexyl)-4-(2-morpholinoethoxy)-3-methoxycinnamamide hydrochloride, which had the following physiochemical properties:
Proton nuclear magnetic resonance spectrum (δ ppm in D₂O):
0.87 (3H, d, J = 6Hz),
0.9 - 2.1 (9H, m),
2.99 (2H, t, J = 6Hz),
3.61 (4H, t, J = 5Hz),
3.74 (4H, t, J = 5Hz), 3.85 (3H, s),
4.21 (2H, t, J = 6Hz),
6.22 (1H, d, J = 16Hz),
6.86 (1H, d, J = 8Hz),
6.9 - 7.1 (2H, m),
7.41 (1H, d, J = 16Hz)
MS [(M-Cℓ)⁺]: 403

| HRMS (C₂₃H₃₅N₂O₄): | |
|---|---|
| Theoretical value: | 403.25969 |
| Measured value: | 403.25999 |

### Example 79 (Amination and Conversion into Non-toxic Salts)

Using 3.9 g of N-(trans-4-methylcyclohexyl)-4-(2-chloroethoxy)-3-methoxycinnamamide (Example 72) and 24 mℓ of 1-methylpiperazine, a reaction similar to that conducted in Example 76 was carried out. As a result, 2.24 g of N-(trans-4-methylcyclohexyl)-4-[2-(4-methylpiperazinyl)ethoxy]-3-methoxycinnamamide (a compound of the present invention) was obtained as light-brownish white crystal, which had the following physioche mical properties:
Proton nuclear magnetic resonance spectrum (δ ppm in CDCℓ₃):
0.89 (3H, d, J = 6Hz),
1.0 - 2.1 (9H, m), 2.25 (3H, s),
2.4 - 2.7 (8H, m),
2.83 (2H, t, J = 6Hz), 2.85 (1H, m),
2.86 (3H, s), 3.43 (2H, t, J = 7Hz),
5.39 (1H, d, J = 8Hz),
6.19 (1H, d, J = 16Hz),
6.83 (1H, d, J = 8Hz),
7.0 - 7.1 (2H, m),
7.49 (1H, d, J = 16Hz)
MS [(M+H)⁺]: 416
The compound described above was reacted in the ordinary method, converting it into N-(trans-4-methylcyclohexyl)-4-[2-(4-methylpiperazinyl)ethoxy]-3-methoxycinnamamide dihydrochloride, which had the following physiochemical properties:
Proton nuclear magnetic resonance spectrum (δ ppm in D₂O):
0.88 (3H, d, J = 6Hz),
0.9 - 2.1 (9H, m),
3.00 (3H, s), 3.2 - 3.5 (8H, m),
3.62 (2H, t, J = 7Hz), 3.84 (3H, s),
4.19 (2H, t, J = 7Hz),
6.22 (1H, d, J = 16Hz),
6.9 - 7.1 (3H, m),
7.41 (1H, d, J = 16Hz)
MS [(M+H-2HCℓ)⁺]: 416

| HRMS (C₂₄H₃₈N₃O₃): | |
|---|---|
| Theoretical value: | 416.29132 |
| Measured value: | 416.29158 |

The compound described above was reacted in the ordinary method, converting it into N-(trans-4-methylcyclohexyl)-4-[2-(4-methylpiperazinyl)ethoxy]-3-methoxycinnamamide difumaric acid salt, which had the following physiochemical properties:
Proton nuclear magnetic resonance spectrum (δ ppm in CD₃OD):
0.90 (3H, d, J = 6Hz),
1.0 - 2.0 (9H, m),
2.80 (3H, s), 2.9 - 3.1 (6H, m),
3.2 - 3.3 (4H, m), 3.6 - 3.8 (1H, m),
3.86 (3H, s), 4.17 (2H, t, J = 5Hz),
6.42 (1H, d, J = 16Hz),
6.72 (4H, s),
6.94 (1H, d, J = 8Hz),
7.08 (1H, dd, J = 2, 8Hz),
7.13 (1H, d, J = 2Hz),
7.40 (1H, d, J = 16Hz)
The compound described above was reacted in the ordinary method, converting it into N-(trans-4-methylcyclohexyl)-4-[2-(4-methylpiperazinyl)ethoxy]-3-methoxycinnamamide disuccinic acid salt, which had the following physiochemical properties:
Proton nuclear magnetic resonance spectrum (δ ppm in CD₃OD):
0.90 (3H, d, J = 6Hz),
1.0 - 2.0 (9H, m),
2.73 (8H, s), 2.9 - 3.0 (6H, m),
3.1 - 3.2 (4H, m), 3.6 - 3.8 (1H, m),
3.86 (3H, s), 4.16 (2H, t, J = 5Hz),
6.43 (1H, d, J = 16Hz),
6.94 (1H, d, J = 8Hz),
7.08 (1H, dd, J = 2, 8Hz),
7.13 (1H, d, J = 2Hz),
7.41 (1H, d, J = 16Hz)

### Example 80 (Amination and Conversion into Non-toxic Salts)

Using 3.9 g of N-(trans-4-methylcyclohexyl)-4-(2-chloroethoxy)-3-methoxycinnamamide (Example 72) and 1.5 g of imidazole, a reaction similar to that conducted in Example 76 was carried out. As a result, 3.38 g of N-(trans-4-methylcyclohexyl)-4-[2-(1-imidazolyl)ethoxy]-3-methoxycinnamamide (a compound of the present invention) was obtained as light-brownish white crystal, which had the following physiochemical properties:
Proton nuclear magnetic resonance spectrum (δ ppm in CDCℓ₃):
0.89 (3H, d, J = 6Hz),
1.0 - 2.1 (9H, m), 3.79 (1H, m),
3.86 (3H, s), 4.23 (2H, t, J = 7Hz),
4.34 (2H, t, J = 7Hz), 5.48 (1H, m),
6.20 (1H, d, J = 16Hz),
6.72 (1H, d, J = 9Hz),
7.0 - 7.1 (4H, m),
7.47 (1H, d, J = 16Hz), 7.65 (1H, s)
MS [(M+H)⁺]: 384
The compound described above was reacted in the ordinary method, converting it into N-(trans-4-methylcyclohexyl)-4-[2-(1-imidazolyl)ethoxy]-3-methoxycinnamamide hydrochloride, which had the following physiochemical properties:
Proton nuclear magnetic resonance spectrum (δ ppm in D₂O):
0.88 (3H, d, J = 6Hz),
0.9 - 2.1 (9H, m),
3.75 (1H, m), 3.84 (3H, s),
4.39 (2H, t, J = 7Hz),
5.21 (2H, t, J = 7Hz),
6.18 (1H, d, J = 16Hz),
6.9 - 7.4 (4H m),
7.44 (2H, d, J = 16Hz), 8.82 (1H, s)
MS [(M+H-2HCℓ)⁺]: 384

| HRMS (C₂₂H₃₀N₃O₃): | |
|---|---|
| Theoretical value: | 384.22872 |
| Measured value: | 384.22889 |

The compound described above was reacted in the ordinary method, converting it into N-(trans-4-methylcyclohexyl)-4-[2-(1-imidazolyl)ethoxy]-3-methoxycinnamamide d-tartaric acid salt, which had the following physiochemical properties:
Proton nuclear magnetic resonance spectrum (δ ppm in CD₃OD):
0.90 (3H, d, J = 6Hz),
0.9 - 2.0 (9H, m),
3.6 - 3.8 (1H, m), 3.85 (3H, s),
4.31 (2H, t, J = 5Hz), 4.46 (2H, s),
4.50 (2H, t, J = 5Hz),
6.42 (1H, d, J = 16Hz),
6.91 (1H, d, J = 8Hz),
7.07 (1H, d, J = 8Hz),
7.14 (1H, s),
7.39 (1H, d, J = 16Hz),
7.49 (1H, s), 8.38 (1H, s)
The compound described above was reacted in the ordinary method, converting it into N-(trans-4-methylcyclohexyl)-4-[2-(1-imidazolyl)ethoxy]-3-methoxycinnamamide p-toluenesulfonic acid salt, which had the following physiochemical properties:
Proton nuclear magnetic resonance spectrum (δ ppm in CD₃OD):
0.90 (3H, d, J = 6Hz),
0.9 - 2.0 (9H, m),
2.35 (3H, s), 3.6 - 3.8 (1H, m),
3.84 (3H, s), 4.2 - 4.3 (2H, m),
4.6 - 4.7 (2H, m),
6.45 (1H, d, J = 16Hz),
6.93 (1H, d, J = 8Hz),
7.07 (1H, d, J = 8Hz),
7.1 -7.3 (3H, m),
7.39 (1H, d, J = 16Hz),
7.56 (1H, s), 7.6 - 7.8 (2H, m),
9.02 (1H, s)

### Example 81 (Reduction)

0.075 g of 10% palladium-carbon was added to a solution of 1.5 g of N-(trans-4-methylcyclohexyl)-4-hydroxy-3-methoxycinnamamide (Example 65) in 100 mℓ of methanol. The solution was vigorously stirred for 16 hours under normal-pressure hydrogen gas. After reaction, the catalyst was filtered out, and the solvent was removed in vacuo from the filtrate, yielding 1.33 g of N-(trans-4-methylcyclohexyl)-3-(4-hydroxy-3-methoxyphenyl)propionamide (a compound of the present as a colorless oil, which had the following physiochemical properties:
Proton nuclear magnetic resonance spectrum (δ ppm in DMSO-d₆):
0.83 (3H, d, J = 6Hz),
0.9 - 1.8 (9H, m),
2.23 (2H, t, J = 7Hz),
2.65 (2H, t, J = 7Hz),
3.73 (3H, s), 3.84 (1H, m),
6.53 (1H, dd, J = 6, 2Hz),
6.62 (1H, d, J = 8Hz),
6.72 (1H, d, J = 2Hz),
8.30 (1H, d, J = 8Hz)
MS (M⁺): 291

### Example 82 (Reduction and Conversion into Non-toxic Salt)

Using 1.5 g of N-(trans-4-methylcyclohexyl)-4-(5-dimethylaminopentyloxy)-3-methoxycinnamamide (Example 69), 0.075 g of 10% palladium-carbon, and 100 mℓ of methanol, a reaction similar to that conducted in Example 147 was carried out. The product obtained was recrystallized from methylene chloride/ether, yielding 1.3 g of N-(trans-4-methylcyclohexyl)-3-[4-(5-dimethylaminopentyloxy)-3-methoxyphenyl]propionamide (a compound of the present invention) as light-greenish white crystal, which had had the following physiochemical properties:
Proton nuclear magnetic resonance spectrum (δ ppm in CDCℓ₃):
0.86 (3H, d, J = 6Hz),
0.9 - 2.0 (15H, m), 2.22 (6H, s),
2.24 (2H, t, J = 7Hz),
2.36 (2H, t, J = 7Hz),
2.85 (2H, t, J = 7Hz), 3.64 (1H, m),
3.82 (3H, s), 3.94 (2H, t, J = 7Hz),
5.13 (1H, m), 6.7 - 6.9 (3H, m)
MS [(M+H)⁺]: 405
The compound described above was reacted in the ordinary method, converting it into N-(trans-4-methylcyclohexyl)-3-[4-(5-dimethylaminopentyloxy)-3-methoxyphenyl]propionamide hydrochloride, which had the following physiochemical properties:
0.86 (3H, d, J = 6Hz),
0.9 - 2.1 (15H, m),
2.41 (2H, t, J = 7Hz),
2.52 (2H, t, J = 7Hz),
2.91 (2H, t, J = 7Hz), 3.01 (6H, s),
3.63 (1H, m), 3.81 (3H, s),
4.12 (2H, t, J = 7Hz),
6.7 - 7.0 (3H, m)
MS [(M-Cℓ)⁺]: 405

| HRMS (C₂₄H₄₁N₂O₃): | |
|---|---|
| Theoretical value: | 405.31172 |
| Measured value: | 405.31243 |

### Example 83 (Reduction and Conversion into Non-toxic Salt)

Using 1 g of N-(trans-4-methylcyclohexyl)-4-(4-dimethylaminobutoxy)-3-methoxycinnamamide (Example 70), 0.05 g of 10% palladium-carbon, and 100 mℓ of methanol, a reaction similar to that conducted in Example 81 was carried out. The product obtained was recrystallized from methylene chloride/ether, yielding 0.81 g of N-(trans-4-methylcyclohexyl)-3-[4-(4-dimethylaminobutoxy)-3-methoxyphenyl]propionamide (a compound of the present invention) as light-greenish white crystal, which had the following physiochemical properties:
Proton nuclear magnetic resonance spectrum (δ ppm in CDCℓ₃):
0.87 (3H, d, J = 6Hz),
0.9 - 2.1 (13H, m), 2.22 (6H, s),
2.24 (2H, t, J = 7Hz),
2.34 (2H, t, J = 7Hz),
2.81 (2H, t, J = 7Hz), 3.64 (1H, m),
3.81 (3H, s), 3.99 (2H, t, J = 7Hz),
5.14 (1H, m), 6.7 - 6.9 (3H, m)
MS [(M+H)⁺]: 391

| HRMS (C₂₃H₃₉N₂O₃): | |
|---|---|
| Theoretical value: | 391.29607 |
| Measured value: | 391.29644 |

The compound described above was reacted in the ordinary method, converting it into N-(trans-4-methylcyclohexyl)-3-[4-(4-dimethylaminobutoxy)-3-methoxyphenyl]propionamide hydrochloride, which had the following physiochemical properties:
Proton nuclear magnetic resonance
spectrum (δ ppm in D₂O): 0.86 (3H, d, J = 6Hz),
0.9 - 2.2 (13H, m),
2.36 (2H, t, J = 7Hz),
2.49 (2H, t, J = 7Hz),
2.86 (2H, t, J = 7Hz), 3.00 (6H, s),
3.63 (1H, m), 3.80 (3H, s),
4.13 (2H, t, J = 7Hz),
6.7 - 7.0 (3H, m)
MS [(M-Cℓ)⁺]: 391

| HRMS (C₂₃H₃₉N₂O₃): | |
|---|---|
| Theoretical value: | 391.29607 |
| Measured value: | 391.29652 |

### Example 84 (Reduction and Conversion into Non-toxic Salt)

Using 1.5 g of N-(trans-4-methylcyclohexyl)-4-(3-dimethylaminopropoxy)-3-methoxycinnamamide (Example 71), 0.075 g of 10% palladium-carbon, and 100 mℓ of methanol, a reaction similar to that conducted in Example 81 was carried out. The product obtained was recrystallized from methylene chloride/ether, yielding 1.42 g of N-(trans-4-methylcyclohexyl)-3-[4-(3-dimethylaminopropoxy)-3-methoxyphenyl]propionamide (a compound of the present invention) as white crystal, which had the following physiochemical properties:
Proton nuclear magnetic resonance spectrum (δ ppm in CDCℓ₃):
0.86 (3H, d, J = 6Hz),
0.9 - 1.9 (9H, m), 2.2 - 2.4 (2H, m),
2.36 (2H, t, J = 7Hz), 2.72 (6H, s),
2.85 (2H, t, J = 7Hz),
3.08 (2H, t, J = 7Hz), 3.66 (1H, m),
3.82 (3H, s), 4.03 (3H, t, J = 7Hz),
4.17 (2H, t, J = 6Hz), 5.21 (1H, m),
6.7 - 6.9 (3H, m)
MS [(M+H)⁺]: 377
The compound described above was reacted in the ordinary method, converting it into N-(trans-4-methylcyclohexyl)-3-[4-(3-dimethylaminopropoxy)-3-methoxyphenyl]propionamide hydrochloride, which had the following physiochemical properties:
Proton nuclear magnetic resonance spectrum (δ ppm in D₂O):
0.86 (3H, d, J = 6Hz),
0.9 - 2.2 (9H, m), 2.4 - 2.6 (2H, m),
2.97 (2H, t, J = 7Hz),
2.99 (6H, s), 3.21 (2H, t, J = 7Hz),
3.66 (1H, m), 4.19 (2H, t, J = 7Hz),
4.42 (2H, t, J = 7Hz),
6.7 - 7.0 (3H, m)
MS [(M-Cℓ)⁺]: 377

| HRMS (C₂₂H₃₇N₂O₃): | |
|---|---|
| Theoretical value: | 377.28042 |
| Measured value: | 377.28069 |

### Example 85 (Reduction and Conversion into Non-toxic Salt)

Using 1.5 g of N-(trans-4-methylcyclohexyl)-4-(2-diethylaminoethoxy)-3-methoxycinnamamide (Example 74), 0.075 g of 10% palladium-carbon, and 100 mℓ of methanol, a reaction similar to that conducted in Example 81 was carried out. As a result, 1.21 g of N-(trans-4-methylcyclohexyl)-3-[4-(2-diethylaminoethoxy)-3-methoxyphenyl]propionamide (a compound of the present invention) was obtained as a colorless oil, which had the following physiochemical properties:
Proton nuclear magnetic resonance spectrum (δ ppm in CDCℓ₃):
0.86 (3H, d, J = 6Hz),
0.9 - 1.9 (9H, m),
1.03 (3H, t, J = 7Hz),
2.36 (2H, t, J = 7Hz),
2.59 (2H, q, J = 7Hz),
2.85 (2H, t, J = 7Hz),
2.88 (2H, t, J = 7Hz), 3.64 (1H, m),
3.83 (3H, s), 4.04 (2H, t, J = 7Hz),
5.12 (1H, m), 6.6 - 6.9 (3H, m)
MS [(M+H)⁺]: 391
The compound described above was reacted in the ordinary method, converting it into N-(trans-4-methylcyclohexyl)-3-[4-(2-diethylaminoethoxy)-3-methoxyphenyl]propionamide hydrochloride, which had the following physiochemical properties:
Proton nuclear magnetic resonance spectrum (δ ppm in D₂O):
0.86 (3H, d, J = 6Hz),
0.9 - 2.0 (9H, m),
1.98 (3H, t, J = 7Hz),
2.86 (2H, t, J = 7Hz),
2.99 (2H, t, J = 7Hz),
3.21 (2H, t, J = 7Hz),
3.56 (q, 2H, J = 7Hz), 3.63 (1H, m),
3.82 (3H, s), 4.26 (2H, t, J = 7Hz),
6.6 - 6.9 (3H, m)
MS [(M-Cℓ)⁺]: 391

| HRMS (C₂₃H₃₉N₂O₃): | |
|---|---|
| Theoretical value: | 391.29607 |
| Measured value: | 391.29625 |

### Example 86 (Reduction and Conversion into Non-toxic Salt)

Using 1 g of N-(trans-4-methylcyclohexyl)-4-(2-piperidinoethoxy)-3-methoxycinnamamide (Example 76), 0.05 g of 10% palladium-carbon, and 100 mℓ of methanol, a reaction similar to that conducted in Example 81 was carried out. The product obtained was recrystallized from methylene chloride/ether, yielding 0.91 g of N-(trans-4-methylcyclohexyl)-3-[4-(2-piperidinoethoxy)-3-methoxyphenyl]propionamide (a compound of the present invention) as white crystal, which had the following physiochemical properties:
Proton nuclear magnetic resonance spectrum (δ ppm in CDCℓ₃):
0.86 (3H, d, J = 6Hz),
0.9 - 1.9 (15H, m),
2.36 (2H, t, J = 7Hz),
2.4 - 2.6 (4H, m),
2.79 (2H, t, J = 6Hz),
2.86 (2H, t, J = 7Hz), 3.64 (1H, m),
3.83 (3H, s), 4.10 (2H, t, J = 6Hz),
5.08 (1H, m), 6.7 - 6.9 (3H, m)
MS [(M+H)⁺]: 403
The compound described above was reacted in the ordinary method, converting it into N-(trans-4-methylcyclohexyl)-3-[4-(2-piperidinoethoxy)-3-methoxyphenyl]propionamide hydrochloride, which had the following physiochemical properties:
Proton nuclear magnetic resonance spectrum (δ ppm in D₂O):
0.85 (3H, d, J = 6Hz),
0.9 - 2.0 (15H, m),
2.40 (2H, t, J = 7Hz),
2.81 (2H, t, J = 7Hz),
3.4 - 3.6 (4H, m),
3.86 (2H, t, J = 7Hz),
3.88 (2H, t, J = 7Hz), 3.90 (3H, s),
4.16 (2H, t, J = 7Hz),
6.6 - 6.9 (3H, m)
MS [(M-Cℓ)⁺]: 403

| HRMS (C₂₄H₃₉N₂O₃): | |
|---|---|
| Theoretical value: | 403.29607 |
| Measured value: | 403.29558 |

### Example 87 (Reduction and Conversion into Non-toxic Salt)

Using 1 g of N-(trans-4-methylcyclohexyl)-4-[2-(1-pyrrolidinyl)ethoxy]-3-methoxycinnamamide (Example 77), 0.05 g of 10% palladium-carbon, and 100 mℓ of methanol, a reaction similar to that conducted in Example 81 was carried out. The product obtained was recrystallized from methylene chloride/ether, yielding 0.88 g of N-(trans-4-methylcyclohexyl)-3-{4-[2-(1-pyrrolidinyl)ethoxy]-3-methoxyphenyl}propionamide (a compound of the present invention) as white crystal, which had the following physiochemical properties:
Proton nuclear magnetic resonance spectrum (δ ppm in CDCℓ₃):
0.86 (3H, d, J = 6Hz),
0.9 - 1.9 (6H, m),
2.36 (2H, t, J = 7Hz), 2.60 (2H, m),
2.85 (2H, t, J = 7Hz),
2.90 (2H, t, J = 7Hz), 3.64 (1H, m),
3.83 (3H, s), 4.09 (2H, t, J = 7Hz),
5.11 (1H, m), 6.7 - 6.9 (3H, m)
MS [(M+H)⁺]: 389
The compound described above was reacted in the ordinary method, converting it into N-(trans-4-methylcyclohexyl)-3-{4-[2-(1-pyrrolidinyl)ethoxy]-3-methoxyphenyl}propionamide hydrochloride, which had the following physiochemical properties:
Proton nuclear magnetic resonance spectrum (δ ppm in D₂O):
0.86 (3H, d, J = 6Hz),
0.9 - 1.9 (6H, m),
2.39 (2H, t, J = 7Hz),
2.97 (2H, t, J = 7Hz), 3.40 (2H, m),
3.64 (2H, d, J = 7Hz), 3.81 (3H, s),
4.17 (2H, t, J = 7Hz),
6.7 - 6.9 (3H, m)
MS [(M-Cℓ)⁺]: 389

| HRMS (C₂₃H₃₇N₂O₃): | |
|---|---|
| Theoretical value: | 387.28242 |
| Measured value: | 389.28066 |

### Example 88 (Reduction and Conversion into Non-toxic Salts)

Using 1 g of N-(trans-4-methylcyclohexyl)-4-[2-(4-methylpiperazinyl)ethoxy]-3-methoxycinnamamide (Example 79), 0.05 g of 10% palladium-carbon, and 100 mℓ of methanol, a reaction similar to that conducted in Example 81 was carried out. The product obtained was recrystallized from methylene chloride/ether, yielding 0.64 g of N-(trans-4-methylcyclohexyl)-3-{4-[2-(4-methylpiperazinyl)ethoxy]-3-methoxyphenyl}propionamide (a compound of the present invention) as light-brownish white crystal, which had the following physiochemical properties:
Proton nuclear magnetic resonance spectrum (δ ppm in CDCℓ₃):
0.86 (3H, d, J = 6Hz),
0.9 - 1.9 (9H, m), 2.3 (3H, s),
2.35 (2H, t, J = 7Hz),
2.4 - 2.7 (8H, m),
2.81 (2H, t, J = 6Hz),
2.84 (2H, t, J = 7Hz), 3.65 (1H, m),
3.83 (3H, s), 4.09 (3H, t, J = 6Hz),
5.08 (1H, m), 6.7 - 6.9 (3H, m)
MS [(M+H)⁺]: 418
The compound described above was reacted in the ordinary method, converting it into N-(trans-4-methylcyclohexyl)-3-{4-[2-(4-methylpiperazinyl)ethoxy]-3-methoxyphenyl}propionamide dihydrochloride, which had the following physiochemical properties:
Proton nuclear magnetic resonance spectrum (δ ppm in D₂O):
0.86 (3H, d, J = 6Hz),
0.9 - 1.9 (9H, m),
2.98 (2H, t, J = 7Hz), 3.01 (3H, s),
3.41 (2H, t, J = 7Hz),
3.3 - 3.6 (8H, m),
3.64 (2H, t, J = 7Hz), 3.82 (3H, s),
4.21 (2H, t, J = 7Hz),
6.9 - 7.1 (3H, m)
MS [(M+H-2HCℓ)⁺]: 418

| HRMS (C₂₄H₄₀N₃O₃): | |
|---|---|
| Theoretical value: | 418.30697 |
| Measured value: | 418.30640 |

The compound described above was reacted in the ordinary method, converting it into N-(trans-4-methylcyclohexyl)-3-{4-[2-(4-methylpiperazinyl)ethoxy]-3-methoxyphenyl}propionamide dimaleic acid salt, which had the following physiochemical properties:
Proton nuclear magnetic resonance spectrum (δ ppm in CD₃OD):
0.87 (3H, d, J = 6Hz),
0.9 - 1.9 (9H, m),
2.37 (2H, t, J = 7Hz), 2.80 (3H, s),
2.80 (2H, t, J = 7Hz),
2.9 - 3.1 (6H, m),
3.2 - 3.4 (4H, m), 3.5 - 3.7 (1H, m),
3.82 (3H, s), 4.11 (2H, t, J = 7Hz),
6.29 (4H, s), 6.9 - 7.1 (3H, m)
The compound described above was reacted in the ordinary method, converting it into N-(trans-4-methylcyclohexyl)-3-{4-[2-(4-methylpiperazinyl)ethoxy]-3-methoxyphenyl}propionamide difumaric acid salt, which had the following physiochemical properties:
Proton nuclear magnetic resonance spectrum (δ ppm in CD₃OD):
0.87 (3H, d, J = 6Hz),
0.9 - 1.9 (9H, m),
2.41 (2H, t, J = 7Hz), 2.78 (3H, s),
2.79 (2H, t, J = 7Hz),
2.9 - 3.1 (6H, m),
3.2 - 3.4 (4H, m), 3.4 - 3.6 (1H, m),
3.81 (3H, s), 4.10 (2H, t, J = 7Hz),
6.72 (4H, s), 6.7 - 6.9 (3H, m)
The compound described above was reacted in the ordinary method, converting it into N-(trans-4-methylcyclohexyl)-3-{4-[2-(4-methylpiperazinyl)ethoxy]-3-methoxyphenyl}propionamide disuccinic acid salt, which had the following physiochemical properties:
Proton nuclear magnetic resonance spectrum (δ ppm in CD₃OD):
0.87 (3H, d, J = 6Hz),
0.9 - 1.9 (9H, m),
2.37 (2H, t, J = 7Hz), 2.52 (8H, s),
2.66 (3H, s), 2.80 (2H, t, J = 7Hz),
2.9 - 3.0 (6H, m), 3.0 - 3.2 (4H, m),
3.4 - 3.6 (1H, m), 3.81 (3H, s),
4.09 (2H, t, J = 7Hz),
6.71 (1H, dd, J = 2, 8Hz),
6.82 (1H, d, J = 2Hz),
6.85 (1H, d, J = 8Hz)

### Example 89 (Reduction and Conversion into Non-toxic Salt)

Using 1 g of N-(trans-4-methylcyclohexyl)-4-(2-morpholinoethoxy)-3-methoxycinnamamide (Example 78), 0.05 g of 10% palladium-carbon, and 100 mℓ of methanol, a reaction similar to that conducted in Example 81 was carried out. The product obtained was recrystallized from methylene chloride/ether, yielding 0.90 g of N-(trans-4methylcyclohexyl)-3-[4-[2-morpholinoethoxy)-3-methoxyphenyl]propionamide (a compound of the present invention) as white crystal, which had the following physiochemical properties:
Proton nuclear magnetic resonance spectrum (δ ppm in CDCℓ₃):
0.86 (3H, d, J = 6Hz),
0.9 - 1.9 (9H, m),
2.36 (2H, t, J = 7Hz), 2.56 (4H, m),
2.79 (2H, t, J = 5Hz),
2.85 (2H, t, J = 8Hz), 3.64 (1H, m),
3.71 (4H, m), 3.83 (3H, s),
4.09 (2H, t, J = 6Hz), 5.09 (1H, m),
6.7 - 6.9 (3H, m)
MS [(M+H)⁺]: 405
The compound described above was reacted in the ordinary method, converting it into N-(trans-4-methylcyclohexyl)-3-[4-(2-morpholinoethoxy)-3-methoxyphenyl]propionamide hydrochloride, which had the following physiochemical properties:
Proton nuclear magnetic resonance spectrum (δ ppm in D₂O):
0.86 (3H, d, J = 6Hz),
0.9 - 1.9 (9H, m),
2.81 (2H, t, J = 7Hz),
2.98 (2H, t, J = 7Hz), 3.59 (4H, m),
3.62 (2H, t, J = 7Hz), 3.72 (4H, m),
3.82 (3H, s), 4.16 (2H, t, J = 7Hz),
6.7 - 6.9 (3H, m)
MS [(M-Cℓ)⁺]: 405

| HRMS (C₂₃H₃₇N₂O₄): | |
|---|---|
| Theoretical value: | 405.27533 |
| Measured value: | 405.27512 |

### Example 90 (Reduction and Conversion into Non-toxic Salts)

Using 1 g of N-(trans-4-methylcyclohexyl)-4-[2-(1-imidazolyl)ethoxy]-3-methoxycinnamamide (Example 80), 0.05 g of 10% palladium-carbon, and 100 mℓ of methanol, a reaction similar to that conducted in Example 81 was carried out. The product obtained was recrystallized from methylene chloride/ether, yielding 0.93 g of N-(trans-4-methylcyclohexyl)-3-{4-[2-(1-imidazolyl)ethoxy]-3-methoxyphenyl}propionamide (a compound of the present invention) as light-brownish white crystal, which had the following physiochemical properties:
Proton nuclear magnetic resonance spectrum (δ ppm in CDCℓ₃):
0.86 (3H, d, J = 6Hz),
0.9 - 2.0 (9H, m),
2.35 (2H, t, J = 7Hz),
2.85 (2H, t, J = 7Hz), 3.65 (1H, m),
3.82 (3H, s), 4.19 (2H, t, J = 5Hz),
4.30 (2H, t, J = 5Hz), 5.48 (1H, m),
6.7 - 6.8 (4H, m),
7.05 (1H, dd, J = 6, 2Hz),
7.62 (1H, s)
MS [(M+H)⁺]: 386
The compound described above was reacted in the ordinary method, converting it into N-(trans-4-methylcyclohexyl)-3-{4-[2-(1-imidazolyl)ethoxy]-3-methoxyphenyl}propionamide hydrochloride, which had the following physiochemical properties:
Proton nuclear magnetic resonance spectrum (δ ppm in D₂O):
0.86 (3H, d, J = 6Hz),
0.9 - 2.0 (1H, m),
2.86 (2H, t, J = 7Hz),
3.08 (2H, t, J = 7Hz), 3.68 (1H, m),
3.80 (3H, s), 4.31 (2H, t, J = 7Hz),
4.71 (2H, t, J = 7Hz),
6.7 - 7.0 (3H, m)
7.52 (1H, d, J = 8Hz), 7.71 (1H, s),
9.06 (1H, s)
MS [(M+H-2HCℓ)⁺]: 386

| HRMS (C₂₂H₃₂N₂O₃): | |
|---|---|
| Theoretical value: | 386.24437 |
| Measured value: | 386.24448 |

The compound described above was reacted in the ordinary method, converting it into N-(trans-4-methylcyclohexyl)-3-{4-[2-(1-imidazolyl)ethoxy]-3-methoxyphenyl}propionamide maleic acid salt, which had the following physiochemical properties:
Proton nuclear magnetic resonance spectrum (δ ppm in CD₃OD):
0.87 (3H, d, J = 6Hz),
0.9 - 1.8 (9H, m),
2.36 (2H, t, J = 7Hz),
2.79 (2H, t, J = 7Hz),
3.4 - 3.6 (1H, m), 3.79 (3H, s),
4.27 (2H, t, J = 7Hz),
4.59 (2H, t, J = 7Hz),
6.25 (2H, s),
6.70 (1H, dd, J = 2, 8Hz),
6.82 (1H, d, J = 2Hz),
6.83 (1H, d, J = 8Hz),
7.53 (1H, t, J = 2Hz),
7.71 (1H, t, J = 2Hz),
8.95 (1H, s)
The compound described above was reacted in the ordinary method, converting it into N-(trans-4-methylcyclohexyl)-3-{4-[2-(1-imidazolyl)ethoxy]-3-methoxyphenyl}propionamide fumaric acid salt, which had the following physiochemical properties:
Proton nuclear magnetic resonance spectrum (δ ppm in CD₃OD):
0.87 (3H, d, J = 6Hz),
0.9 - 1.8 (9H, m), 2.35 (2H, t, J = 7Hz),
2.79 (2H, t, J = 7Hz),
3.4 - 3.6 (1H, m), 3.79 (3H, s),
4.21 (2H, t, J = 7Hz),
4.43 (2H, t, J = 7Hz),
6.70 (1H, dd, J = 2, 8Hz),
6.71 (2H, s)
6.79 (1H, d, J = 8Hz),
6.82 (1H, d, J = 2Hz),
7.16 (1H, t, J = 2Hz),
7.41 (1H, t, J = 2Hz), 8.20 (1H, s)

### Example 91 (Reduction and Conversion into Non-toxic Salts)

Using 2 g of N-(trans-4-methylcyclohexyl)-4-(2-dimethylaminoethoxy)-3-methoxycinnamamide (Example 73), 0.1 g of 10% palladium-carbon, and 100 mℓ of methanol, a reaction similar to that conducted in Example 81 was carried out. The product obtained was recrystallized from methylene chloride/ether, yielding 1.89 g of N-(trans-4-methylcyclohexyl)-3-[4-(2-dimethylaminoethoxy)-3-methoxyphenyl]propionamide (a compound of the present invention) as white crystal, which had the following physiochemical properties:
Proton nuclear magnetic resonance spectrum (δ ppm in CDCℓ₃):
0.86 (3H, d, J = 6Hz),
0.9 - 2.0 (9H, m), 2.34 (6H, s),
2.36 (1H, t, J = 7Hz),
2.73 (2H, t, J = 7Hz),
2.85 (2H, t, J = 7Hz), 3.62 (1H, m),
3.83 (3H, s), 4.05 (2H, t, J = 7Hz),
5.08 (1H, m), 6.6 - 6.9 (3H, m)
MS [(M+H)⁺]: 363
The compound described above was reacted in the ordinary method, converting it into N-(trans-4-methylcyclohexyl)-3-[4-(2-dimethylaminoethoxy)-3-methoxyphenyl]propionamide hydrochloride, which had the following physiochemical properties:
Proton nuclear magnetic resonance spectrum (δ ppm in D₂O):
0.81 (3H, d, J = 6Hz),
0.8 - 1.7 (9H, m),
2.44 (2H, t, J = 7Hz),
2.85 (2H, t, J = 7Hz), 3.00 (6H, s),
3.58 (2H, t, J = 7Hz), 3.86 (3H, s),
4.33 (2H, t, J = 7Hz),
6.8 - 7.1 (3H, m)
MS [(M-Cℓ)⁺]: 363

| HRMS (C₂₁H₃₅N₂O₃): | |
|---|---|
| Theoretical value: | 363.26477 |
| Measured value: | 363.26427 |

### Example 92 (Amination and Conversion into Non-toxic Salt)

Using 1.9 g N-(trans-4-methylcyclohexyl)-4-(2-chloroethoxy)-3-methoxycinnamamide (Example 72), 3.4 mℓ of diallyamine, and 50 mℓ of methylisobutylketone, a reaction similar to that conducted in Example 76 was carried out. As a result, 1.16 g of N-(trans-4-methylcyclohexyl)-4-(2-diallylaminoethoxy)-3-methoxycin-namamide (a compound of the present invention) was obtained as white crystal, which had the following physiochemical properties:
Proton nuclear magnetic resonance spectrum (δ ppm in CDCℓ₃):
0.89 (3H, d, J = 6Hz),
1.0 - 2.1 (9H, m),
2.91 (2H, t, J = 7Hz),
3.17 (4H, d, J = 6Hz),
3.71 (1H, m), 3.87 (3H, s),
4.08 (2H, t, J = 7Hz),
5.1 - 5.3 (4H, m),
5.36 (1H, d, 18Hz), 5.86-6.0 (2H, m),
6.18 (1H, d, J=15Hz),
6.83 (1H, d, J = 8Hz),
7.00 (1H, d, J = 2Hz),
7.02 (1H, dd, J = 2, 8Hz),
7.49 (1H, d, J = 15Hz)
MS [M⁺]: 412

| HRMS (C₂₅H₃₆N₂O₃): | |
|---|---|
| Theoretical value: | 412.27259 |
| Measured value: | 412.27255 |

The compound described above was reacted in the ordinary method, converting it into N-(trans-4-methylcyclohexyl)-4-(2-diallylaminoethoxy)-3-methoxycinnamamide hydrochloride, which had the following physiochemical properties:
Proton nuclear magnetic resonance spectrum (δ ppm in CD₃OD):
0.90 (3H d, J = 6Hz),
0.9 - 2.0 (9H, m),
3.58 (2H, t, J = 4Hz), 3.70 (1H, m),
3.91 (3H, s), 3.98 (2H, d, J = 7Hz),
4.39 (3H, t, J = 5Hz),
5.6 - 5.8 (4H, m),
5.9 - 6.2 (2H, m),
6.47 (1H, d, J = 16Hz),
7.02 (1H, d, J = 8Hz),
7.12 (1H, dd, J = 2, 8Hz),
7.20 (1H, d, J = 2Hz),
7.42 (1H, d, J = 16Hz)
MS [M⁻]: 448

### Example 93 (Amination and Conversion into Non-toxic Salt)

Using 1.9 g N-(trans-4-methylcyclohexyl)-4-(2-chloroethoxy)-3-methoxycinnamamide (Example 72), 5.6 mℓ of dipentylamine, and 50 mℓ of methylisobutylketone, a reaction similar to that conducted in Example 142 was carried out. As a result, 2.97 g of N-(trans-4-methylcyclohexyl)-4-(2-dipentylaminoethoxy)-3-methoxycinnamamide (a compound of the present invention) was obtained as white crystal, which had the following physiochemical properties:
Proton nuclear magnetic resonance spectrum (δ ppm in CDCℓ₃):
0.86 (3H, d, J = 6Hz),
0.89 (6H, d, J = 6Hz),
0.9 - 2.1 (21H, m),
2.47 (6H, t, J = 8Hz),
2.88 (2H, t, J=7Hz), 3.81 (1H, m),
3.89 (3H, s), 4.06 (2H, t, J = 7Hz),
5.38 (1H, d, J = 8Hz),
6.19 (1H, d, J = 15Hz),
6.84 (1H, d, J = 8Hz),
7.00 (1H, d, J = 2Hz),
7.03 (1H, d, J = 8Hz),
7.49 (1H, d, J = 16Hz)
MS [M⁺]: 472

| HRMS (C₂₉H₄₈N₂O₃): | |
|---|---|
| Theoretical value: | 472.36649 |
| Measured value: | 472.36745 |

The compound described above was reacted in the ordinary method, converting it into N-(trans-4-methylcyclohexyl)-4-(2-dipentylaminoethoxy)-3-methoxycinnamamide hydrochloride, which had the following physiochemical properties:
Proton nuclear magnetic resonance spectrum (δ ppm in CD₃OD):
0.90 (3H, d, J = 6Hz),
0.94 (6H, t, J = 7Hz),
0.9 - 2.0 (21H, m), 3.25 (4H, m),
3.65 (2H, t, J = 5Hz), 3.71 (1H, m),
3.90 (3H, s), 4.37 (2H, t, J = 5Hz),
6.47 (1H, d, J = 16Hz),
7.01 (1H, d, J = 8Hz),
7.12 (1H, dd, J = 2, 8Hz),
7.20 (1H, d, J = 2Hz),
7.42 (1H, d, J = 16Hz)
MS [M⁻]: 508

### Example 94 (Reduction)

Using 0.2 g of N-(trans-4-methylcyclohexyl)-4-(2-dipentylaminoethoxy)-3-methoxycinnamamide (Example 93), 0.01 g of 10% palladium-carbon, and 35 mℓ of methanol, a reaction similar to that conducted in Example 81 was carried out. As a result, 0.12 g of N-(trans-4-methylcyclohexyl)-3-[4-(2-dipentylaminoethoxy)-3-methoxyphenyl]propionamide (a compound of the present invention) was obtained was white crystal, which had the following physiochemical properties:
Proton nuclear magnetic resonance spectrum (δ ppm in CDCℓ₃):
0.86 (3H, d, J = 7Hz),
0.86 (6H, d, J = 7Hz),
0.9 - 2.0 (21H, m),
2.36 (2H, t, J = 7Hz),
2.43 (4H, t, J = 6Hz),
2.86 (2H, t, J = 7Hz),
3.66 (1H, m), 3.83 (3H, s),
4.01 (2H, t, J = 7Hz),
5.11 (1H, d, J = 8Hz),
6.68 (1H, dd, J = 2, 8Hz),
6.72 (1H, s),
6.79 (1H, dd, J = 2, 8Hz)
MS [(M-H)⁺]: 473

### Example 95 (Amination)

Using 1.9 g of N-(trans-4-methylcyclohexyl)-4-(2-chloroethoxy)-3-methoxycinnamamide (Example 72), 8.3 mℓ of dioctylamine, and 50 mℓ of methylisobutylketone, a reaction similar to that conducted in Example 76 was carried out. As a result, 0.9 g of N-(trans-4-methylcyclohexyl)-4-(2-dioctylaminoethoxy)-3-methoxycinnamamide (a compound of the present invention) was obtained as white crystal, which had the following physiochemical properties:
Proton nuclear magnetic resonance spectrum (δ ppm in CDCℓ₃):
0.86 (3H, d, J = 6Hz),
0.88 (6H, d, J = 7Hz),
0.9 - 2.1 (33H, m),
2.43 (4H, t, J = 7Hz),
2.91 (2H, t, J = 5Hz), 3.82 (1H, m),
3.91 (3H, s), 4.03 (2H, t, J = 5Hz),
5.39 (1H, d, J = 8Hz),
6.19 (1H, d, J = 15Hz),
6.85 (1H, d, J = 8Hz),
7.01 (1H, dd, J = 2, 8Hz),
7.05 (1H, s), 7.49 (1H, d, J = 16Hz)

### Example 96 (Amination and Conversion into Non-toxic Salt)

Using 1.9 g N-(trans-4-methylcyclohexyl)-4-(2-chloroethoxy)-3-methoxycinnamamide (Example 72), 4.6 g of 4-piperidinopiperidine, and 50 mℓ of methylisobutylketone, a reaction similar to that conducted in Example 76 was carried out. As a result, 1.51 g of N-(trans-4-methylcyclohexyl)-4-[2-(4-piperidinopiperidino)ethoxy]-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
0.9 - 2.6 (26H, m),
2.79 (2H, t, J = 7Hz),
3.0 - 3.1 (2H, m),
3.79 (1H, m), 3.88 (3H, s),
4.12 (2H, t, J =7Hz),
5.81 (1H, d, J = 8Hz),
6.19 (1H, d, J = 16Hz),
6.84 (1H, d, J = 8Hz),
7.00 (1H, d, J = 2Hz),
7.02 (1H, dd, J = 2, 8Hz),
7.49 (1H, d, J = 16Hz)
MS [M⁺]: 483

| HRMS (C₂₉H₄₅N₃O₃): | |
|---|---|
| Theoretical value: | 483.34609 |
| Measured value: | 483.34624 |

The compound described above was reacted in the ordinary method, converting it into N-(trans-4-methylcyclohexyl)-4-[2-(4-piperidinopiperidino)-ethoxy]-3-methoxycinnamamide hydrochloride, which had the following physiochemical properties:
Proton nuclear magnetic resonance spectrum (δ ppm in CD₃OD):
0.90 (3H, d, J = 6Hz),
0.9 - 2.6 (18H, m),
2.9 - 3.8 (8H, m),
3.62 (2H, t, J = 7Hz),
3.79 (1H, m), 3.92 (3H, s),
3.9 - 4.1 (1H, m),
4.42 (2H, t, J = 7Hz),
6.48 (1H, d, J = 16Hz),
7.04 (1H, d, J = 8Hz),
7.13 (1H, dd, J = 2, 8Hz),
7.20 (1H, d, J = 2Hz),
7.42 (1H, d, J 16Hz)
MS [(M-H)⁻]: 555

### Example 97 (Reduction)

Using 0.2 g of N-(trans-4-methylcyclohexyl)-4-[2-(4-piperidinopiperidino)ethoxy]-3-methoxycinnamamide (Example 96), 0.01 g of 10% palladium-carbon, and 30 mℓ of methanol, a reaction similar to that conducted in Example 81 was carried out. As a result, 0.12 g of N-(trans-4-methylcyclohexyl)-3-{4-[2-(4-piperidinopiperidino)ethoxy]-3-methoxyphenyl}-propionamide (a compound of the present invention) was obtained as white crystal, which had the following physiochemical properties:
Proton nuclear magnetic resonance spectrum (δ ppm in CDCℓ₃):
0.86 (3H, d, J = 6Hz),
0.9 - 2.4 (22H, m),
2.36 (2H, t, J = 7Hz),
2.4 - 2.6 (4H, m),
2.77 (2H, t, J = 6Hz),
2.85 (2H, t, J = 7Hz),
3.0 - 3.2 (2H, m), 3.6 - 3.8 (1H, m),
3.83 (3H, s), 4.08 (2H, t, J = 7Hz),
5.11 (1H, d, J = 8Hz),
6.68 (1H, d, J = 8Hz),
6.72 (1H, s), 6.79 (1H, d, J = 8Hz)
MS [(M-H)⁺]: 484

### Example 98 (Amination and Conversion into Non-toxic Salt)

Using 1.9 g N-(trans-4-methylcyclohexyl)-4-(2-chloroethoxy)-3-methoxycinnamamide (Example 72), 2.7 g of 2-ethylimdazole, and 50 mℓ of methylisobutylketone, a reaction similar to that conducted in Example 76 was carried out. As a result, 1.78 g of N-(trans-4-methylcyclohexyl)-4-{2-[1-(2-ethyl)imidazolyl]ethoxy}-3-methoxycinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
0.9 - 2.1 (9H, m),
1.32 (3H, t, J = 8Hz),
2.73 (2H, q, J = 8Hz),
3.7 - 3.8 (1H, m), 3.84 (3H, s),
4.20 (2H, t, J =5Hz),
4.24 (2H, t, J = 4Hz),
5.59 (1H, d, J = 8Hz),
6.22 (1H, d, J = 16Hz),
6.69 (1H, d, J = 9Hz),
6.95 (1H, d, J = 1Hz),
6.95 (1H, d, J = 2Hz),
6.96 (1H, dd, J=1, 8Hz),
6.98 (1H, d, J = 2Hz),
7.47 (1H, d, J = 16Hz)
MS [M⁺]: 411

| HRMS (C₂₄H₃₃N₃O₃): | |
|---|---|
| Theoretical value: | 411.25219 |
| Measured value: | 411.25200 |

The compound described above was reacted in the ordinary method, converting it into N-(trans-4-methylcyclohexyl)-4-{2-[1-(2-ethyl)imidazolyl]ethoxy}-3-methoxycinnamamide hydrochloride, which had the following physiochemical properties:
Proton nuclear magnetic resonance spectrum (δ ppm in CD₃OD):
0.90 (3H, d, J = 6Hz),
0.9 - 2.0 (9H, m),
1.41 (3H, t, J = 8Hz),
3.17 (2H, q, J = 8Hz),
3.6 - 3.8 (1H, m), 3.83 (3H, s),
4.34 (2H, t, J = 6Hz),
4.59 (2H, t, J = 5Hz),
6.42 (1H, d, J = 16Hz),
6.91 (1H, d, J = 8Hz),
7.07 (1H, dd, J = 2, 8Hz),
7.12 (1H, d, J = 1Hz),
7.39 (1H, d, J = 16Hz),
7.46 (1H, d, J = 2Hz),
7.63 (1H, d, J = 2Hz)
MS [M⁻]: 447

### Example 99 (Reduction)

Using 0.2 g of N-(trans-4-methylcyclohexyl)-4-{2-[1-(2-ethyl)imidazolyl]ethoxy}-3-methoxycinnamamide (Example 96), 0.01 g of 10% palladium-carbon, and 25 mℓ of methanol, a reaction similar to that conducted in Example 81 was performed. As a result, 0.14 g of N-(trans-4-methylcyclohexyl)-3-{4-{2-[1-(2-ethyl)imidazolyl]ethoxy}-3-methoxyphenyl}propionamide (a compound of the present invention) was obtained as white crystal, which had the following physiochemical properties:
Proton nuclear magnetic resonance spectrum (δ ppm in CDCℓ₃):
0.86 (3H, d, J = 6Hz),
0.9 - 2.0 (9H, m),
1.32 (3H, t, J = 8Hz),
2.34 (2H, t, J = 7Hz),
2.71 (2H, q, J = 8Hz),
2.84 (2H, t, J = 8Hz),
3.6 - 3.8 (1H, m), 3.81 (3H, s),
4.16 (2H, t, J = 4Hz),
4.23 (2H, t, J = 4Hz),
5.19 (1H, d, J = 8Hz),
6.67 (2H, d, J = 1Hz),
6.73 (1H, s), 6.95 (1H, d, J = 1Hz),
6.98 (1H, d, J = 1Hz)
MS [M⁺]: 413

### Example 100 (Amination and Conversion into Non-toxic Salts)

Using 1.9 g N-(trans-4-methylcyclohexyl)-4-(2-chloroethoxy)-3-methoxycinnamamide (Example 72), 3.3 g of benzimidazole, and 100 mℓ of methylisobutylketone, a reaction similar to that conducted in Example 76 was carried out. As a result, 1.14 g of N-(trans-4-methylcyclohexyl)-4-[2-[1-benzimidazolyl)ethoxy]-3-methoxycinnamamide (a compound of the present invention) was obtained as white crystal, which had the following physiochemical properties:
Proton nuclear magnetic resonance spectrum (δ ppm in CD₃OD):
0.89 (3H, d, J = 6Hz),
0.9 - 2.0 (9H, m),
3.6 - 3.8 (1H, m), 3.75 (3H, s),
4.33 (2H, t, J = 5Hz),
4.64 (2H, t, J = 5Hz),
6.38 (1H, d, J = 16Hz),
6.82 (1H, d, J = 8Hz),
6.98 (1H, dd, J = 2, 8Hz),
7.07 (1H, d, J = 2Hz),
7.2 - 7.8 (4H, m),
8.27 (1H, s)
MS [M⁺]: 433

| HRMS (C₂₆H₃₁N₃O₃): | |
|---|---|
| Theoretical value: | 433.23654 |
| Measured value: | 433.23594 |

The compound described above was reacted in the ordinary method, converting it into N-(trans-4-methylcyclohexyl)-4-[2-(1-benzimidazolyl)ethoxy]-3-methoxycinnamamide hydrochloride, which had the following physiochemical properties:
Proton nuclear magnetic resonance spectrum (δ ppm in CD₃OD):
0.90 (3H, d, J = 6Hz),
0.9 - 2.0 (9H, m),
3.6 - 3.8 (1H, m), 3.73 (3H, s),
4.46 (2H, t, J = 4Hz),
4.96 (2H, t, J = 5Hz),
6.40 (1H, d, J = 16Hz),
6.93 (1H, d, J = 8Hz),
7.05 (1H, dd, J = 2, 8Hz),
7.09 (1H, s),
7.36 (1H, d, J = 16Hz),
7.6 - 8.2 (4H, m), 9.55 (1H, s)
MS [M⁻]: 469

### Example 101 (Reduction)

Using 0.2 g of N-(trans-4-methylcyclohexyl)-4-[2-[1-benzimidazolyl)ethoxy]-3-methoxycinnamamide (Example 100), 0.01 g of 10% palladium-carbon, and 35 mℓ of methanol, a reaction similar to that conducted in Example 81 was carried out. As a result, 0.11 g of N-(trans-4-methylcyclohexyl)-3-{4-[2-(1-imidazolyl)ethoxy}-3-methoxyphenyl}propionamide (a compound of the present invention) was obtained as white crystal, which had the following physiochemical properties:
Proton nuclear magnetic resonance spectrum (δ ppm in CDCℓ₃):
0.85 (3H, d, J = 6Hz),
0.9 - 2.0 (9H, m),
2.32 (2H, t, J = 7Hz),
2.82 (2H t, J = 8Hz),
3.6 - 3.8 (1H, m), 3.76 (3H, s),
4.28 (2H, t, J = 5Hz),
4.54 (2H, t, J = 5Hz),
5.2 - 5.3 (1H, d, J = 8Hz),
6.64 (1H, d, J = 2Hz),
6.65 (1H, s), 6.70 (1H, d, J = 2Hz),
7.2 - 7.6 (3H, m), 7.8 - 7.9 (1H, m),
8.20 (1H, s)
MS [M⁺]: 435

### Example 102 (Amination)

1.9 g of N-(trans-4-methylcyclohexyl)-4-(2-chloroethoxy)-3-methoxycinnamamide (Example 72), 4.2 mℓ of ethyl 4-pieperidinecarboxylate, and 50 mℓ of methylisobutylketone, a reaction similar to that conducted in Example 76 was carried out. As a result, 1.47 g of N-(trans-4-methylcyclohexyl)-4-{2-[4-(ethoxycarbonyl)piperidino]ethoxy}-3-methoxycinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
0.9 - 2.4 (16H, m),
1.21 (3H, t, J = 7Hz),
2.80 (2H, t, J = 6Hz),
2.8 - 3.0 (2H, m), 3.7 - 3.9 (1H, m),
3.86 (3H, s), 4.08 (2H, t, J = 7Hz),
4.13 (2H, t, J = 6Hz),
5.44 (1H, d, J = 8Hz),
6.21 (1H, d, J = 15Hz),
6.83 (1H, d, J = 8Hz),
7.01 (1H, d, J = 2Hz),
7.01 (1H, dd, J = 2, 8Hz),
7.49 (1H, d, J = 15 Hz)
MS [M⁺]: 472

| HRMS (C₂₇H₄₀N₂O₅): | |
|---|---|
| Theoretical value: | 472.29372 |
| Measured value: | 472.29248 |

### Example 103 (Hydrolysis and Conversion into Non-toxic Salt)

10 mℓ of 2M aqueous potassium hydroxide solution was added to a solution of 0.5 g of N-(trans-4-methylcyclohexyl)-4-{2-[4-(ethoxycarbonyl)piperidino]ethoxy}-3-methoxycinnamamide (Example 102) in 50 mℓ of methanol. The solution was reacted for 1 hours, while it was refluxed. After reaction, 2N hydrochloric acid to the reaction solution, acidifying the solution. The solution was then extracted twice with 50 mℓ of methylene chloride. The organic layer obtained was dried over magnesium sulfate. Thereafter, the solvent was removed in vacuo. The product obtained was recrystallized from methanol/ether, yielding 0.43 g of N-(trans-4-methylcyclohexyl)-4-[2-(4-carboxypiperidino)ethoxy]-3-methoxycinnamamide (a compound of the present invention) as white crystal, which had the following physiochemical properties:
Proton nuclear magnetic resonance spectrum (δ ppm in CD₃OD):
0.91 (3H, d, J = 6Hz),
0.9 - 2.4 (14H, m),
2.6 - 2.8 (2H, m), 3.1 - 3.3 (1H, m),
3.60 (2H, t, J = 5Hz),
3.7 - 3.9 (1H, m),
3.92 (3H, s), 4.41 (2H, t, J = 4Hz),
6.47 (1H, d, J = 16Hz),
7.03 (1H, d, J = 8Hz),
7.14 (1H, d, J = 8Hz), 7.21 (1H, s),
7.42 (1H, d, J = 16Hz)
The compound described above was reacted in the ordinary method, converting it into N-(trans-4-methylcyclohexyl)-4-[2-(4-carboxypiperidino)ethoxy]-3-methoxycinnamamide sodium salt, which had the following physiochemical properties:
Proton nuclear magnetic resonance spectrum (δ ppm in CD₃OD):
0.90 (3H, d, J = 6Hz),
0.9 - 2.2 (13H, m),
2.3 - 2.5 (1H, m), 3.1 - 3.3 (1H, m),
3.47 (2H, t, J = 6Hz),
3.5 - 3.8 (2H, m),
3.91 (3H, s), 4.34 (2H, t, J = 5Hz),
6.47 (1H, d, J = 16Hz),
7.01 (1H, d, J = 8Hz),
7.11 (1H, dd, J = 2, 8Hz),
7.19 (1H, d = 2Hz),
7.42 (1H, d, J = 16Hz),

### Example 104 (Reduction)

Using 0.2 g of N-(trans-4-methylcyclohexyl)-4-[2-(4-carboxypiperidino)ethoxy]-3-methoxycinnamamide (Example 103), 0.01 g of 10% palladium-carbon, and 25 mℓ of methanol, a reaction similar to that conducted in Example 81 was carried out. As a result, 0.04 g of N-(trans-4-methylcyclohexyl)-3-{4-[2-(4-carboxypiperidino}-3-methoxyphenyl}propionamide (a compound of the present invention) was obtained as amorphous powder, which had the following physiochemical properties:
Proton nuclear magnetic resonance spectrum (δ ppm in CD₃OD):
0.87 (3H, d, J = 6Hz),
0.9 - 2.4 (14H, m),
2.38 (1H, J = 7Hz), 2.5 - 2.7 (1H, m),
2.82 (2H, t, J = 8Hz),
3.1 - 3.3 (1H, m),
3.42 (2H, t, J = 5Hz),
3.7 - 3.9 (1H, m),
3.87 (3H, s), 4.26 (2H, t, J = 5Hz),
6.75 (1H, d, J = 8Hz),
6.96 (1H, d, J = 2Hz),
6.93 (1H, d, J = 8Hz)
MS [M⁺]: 448

### Example 105 (Alkylation)

20.7 g of potassium carbonate and 5 mℓ of methanol were added to a solution of 28.9 g of N-(trans-4-methylcyclohexyl)-4-hydroxy-3-methoxycinnamamide (Example 65) in 600 mℓ of methylisobutylketone. The solution was reacted for 30 minutes, while it was refluxed. Next, 27.7 mℓ of ethyl bromoacetate was added dropwise into the solution over about 20 minutes by means of a dropping funnel. Thereafter, the solution was stirred for 4 hours. After reaction, the solution was allowed to cool to room temperature. After the potassium carbonate has been filtered out from the filtrate, the solvent was removed in vacuo from the filtrate. The product obtained was recrystallized from methylene chloride/ether, yielding 35.2 g of N-(trans-4-methylcyclohexyl)-4-(ethoxycarbonylmethoxy)-3-methoxycinnamamide (a compound of the present invention) as white crystal, which had the following physiochemical properties:
Proton nuclear magnetic resonance spectrum (δ ppm in CDCℓ₃):
0.88 (3H, d, J = 6Hz),
0.9 - 2.1 (9H, m),
1.25 (3H, t, J = 7Hz),
3.7 - 3.9 (1H, m),
3.89 (3H, s), 4.20 (2H, q, J = 7Hz),
4.66 (2H, s), 6.52 (1H, d, J = 8Hz),
6.22 (1H, d, J = 15Hz),
6.74 (1H, d, J = 8Hz),
7.00 (1H, dd, J = 2, 6Hz),
7.03 (1H, s), 7.48 (1H, d, J = 16Hz)
MS [(M+H))⁺]: 375

| HRMS (C₂₁H₂₉NO₅): | |
|---|---|
| Theoretical value: | 375.20457 |
| Measured value: | 375.20417 |

### Example 106 (Hydrolysis and Conversion into Non-toxic Salts)

Using 28 g of N-(trans-4-methylcyclohexyl)-4-(ethoxycarbonylmethoxy)-3-methoxycinnamamide (Example 105), 100 mℓ of 2M aqueous potassium hydroxide solution, and 600 mℓ of methanol, a reaction similar to that conducted in Example 103 was carried out. As a result, 23.9 g of N-(trans-4-methylcyclohexyl)-4-(carboxymethoxy)-3-methoxycinnamamide (a compound of the present invention) was obtained as white crystal, which had the following physiochemical properties:
Proton nuclear magnetic resonance spectrum (δ ppm in CD₃OD):
0.90 (3H, d, J = 6Hz),
0.9 - 2.0 (9H, m),
3.6 - 3.8 (1H, m), 3.88 (3H, s),
4.70 (2H, s),
6.42 (1H, d, J = 16Hz),
6.87 (1H, d, J = 8Hz),
7.06 (1H, dd, J = 2, 8Hz),
7.16 (1H, d, J = 2Hz),
7.40 (1H, d, J = 16Hz)
MS [M⁺]: 347

| HRMS (C₁₉H₂₅NO₅): | |
|---|---|
| Theoretical value: | 347.17327 |
| Measured value: | 347.17295 |

The compound described above was reacted in the ordinary method, converting it into N-(trans-4-methylcyclohexyl)-4-(carboxymethoxy)-3-methoxycinnamamide sodium salt, which had the following hysiochemical properties:
Proton nuclear magnetic resonance spectrum (δ ppm in CD₃OD):
0.90 (3H, d, J = 6Hz),
0.9 - 2.0 (9H, m)
3.6 - 3.8 (1H, m), 3.90 (3H, s),
4.42 (2H, s),
6.42 (1H, d, J = 16Hz),
6.89 (1H, d, J = 8Hz),
7.09 (1H, dd, J = 2, 8Hz),
7.16 (1H, s), 7.40 (1H, d, J = 16Hz)
MS [(M-H)⁺]: 368

| HRMS (C₁₉H₂₃NO₅Na): | |
|---|---|
| Theoretical value: | 368.14742 |
| Measured value: | 368.14690 |

The compound described above was reacted in the ordinary method, converting it into N-(trans-4-methylcyclohexyl)-4-(carboxymethoxy)-3-methoxycinnamamide diethanolmethylamine salt, which had the following physiochemical properties:
Proton nuclear magnetic resonance spectrum (δ ppm in CD₃OD):
0.86 (3H, d, J = 6Hz),
0.9 - 1.9 (9H, m),
2.27 (3H, s), 2.49 (4H, t, J = 6Hz),
3.45 (4H, t, J = 6Hz), 3.79 (3H, s),
4.42 (2H, s),
6.42 (1H, d, J = 16Hz),
6.74 (1H, d, J = 8Hz),
7.02 (1H, d, J = 8Hz),
7.11 (1H, s),
7.26 (1H, d, J = 16Hz),
7.84 (1H, d, J = 8Hz)
The compound described above was reacted in the ordinary method, converting it into N-(trans-4-methylcyclohexyl)-4-(carboxymethoxy)-3-methoxycinnamamide trishydroxymethylaminomethane salt, which had the following physiochemical properties:
Proton nuclear magnetic resonance spectrum (δ ppm in DMSO-d₆):
0.86 (3H, d, J = 6Hz),
0.9 - 1.9 (9H, m),
3.5 - 3.7 (1H, m), 3.79 (3H, s),
4.24 (2H, s),
6.41 (1H, d, J = 16Hz),
6.69 (1H, d, J = 8Hz),
6.99 (1H, d, J = 8Hz),
7.09 (1H, s),
7.26 (1H, d, J = 16Hz),
7.82 (1H, d, J = 8Hz)
The compound described above was reacted in the ordinary method, converting it into ammonium {4-{2-[N-trans-4-methylcyclohexyl)carbamoyl]vinyl}-2-methoxy}-phenoxyacetate salt, which had the following physiochemical properties:
Proton nuclear magnetic resonance spectrum (δ ppm in DMSO-d₆):
0.86 (3H, d, J = 6Hz),
0.9 - 0.9 (9H, m),
3.4 - 3.6 (1H, m), 3.79 (3H, s),
4.22 (2H, s),
6.42 (1H, d, J = 16Hz),
6.70 (1H, d, J = 8Hz),
6.98 (1H, d, J = 8Hz),
7.09 (1H, d, J = 1Hz),
7.26 (1H, d, J = 16Hz),
7.86 (1H, d, J = 8Hz)

### Example 107 (Reduction and Conversion into Non-toxic Salt)

Using 0.5 of N-(trans-4-methylcyclohexyl)-4-(carboxymethoxy)-3-methoxycinnamamide (Example 106), 0.025 g of 10% palladium-carbon, and 70 mℓ of methanol, a reaction similar to that conducted in Example 81 was carried out. As a result, 0.34 g of N-(trans-4-methylcyclohexyl)-3-[4-(carboxymethoxy)-3-methoxyphenyl]propionamide (a compound of the present invention) was obtained as white crystal, which had the following physiochemical properties:
Proton nuclear magnetic resonance spectrum (δ ppm in CDCℓ₃):
0.86 (3H, d, J = 6Hz),
0.9 - 2.0 (9H, m),
2.37 (2H, t, J = 7Hz),
2.85 (2H, t, J = 8Hz),
3.6 - 3.8 (1H, m), 3.86 (3H, s),
4.22 (2H, s), 5.19 (1H, d, J = 8Hz),
6.68 (1H, d, J = 8Hz), 6.77 (1H, s),
6.81 (1H, d, J = 8Hz)
MS [M⁺]: 349
The compound described above was reacted in the ordinary method, converting it into N-(trans-4-methylcyclohexyl)-3-[4-(carboxymethoxy)-3-methoxyphenyl]propionamide sodium salt, which had the following physiochemical properties:
Proton nuclear magnetic resonance spectrum (δ ppm in CD₃OD):
0.87 (3H, d, J = 6Hz),
0.9 - 1.9 (9H, m),
2.36 (2H, t, J = 7Hz),
2.80 (2H, t, J = 8Hz),
3.6 - 3.8 (1H, m), 3.84 (3H, s),
4.46 (2H, s),
6.69 (1H, dd, J = 2, 8Hz),
6.81 (1H, s), 6.83 (1H, d, J = 8Hz)

### Example 108 (Alkylation)

Using 2 g of N-(trans-4-methylcyclohexyl)-4-hydroxy-3-methoxycinnamamide (Example 131), 1.4 g of potassium carbonate, 4 mℓ of ethyl 4-bromobutylate, and 50 mℓ of methylisobutylketone, a reaction similar to that conducted in Example 81 was carried out. As a result, 2.7 g of N-(trans-4-methylcyclohexyl)-4-[3-(ethoxycarbonyl)propoxy]-3-methoxycinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
0.9 - 2.1 (9H, m),
1.22 (3H, t, J = 7Hz),
2.09 (2H, q, J = 7Hz),
2.49 (2H, t, J = 7Hz),
3.7 - 3.9 (1H, m),
3.86 (3H, s), 4.05 (2H, d, J = 6Hz),
4.08 (2H, q, J = 7Hz),
5.48 (1H, d, J = 8Hz),
6.21 (1H, d, J = 16Hz),
6.82 (1H, d, J = 8Hz),
7.01 (1H, s),
7.02 (1H, dd, J = 2, 8Hz),
7.49 (1H, d, J = 16H)
MS [M⁺]: 403

| HRMS (C₂₃H₃₃NO₅): | |
|---|---|
| Theoretical value: | 403.23587 |
| Measured value: | 403.23607 |

### Example 109 (Hydrolysis and Conversion into Non-toxic Salt)

Using 1.6 g of N-(trans-4-methylcyclohexyl)-4-[3-(ethoxycarbonyl)propoxy]-3-methoxycinnamamide (Example 108), 20 mℓ of 2M aqueous potassium hydroxide solution, and 80 mℓ of methanol, a reaction similar to that conducted in Example 103 was carried out. As a result, 1.36 g of N-(trans-4-methylcyclohexyl)-4-(3-carboxypropoxy)-3-methoxycinnamamide (a compound of the present invention) was obtained as white crystal, which had the following physiochemical properties:
Proton nuclear magnetic resonance spectrum (δ ppm in CDCℓ₃):
0.90 (3H, d, J = 6Hz),
0.9 - 2.0 (9H, m),
2.04 (2H, q, J = 7Hz),
2.48 (2H, t, J = 7Hz),
3.6 - 3.8 (1H, m), 3.86 (3H, s),
4.03 (2H, t, J = 6Hz),
6.40 (1H, d, J = 16Hz),
6.92 (1H, d, J = 8Hz),
7.07 (1H, d, J = 8Hz),
7.13 (1H, s), 7.40 (1H, d, J = 16Hz)
MS [(M+H))⁺]: 376

| HRMS (C₂₁H₃₀NO₅): | |
|---|---|
| Theoretical value: | 376.21240 |
| Measured value: | 376.21494 |

The compound described above was reacted in the ordinary method, converting it into N-(trans-4-methylcyclohexyl)-4-(3-carboxypropoxy)-3-methoxycinnamamide sodium salt, which had the following physiochemical properties:
Proton nuclear magnetic resonance spectrum (δ ppm in CD₃OD):
0.91 (3H, d, J = 6Hz),
0.9 - 2.0 (9H, m),
2.04 (2H, q, J = 7H),
2.48 (2H, t, J = 7Hz),
3.6 - 3.8 (1H, m), 3.86 (3H, s),
4.03 (2H, t, J = 6Hz),
6.41 (1H, d, J = 16Hz),
6.93 (1H, d, J = 8Hz),
7.05 (1H, dd, J = 2, 8Hz),
7.13 (1H, s), 7.40 (1H, d, J = 16Hz)

### Example 110 (Reduction)

Using 0.2 g of N-(trans-4-methylcyclohexyl)-4-(3-carboxypropoxy)-3-methoxycinnamamide (Example 109), 0.01 g of 10% palladium-carbon, and 35 mℓ of methanol, a reaction similar to that conducted in Example 81 was carried out. As a result, 0.13 g of N-(trans-4-methylcyclohexyl)-3-[4-(3-carboxypropoxy)-3-methoxyphenyl]propionamide (a compound of the present invention) was obtained as white crystal, which had the following physiochemical properties:
Proton nuclear magnetic resonance spectrum (δ ppm in CDCℓ₃):
0.86 (3H, d, J = 6Hz),
0.9 - 1.9 (9H, m),
2.07 (2H, q, J = 7Hz),
2.36 (2H, t, J = 7Hz),
2.57 (2H, t, J = 7Hz),
2.85 (2H, t, J = 7Hz),
3.6 - 3.8 (1H, m), 3.83 (3H, s),
4.02 (2H, t, J = 6Hz),
5,12 (1H, d, J = 8Hz),
6.67 (1H, dd, J = 3, 8Hz),
7.72 (1H, s),
7.13 (1H, s), 7.40 (1H, d, J = 16Hz)
MS [M⁺]: 377

### Example 111 (Alkylation)

Using 2 g of N-(trans-4-methylcyclohexyl)-4-hydroxy-3-methoxycinnamamide (Example 131), 1.4 g of potassium carbonate, 4 mℓ of ethyl 6-bromohexanoate, and 50 mℓ of methylisobutylketone, a reaction similar to that conducted in Example 105 was carried out. As a result, 2.89 g of N-(trans-4-methylcyclohexyl)-4-[5-(ethyoxycarbonyl)pentyloxy]-3-methoxycinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
0.9 - 2.1 (13H, m),
1.21 (3H, t, J = 7Hz),
2.2 - 2.4 (2H, m),
3.7 - 3.9 (1H, m), 3.86 (3H, s),
4.03 (2H, t, J = 6Hz),
4.07 (2H, q, J = 7Hz),
5.48 (1H, d, J = 8Hz),
6.21 (1H, d, J = 16Hz),
6.80 (1H, d, J = 8Hz), 7.00 (1H, s),
7.02 (1H, dd, J = 2, 8Hz),
7.49 (1H, d, J = 16Hz)
MS [M⁺]: 431

| HRMS (C₂₅H₃₇NO₅): | |
|---|---|
| Theoretical value: | 431.26717 |
| Measured value: | 431.26735 |

### Example 112 (Hydrolysis and Conversion into Non-toxic Salt)

Using 2.5 g of N-(trans-4-methylcyclohexyl)-4-[5-(ethyoxycarbonyl)pentyloxy]-3-methoxycinnamamide (Example 111), 20 mℓ of 2M aqueous potassium hydroxide solution, and 80 mℓ of methanol, a reaction similar to that conducted in Example 103 was carried out. As a result, 2.18 g of N-(trans-4-methylcyclohexyl)-4-(5-carboxypentyloxy)-3-methoxycinnamamide (a compound of the present invention) was obtained as white crystal, which had the following physiochemical properties:
Proton nuclear magnetic resonance spectrum (δ ppm in CD₃OD):
0.90 (3H, d, J = 6Hz),
0.9 - 2.0 (15H, m),
2.29 (2H, t, J = 7Hz),
3.6 - 3.8 (1H, m),
3.86 (3H, s), 4.02 (2H, t, J = 6Hz),
4.87 (2H, s),
6.48 (1H, d, J = 16Hz),
6.90 (1H, d, J = 8Hz),
7.07 (1H, d, J = 8Hz),
7.12 (1H, s), 7.40 (1H, d, J = 16Hz)
The compound described above was reacted in the ordinary method, converting it into N-(trans-4-methylcyclohexyl)-4-(5-carboxypentyloxy)-3-methoxycinnamamide sodium salt, which had the following physiochemical properties:
Proton nuclear magnetic resonance spectrum (δ ppm in CD₃OD):
0.90 (3H, d, J = 6Hz),
0.9 - 2.0 (15H, m),
2.22 (2H, t, J = 7Hz),
3.6 - 3.8 (1H, m),
3.86 (3H, s), 3.99 (2H, t, J = 6Hz),
4.87 (2H, s),
6.40 (1H, d, J = 16Hz),
6.91 (1H, d, J = 8Hz),
7.07 (1H, d, J = 8Hz),
7.12 (1H, s), 7.40 (1H, d, J = 16Hz)

### Example 113 (Reduction)

Using 0.2 g of N-(trans-4-methylcyclohexyl)-4-(5-carboxypentyloxy)-3-methoxycinnamamide, 0.01 g of 10% palladium-carbon, and 35 mℓ of methanol, a reaction similar to that conducted in Example 147 was carried out. As a result, 0.14 g of N-(trans-4-methylcyclohexyl)-3-[4-(5-carboxypentyloxy)-3-methoxyphenyl]propionamide (a compound of the present invention) was obtained as white crystal, which had the following physiochemical properties:
Proton nuclear magnetic resonance spectrum (δ ppm in CDCℓ₃):
0.85 (3H, d, J = 6Hz),
0.9 - 2.0 (15H, m),
2.3 - 2.5 (4H, m),
2.85 (2H, t, J = 7Hz),
3.6 - 3.8 (1H, m), 3.83 (3H, s),
3.95 (2H, t, J = 6Hz),
5.16 (1H, d, J = 8Hz),
6.6 - 6.8 (3H, m)
MS [M⁺]: 405

### Example 114 (Amination and Conversion into Non-toxic Salt)

0.5 g of sodium hydride was added to a solution of 0.68 g of pyrazole in 50 mℓ of THF. Next, 1.9 g of N-(trans-4-methylcyclohexyl)-4-(2-chloroethoxy)-3-methoxycinnamamide (Example 72) was added to the solution. The solution was reacted for 24 hours, while it was refluxed. After reaction, 2N hydrochloric acid was added, acidifying the reaction solution. The solution was washed with 100 mℓ of methylene chloride. Ammonia water was added to the aqueous layer obtained, alkalizing the solution. Thereafter, the solution was extracted with 100 mℓ of methylene chloride. The organic layer obtained was dried over magnesium sulfate, and the solvent was removed in vacuo. The product obtained was refined by means of silica gel column chromatography using methylene chloride only. Thereafter, the solvent was removed in vacuo, yielding 0.44 g of N-(trans-4-methylcyclohexyl)-4-(2-pyrazolylethoxy)-3-methoxycinnamamide (a compound of the present invention) as white crystal, which had the following physiochemical properties:
Proton nuclear magnetic resonance spectrum (δ ppm in CDCℓ₃):
0.88 (3H, d, J = 6Hz),
0.9 - 2.1 (9H, m),
3.7 - 3.9 (1H, m), 3.84 (3H, s),
4.35 (2H, t, J = 5Hz),
4.54 (2H, t, J = 5Hz),
5.49 (1H, d, J = 8Hz),
6.18 (1H, d, J = 16Hz),
6.24 (1H, t, J = 2Hz),
6.70 (1H, d, J = 8Hz),
6.97 (1H, s),
6.97 (1H, dd, J = 2, 8Hz),
7.46 (1H, d, J = 16Hz),
7.56 (1H, d, J = 2Hz),
7.59 (1H, dd, J = 1, 2Hz)
MS [M⁺]: 383

| HRMS (C₂₂H₂₉N₃O₃): | |
|---|---|
| Theoretical value: | 383.22089 |
| Measured value: | 383.22099 |

The compound described above was reacted in the ordinary method, converting it into N-(trans-4-methylcyclohexyl)-4-(2-pyrazolylethoxy)-3-methoxycinnamamide hydrochloride, which had the following physiochemical properties:
Proton nuclear magnetic resonance spectrum (δ ppm in CD₃OD):
0.90 (3H, d, J = 6Hz),
0.9 - 2.0 (9H, m),
3.6 - 3.8 (1H, m), 3.82 (3H, s),
4.33 (2H, t, J = 5Hz),
4.57 (2H, t, J = 5Hz),
5.49 (1H, d, J = 8Hz),
6.37 (1H, t, J = 2Hz),
6.41 (1H, d, J = 16Hz),
6.86 (1H, d, J = 8Hz),
7.04 (1H, dd, J = 2, 8Hz),
7.12 (1H, d, J = 2Hz),
7.39 (1H, d, J = 16Hz)
7.64 (1H, t, J = 1Hz),
7.86 (1H, d, J = 3Hz)
MS [(M-Cℓ)⁺]: 384

### Example 115 (Reduction)

Using 0.1 g of N-(trans-4-methylcyclohexyl)-4-(2-pyrazolylethoxy)-3-methoxycinnamamide (Example 114), 0.007 g of 10% palladium-carbon, and 30 mℓ of methanol, a reaction similar to that conducted in Example 81 was carried out. As a result, 0.05 g of N-(trans-4-methylcyclohexyl)-3-[4-(2-pyrazolylethoxy)-3-methoxyphenyl]propionamide (a compound of the present invention) was obtained as light-yellowish brown crystal, which had the following physiochemical properties:
Proton nuclear magnetic resonance spectrum (δ ppm in CDCℓ₃):
0.86 (3H, d, J = 6Hz),
0.9 - 2.0 (9H, m),
2.34 (2H, t, J = 6Hz),
2.84 (2H, t, J = 7Hz),
3.5 - 3.7 (1H, m), 3.82 (3H, s),
4.30 (2H, t, J = 6Hz),
4.51 (2H, t, J = 5Hz),
5.07 (1H, d, J = 8Hz),
6.24 (1H, t, J = 2Hz),
6.67 (1H, s),
6.67 (1H, dd, J = 2, 8Hz),
6.72 (1H, d, J = 2, 8Hz),
7.51 (1H, d, J = 2Hz),
7.61 (1H, d, J = 2Hz)

### Example 116 (Amination and Conversion into Non-toxic Salt)

Using 1.9 g of N-(trans-4-methylcyclohexyl)-4-(2-chloroethoxy)-3-methoxycinnamamide (Example 138), 1.44 g of 2-phenylimidazole, 0.5 g of sodium hydride, and 50 mℓ of THF, a reaction similar to that conducted in Example 114 was carried out. As a result, 0.44 g of N-(trans-4-methylcyclohexyl)-4-{2-[1-(2-phenyl)imidazolyl]ethoxy}-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
0.9 - 2.1 (9H, m),
3.7 - 3.9 (1H, m), 3.85 (3H, s),
4.24 (2H, t, J = 5Hz),
4.40 (2H, t, J = 5Hz),
5.44 (1H, d, J = 8Hz),
6.19 (1H, d, J = 16Hz),
6.73 (1H, t, J = 8Hz),
7.0 - 7.1 (2H, m),
7.71 (1H, d, J = 9Hz),
7.4 - 7.6 (6H, m),
7.6 - 7.6 (2H, m)
MS [M⁺]: 459
The compound described above was reacted in the ordinary method, converting it into N-(trans-4-methylcyclohexyl)-4-{2-[1-(2-phenyl)imidazolyl]ethoxy}-3-methoxycinnamamide hydrochloride, which had the following physiochemical properties:
Proton nuclear magnetic resonance spectrum (δ ppm in CD₃OD):
0.90 (3H, d, J = 6Hz),
0.9 - 2.0 (9H, m),
3.6 - 3.8 (1H, m), 3.83 (3H, s),
4.43 (2H, t, J = 5Hz),
4.62 (2H, t, J = 5Hz),
6.44 (1H, d, J = 16Hz),
6.92 (1H, t, J = 8Hz),
7.07 (1H, dd, J = 2, 8Hz),
7.14 (1H, d, J = 2Hz),
7.39 (1H, d, J = 16Hz),
7.6 - 7.8 (5H, m),
7.9 - 8.0 (4H, m)
MS [M⁻]: 405

### Example 117 (Reduction)

Using 0.2 g of N-(trans-4-methylcyclohexyl)-5-{2-[1-(2-phenyl)imidazolyl]ethoxy}-3-methoxycinnamamide (Example 116), 0.01 g of 10% palladium-carbon, 30 mℓ of methanol, and 10 mℓ of methylene choride, a reaction similar to that conducted in Example 81 was carried out. As a result, 0.17 g of N-(trans-4-methylcyclohexyl)-3-[4-{2-[1-(2-phenyl)imidazolyl]ethoxy}-3-methoxyphenyl}propionamide (a compound of the present invention) was obtained as white crystal, which had the following physiochemical properties:
Proton nuclear magnetic resonance spectrum (δ ppm in CDCℓ₃):
0.86 (3H, d, J = 6Hz),
0.9 - 2.0 (9H, m),
2.35 (2H, t, J = 7Hz),
2.85 (2H, t, J = 8Hz),
3.6 - 3.8 (1H, m), 3.81 (3H, s),
4.20 (2H, t, J = 5Hz),
4.38 (2H, t, J = 5Hz),
5.21 (1H, d, J = 8Hz),
6.66 (1H, d, J = 1Hz),
6.74 (1H, s), 7.16 (1H, s),
7.3 - 7.5 (5H, m), 7.4 - 7.5 (2H, m)
MS [M⁺]: 461

### Example 118 (Amination and Conversion into Non-toxic Salt)

Using 1.9 g of N-(trans-4-methylcyclohexyl)-4-(2-chloroethoxy)-3-methoxycinnamamide (Example 72), 0.89 g of thiazolidine, 0.5 g of sodium hydride, and 30 mℓ THF, a reaction similar to that conducted in Example 114 was carried out. As a result, 0.45 g of N-(trans-4-methylcyclohexyl)-4-{2-[(2-mercaptoethyl)amino]ethoxy}-3-methoxycinnamamide (a compound of the present invention) was obtained as white crystal, which had the following physiochemical properties:
Proton nuclear magnetic resonance spectrum (δ ppm in CDCℓ₃):
0.89 (3H, d, J = 6Hz),
0.9 - 2.1 (9H, m),
2.70 (2H, t, J = 6Hz),
2.89 (2H, t, J = 6Hz),
2.91 (2H, t, J = 7Hz),
3.7 - 3.9 (1H, m),
3.88 (3H, s), 4.17 (2H, t, J = 7Hz),
5.48 (1H, d, J = 8Hz),
6.20 (1H, d, J = 16Hz),
6.82 (1H, t, J = 8Hz),
7.01 (1H, d, J = 2Hz),
7.02 (1H, dd, J = 2, 8Hz),
7.49 (1H, d, J = 16Hz)
MS [(M+H))⁺]: 393
The compound described above was reacted in the ordinary method, converting it into N-(trans-4-methylcyclohexyl)-4-{2-[(2-mercaptoethyl)amino]ethoxy}-3-methoxycinnamamide hydrochloride, which had the following physiochemical properties:
Proton nuclear magnetic resonance spectrum (δ ppm in CD₃OD):
0.91 (3H, d, J = 6Hz),
0.9 - 2.0 (9H, m),
2.95 (2H, t, J = 6Hz),
2.98 (2H, t, J = 6Hz),
3.23 (2H, t, J = 7Hz),
3.6 - 3.8 (1H, m),
3.89 (3H, s), 4.22 (2H, t, J = 7Hz),
6.43 (1H, d, J = 16Hz),
6.95 (1H, t, J = 8Hz),
7.09 (1H, dd, J = 2, 8Hz),
7.17 (1H, d, J = 2Hz),
7.41 (1H, d, J = 16Hz)
MS [(M+H)⁻]: 429

### Example 119 (Amination)

Using 1.9 g of N-(trans-4-methylcyclohexyl)-4-(2-chloroethoxy)-3-methoxycinnamamide (Example 72), 1.1 g of 2-ethyl-4-methylimidazole, 0.5 g of sodium hydride, and 50 mℓ of THF, a reaction similar to that conducted in Example 114 was carried out. As a result, 0.31 g of white crystal which was about 2:1 mixture of N-(trans-4-methylcyclohexyl)-4-{2-[1-(2-ethyl-4-methyl)imidazolyl]ethoxy}-3-methoxycinnamamide (a compound of the present invention) and N-(trans-4-methylcyclohexyl)-4-{2-[1-(2-ethyl-5-methyl)imidazolyl]ethoxy}-3-methoxycinnamamide (a compound of the present invention) was obtained, which had the following physiochemical properties:
Proton nuclear magnetic resonance spectrum (δ ppm in CDCℓ₃):
0.87 (3H, d, J = 6Hz),
0.9 - 2.1 (9H, m),
1.29 (6/3H, t, J = 6Hz),
1.31 (3/3H, t, J = 6Hz),
2.17 (6/3H, d, J = 1Hz),
2.24 (3/3H, d, J = 1Hz),
2.68 (4/3H, q, J = 7Hz),
2.71 (2/3H, t, J = 7Hz),
3.7 - 3.9 (1H, m), 3.80 (3/3H, s),
3.81 (6/3H, s), 4.1 - 4.3 (4H, m),
5.98 (1H, d, J = 8Hz),
6.27 (1H, d, J = 16Hz),
6.6 - 6.8 (2H, m), 6.9 - 7.1 (2H, m),
7.02 (1H, dd, J = 2, 8Hz),
7.47 (1H, d, J = 16Hz)

### Example 120 (Amination)

Using 1.9 g of N-(trans-4-methylcyclohexyl)-4-(2-chloroethoxy)-3-methoxycinnamamide (Example 72), 0.82 g of 4-methylimidazole, 0.5 g of sodium hydride, and 50 mℓ of THF, a reaction similar to that conducted in Example 114 was carried out. As a result, 1.23 g of white crystal which was about 1:1 mixture of N-(trans-4-methylcyclohexyl)-4-{2-[1-(4-methyl)imidazolyl]ethoxy}-3-methoxycinnamamide (a compound of the present invention) and N-(trans-4-methylcyclohexyl)-4-{2-[1-(5-methyl)imidazolyl]ethoxy}-3-methoxycinnamamide (a compound of the present invention) was obtained, which had the following physiochemical properties:
Proton nuclear magnetic resonance spectrum (δ ppm in CDCℓ₃):
0.86 (3H, d, J = 6Hz),
0.9 - 2.1 (9H, m),
2.21 (3/2H, d, J = 1Hz),
2.27 (3/2H, d, J = 1Hz),
3.7 - 3.9 (1H, m), 3.85 (3/2H, s),
3.87 (3/2H, s), 4.1 - 4.3 (4H, m),
5.45 (1H, d, J = 8Hz),
6.20 (1H, d, J = 16Hz),
6.7 - 6.8 (2H, m), 6.9 - 7.1 (2H, m),
7.48 (1/2H, d, J = 16Hz),
7.52 (1/2H, d, J = 16Hz)

### Example 121 (Esterification and Amidation)

1.9 g of N-(trans-4-methylcyclohexyl)-4-(2-chloroethoxy)-3-methoxycinnamamide (Example 72) was added to a solution of 0.5 g of sodium hydride in 50 mℓ of THF. The solution was reacted for 72 hours, while it was refluxed. After reaction, 2N hydrochloric acid was added to the reaction solution, acidifying the solution, and the solution was extracted with 100 mℓ of methylene chloride. The organic layer obtained was dried over magnesium sulfate, and the solvent was removed in vacuo. The product obtained was recrystallized from methylene chloride/ether, yielding 0.54 g of N-(trans-4-methylcyclohexyl)-4-vinyloxy-3-methoxycinnamamide (a compound of the present invention) as white crystal, which had the following physiochemical properties:
Proton nuclear magnetic resonance spectrum (δ ppm in CDCℓ₃):
0.89 (3H, d, J = 6Hz),
0.9 - 2.1 (9H, m),
3.7 - 3.9 (1H, m), 3.89 (3H, s),
4.45 (1H, dd, J = 2, 4Hz),
4.74 (1H, dd, J = 2, 12Hz),
5.35 (1H, d, J = 8Hz),
6.23 (1H, d, J = 16Hz),
6.55 (1H, dd, J = 6, 8Hz),
6.94 (1H, d, J = 9Hz),
7.05 (1H, s), 7.05 (1H, d, J = 8Hz),
7.50 (1H, d, J = 16Hz)
MS [(M+H)⁺]: 316

| HRMS (C₁₉H₂₆NO₃): | |
|---|---|
| Theoretical value: | 316.19127 |
| Measured value: | 316.19027 |

### Example 122 (Esterification and Amidation, and Conversion into Non-toxic Salt)

A solution of 1.9 of N-(trans-4-methylcyclohexyl)-4-(carboxymethoxy)-3-methoxycinnamamide (Example 106), 1.2 mℓ of diethyl chlorophosphate and 2.3 mℓ of triethylamine, mixed in 60 mℓ of methylene chloride, was stirred for 1 hour at room temperature. Next, 0.6 mℓ of 4-methylpiperazine and 0.04 g of 4-dimethylaminopyridine were added to the solution under ice-cooling, and the solution was further stirred for 4 hours at room temperature.

After reaction, the reaction solution was washed twice with 200 mℓ of an aqueous sodium hydrogencarbonate solution. The organic layer obtained was dried over magnesium sulfate, and the solvent was removed in vacuo. The product obtained was refined by means of silica gel column chromatography using methylene chloride only. Thereafter, the solvent was removed in vacuo, yielding 0.45 g of 1-{4-{2-[N-(trans-4-methylcyclohexyl)carbamoyl]vinyl}-2-methoxyphenoxyacetyl}-4-methylpiperazine (a compound of the present invention) as white crystal, which had the following physiochemical properties:
Proton nuclear magnetic resonance spectrum (δ ppm in CDCℓ₃):
0.89 (3H, d, J = 6Hz),
0.9 - 2.1 (9H, m),
2.28 (3H, s), 2.3 - 2.4 (4H, m),
3.6 - 3.7 (4H, m), 3.7 - 3.9 (1H, m),
3.88 (3H, s), 4.77 (2H, s),
5.39 (1H, d, J = 8Hz),
6.20 (1H, d, J = 16Hz),
6.89 (1H, d, J = 8Hz), 7.02 (1H, s),
7.02 (1H, d, J = 8Hz),
7.48 (1H, d, J = 16Hz)
MS [(M+H)⁺]: 429

| HRMS (C₂₄H₃₅N₃O₄): | |
|---|---|
| Theoretical value: | 429.26276 |
| Measured value: | 429.26326 |

The compound described above was reacted in the ordinary method, converting it into 1-{4-{2-[N-(trans-4-methylcyclohexyl)carbamoyl]vinyl}-2-methoxyphenoxyacetyl}-4-methylpiperazine hydrochloride, which had the following physiochemical properties:
Proton nuclear magnetic resonance spectrum (δ ppm in CD₃OD):
0.91 (3H, d, J = 6Hz),
0.9 - 2.0 (9H, m),
2.95 (3H, s), 3.0 - 3.2 (2H, m),
3.4 - 3.8 (5H, m), 3.90 (3H, s),
4.2 - 4.4 (1H, m), 4.5 - 4.7 (1H, m),
4.88 (2H, s)
6.44 (1H, d, J = 16Hz),
6.96 (1H, t, J = 8Hz),
7.00 (1H, dd, J = 2, 8Hz),
7.18 (1H, d, J = 2Hz),
7.41 (1H, d, J = 16Hz)
MS [M⁻]: 465

### Example 123 (Reduction)

Using 0.2 g of 1-{4-{2-[N-(trans-4-methylcyclohexyl)carbamoyl]vinyl}-2-methoxyphenoxyacetyl}-4-methylpiperazine (Example 122), 0.01 g of 10% palladium-carbon, and 30 mℓ of methanol, a reaction similar to that conducted in Example 81 was carried out. As a result, 0.13 g of 1-{4-{2-[N-(trans-4-methylcyclohexyl)carbamoyl]ethyl}-2-methoxyphenoxyacetyl}-4-methylpiperazine (a compound of the present invention) was obtained as white crystal, which had the following physiochemical properties:
Proton nuclear magnetic resonance spectrum (δ ppm in CD₃OD):
0.86 (3H, d, J = 6Hz),
0.9 - 2.0 (3H, s), 2.29 (3H, s),
2.3 - 2.5 (6H, m),
2.34 (2H, t, J = 5Hz),
3.5 - 3.7 (5H, m),
3.83 (3H, s), 4.69 (2H, s),
5.29 (1H, d, J = 8Hz),
6.66 (1H, dd, J = 2, 8Hz),
6.74 (1H, d, J = 2Hz), 7.02 (1H, s),
7.02 (1H, d, J = 8Hz),
6.83 (1H, d, J = 8Hz)
MS [M⁺]: 431

### Example 124 (Esterification and Amidation, and Conversion into Non-toxic Salt)

Using 1.9 g of N-(trans-4-methylcyclohexyl)-4-(carboxymethoxy)-3-methoxycinnamamide (Example 106), 1.2 mℓ of diethyl chlorophosphate, 2.3 mℓ of triethylamine, 0.92 g of 4-piperidinopiperidine, 0.04 g of 4-dimethylaminopyridine, and 100 mℓ of methylene chloride, a reaction similar to that conducted in Example 122 was carried out. As a result, 1.51 g of 1-{4-{2-[N-(trans-4-methylcyclohexyl)carbamoyl]vinyl}-2-methoxyphenoxyacetyl}-4-piperidinopiperidine (a compound of the present invention) was obtained as white crystal, which had the following physiochemical properties:
Proton nuclear magnetic resonance spectrum (δ ppm in CDCℓ₃):
0.89 (3H, d, J = 6Hz),
0.9 - 2.1 (17H, m),
2.3 - 2.4 (6H, m), 2.9 - 3.1 (1H, m),
3.7 - 3.9 (1H, m), 3.88 (3H, s),
4.0 - 4.2 (1H, m), 4.5 - 4.7 (1H, m),
4.76 (2H, s), 5.39 (1H, d, J = 8Hz),
6.19 (1H, d, J = 16Hz),
6.88 (1H, d, J = 8Hz),
7.02 (1H, s), 7.02 (1H, d, J = 8Hz),
7.48 (1H, d, J = 16Hz)
MS [(M+H)⁺]: 498

| HRMS (C₂₉H₄₄N₃O₄): | |
|---|---|
| Theoretical value: | 498.33318 |
| Measured value: | 498.33402 |

The compound described above was reacted in the ordinary method, converting it into 1-{4-{2-[N-(trans-4-methylcyclohexyl)carbamoyl]vinyl}-2-methoxyphenoxyacetyl}-4-piperidinopiperidine hydrochloride, which had the following physiochemical properties:
Proton nuclear magnetic resonance spectrum (δ ppm in D₂O):
0.91 (3H, d, J = 6Hz),
0.9 - 2.2 (19H, m),
2.6 - 2.7 (1H, m), 2.9 - 3.3 (2H, m),
3.4 - 3.8 (3H, m), 3.89 (3H, s),
4.1 - 4.3 (1H, m), 4,4 - 4.6 (1H, m),
4.8 - 4.9 (2H, m),
6.44 (1H, d, J = 16Hz),
6.93 (1H, d, J = 8Hz),
7.07 (1H, dd, J = 2, 8Hz),
7.18 (1H, d, J = 2Hz),
7.41 (1H, d, J = 16Hz)
MS [M⁻]: 533

### Example 125 (Reduction)

Using 0.2 g of 1-{4-{2-[N-(trans-4-methylcyclohexyl)carbamoyl]vinyl}-2-methoxyphenoxyacetyl}-4-piperidinopiperidine (Example 124), 0.01 g of 10% palladium-carbon, and 30 mℓ of methanol, a reaction similar to that conducted in Example 81 was carried out. As a result, 0.12 g of 1-{4-{2-[N-(trans-4-methylcyclohexyl)carbamoyl]ethyl}-2-methoxyphenoxyacetyl}-4-piperidinopiperidine (a compound of the present invention) was obtained as white crystal, which had the following physiochemical properties:
Proton nuclear magnetic resonance spectrum (δ ppm in CD₃OD):
0.85 (3H, d, J = 6Hz),
0.9 - 2.0 (19H, m),
2.2 - 2.3 (1H, m),
2.37 (2H, t, J = 7Hz),
2.5 - 2.7 (1H, m),
2.85 (2H, t, J = 7Hz),
3.0 - 3.8 (4H, m), 3.84 (3H, s),
4.2 - 4.4 (1H, m), 4.6 - 4.8 (1H, m),
4.66 (2H, d, J = 6Hz),
5.47 (1H, d, J = 8Hz),
6.65 (1H, dd, J = 2, 8Hz),
6.76 (1H, d, J = 2Hz),
6.81 (1H, d, J = 8Hz)
MS [M⁺]: 499

### Example 126 (Esterification and Amidation)

A solution of 3.5 g of N-(trans-4-methylcyclohexyl)-4-hydroxy-3-methoxycinnamamide (Example 65), 3.5 mℓ of diethyl chlorophosphate, and 4 mℓ of triethylamine, mixed in 60 mℓ of methylene chloride was stirred for 1 hour at room temperature. Next, 4 g of N-t-butoxycarbonylglycine hydrochloride and 1 g of 4-dimethylaminopiridine were added to the solution under ice-cooling. The solution was further stirred for 4 hours at room temperature.

After reaction, the reaction solution was washed five times with 200 mℓ of an aqueous sodium hydrogencarbonate solution. The organic layer obtained was dried over magnesium sulfate, and the solvent was removed in vacuo, yielding 3.83 g of N-(trans-4-methylcyclohexyl)-4-(N-t-butoxycarbonylglycyloxy)-3-methoxycinnamamide (a compound of the present invention), which had the following physiochemical properties:
Proton nuclear magnetic resonance spectrum (δ ppm in CDCℓ₃):
0.92 (3H, d, J = 6Hz),
0.9 - 1.8 (9H, m),
1.46 (9H, s), 3.1 - 3.3 (1H, m),
3.84 (3H, s), 4.75 (2H, s),
5.7 - 5.8 (1H, m),
6.31 (1H, d, J = 16Hz),
7.0 - 7.2 (3H, m),
7.52 (1H, d, J = 16Hz)
MS [M⁺]: 446

### Example 127 (Esterification and Amidation, and Hydrolysis)

Using 5.9 g of 3-acetoxy-4-methoxycinnamic acid derived from isoferulic acid by the acetylation thereof, 4.34 mℓ of diethyl chlorophosphate, 8.35 mℓ of triethylamine, 500 mℓ of methylene chloride, 3.74 g of trans-4-methylcyclohexylammonium chloride, and 0.18 g of 4-dimethylaminopyridine, a reaction similar to that conducted in Example 40 was carried out. As a result, 6.3 g of N-(trans-4-methylcyclohexyl)-3-acetoxy-4-methoxycinnamamide (a compound of the present invention) was obtained as white crystal, which had the following physiochemical properties:
Proton nuclear magnetic resonance spectrum (δ ppm in CDCℓ₃):
0.88 (3H, d, J = 6Hz),
1.0 - 2.1 (9H, m),
2.31 (3H, s),
3.84 (3H, s), 5.43 (1H, m),
6.17 (1H, d, J = 16Hz),
6.90 (1H, d, J = 8Hz),
7.19 (1H, d, J = 2Hz),
7.28 (1H, dd, J = 2, 6Hz),
7.47 (1H, d, J = 16Hz)
MS [M⁺]: 331

### Example 128 (Alkylation and Conversion into Non-toxic Salt)

2.07 g of potassium carbonate was added to a solution of 4.97 g of N-(trans-4-methylcyclohexyl)-3-acetoxy-4-methoxycinnamamide (Example 127) in 200 mℓ of ethanol. The solution was stirred for 20 minutes, while it was refluxed. Next, 2.0 mℓ of ethyl bromoacetate was slowly added dropwise to the solution by means of a dropping funnel. Thereafter, the solution was stirred for 2 hours. After reaction, the solution was allowed to cool to room temperature. The crystal and the potassium carbonate, both precipitated from the reaction solution, were filtered out, and the solvent was removed in vacuo from the filtrate. The product obtained was recrystallized from methylene chloride/ether, yielding 1.46 g of N-(trans-4-methylcyclohexyl)-3-(ethoxycarbonylmethoxy)-4-methoxycinnamamide (a compound the present invention) as white crystal, which had the following physiochemical properties:
Proton nuclear magnetic resonance spectrum (δ ppm in CD₃OD):
0.90 (3H, d, J = 6Hz),
0.9 - 2.0 (9H, m),
1.25 (3H, t, J = 7Hz),
3.6 - 3.8 (1H, m),
3.89 (3H, s), 4.20 (2H, q, J = 7Hz),
4.73 (2H, s),
6.39 (1H, d, J = 16Hz),
6.99 (1H, d, J = 8Hz),
7.13 (1H, d, J = 2Hz),
7.17 (1H, dd, J = 2, 8Hz),
7.38 (1H, d, J = 16Hz)
MS [M⁺]: 375
Water was added to the crystal and the potassium carbonate, both filtered out. Then, 100 mℓ of methylene chloride and 100 mℓ of 2N hydrochloric acid were added to the solution, which was subjected to extraction. The aqueous layer obtained was again extracted with 100 mℓ of methylene chloride. The obtained organic layers were combined, washed with an aqueous sodium chloride solution, and dried over magnesium sulfate. Thereafter, the solvent was removed in vacuo.

The product obtained was recrystallized from methylene chloride, yielding 2.58 g of N-(trans-4-methylcyclohexyl)-3-(carboxymethoxy)-4-methoxycinnamamide (a compound of the present invention) as white crystal, which had the following physiochemical properties:
Proton nuclear magnetic resonance spectrum (δ ppm in CD₃OD):
0.90 (3H, d, J = 6Hz),
0.9 - 2.0 (9H, m),
3.6 - 3.8 (1H, m), 3.87 (3H, s),
4.70 (2H, s),
6.37 (1H, d, J = 16Hz),
6.97 (1H, d, J = 8Hz),
7.01 (1H, d, J = 2Hz),
7.15 (1H, dd, J = 2, 8Hz),
7.38 (1H, d, J = 16Hz
MS [M⁺]: 347
The compound described above was reacted in the ordinary method, converting it into N-(trans-4-methylcyclohexyl)-3-(carboxymethoxy)-4-methoxycinnamamide sodium salt, which had the following physiochemical properties:
Proton nuclear magnetic resonance spectrum (δ ppm in CD₃OD):
0.90 (3H, d, J = 6Hz),
0.9 - 2.0 (9H, m),
3.6 - 3.8 (1H, m), 3.88 (3H, s),
4.43 (2H, s),
6.39 (1H, d, J = 16Hz),
6.94 (1H, d, J = 8Hz),
7.05 (1H, dd, J = 2, 6Hz),
7.13 (1H, d, J = 2Hz),
7.39 (1H, d, J = 16Hz)
MS [M⁺]: 369

### Example 129 (Alkylation)

1.03 g of potassium carbonate was added to a solution of 2.15 g of N-(cis-4-methylcyclohexyl)-4-hydroxy-3-methoxycinnamamide (Example 41) in 200 mℓ of ethanol. The solution was stirred for 20 minutes, while it was refluxed. Next, 8.0 mℓ of ethyl bromoacetate was slowly added dropwise to the solution by means of a dropping funnel. Thereafter, the solution was stirred for 4 hours. After reaction, the potassium carbonate was filtered from the reaction solution, and the solvent was removed in vacuo from the filtrate. The product obtained was recrystallized from methylene chloride/ether, yielding 2.12 g of N-(cis-4-methylcyclohexyl)-4-(ethoxycarbonylmethoxy)-3-methoxycinnamamide (a compound of the present invention) as white crystal, which had the following physiochemical properties:
Proton nuclear magnetic resonance spectrum (δ ppm in CDCℓ₃):
0.91 (3H, d, J = 7Hz),
0.9 - 1.8 (9H, m),
1.25 (3H, t, J = 7Hz), 3.91 (3H, s),
4.1 - 4.3 (1H, m),
4.21 (2H, q, J =7Hz),
4.70 (2H, s), 5.64 (1H, s),
6.26 (1H, d, J = 16Hz),
6.75 (1H, d, J = 9Hz),
7.00 (1H, dd, J = 2, 7Hz),
7.05 (1H, d, J = 2Hz),
7.50 (1H, d, J = 16Hz),
MS [M⁺]: 375

### Example 130 (Esterification and Amidation)

30 mℓ of diisopropylethylamine and 1.3 mℓ of 1-iodopentane were added to a solution of 1.84 g of N-(trans-4-methylcyclohexyl)-4-(carboxymethoxy)-3-methoxycinnamamide (Example 106) in 50 mℓ of methylene chloride. The solution was stirred for 16 hours, while it was refluxed. After reaction, the reaction solution was washed three times with 200 mℓ of an aqueous sodium thiosulfate solution and once with an aqueous sodium chloride solution, and was dried over magnesium sulfate. Then, the solvent was removed in vacuo. The product obtained was recrystallized from methylene chloride/ether, yielding 1.56 g of N-(trans-4-methylcyclohexyl)-4-(pentyloxycarbonylmethoxy)-3-methoxycinnamamide (a compound of the present invention) as white crystal, which had the following physiochemical properties:
Proton nuclear magnetic resonance spectrum (δ ppm in CDCℓ₃):
0.90 (3H, d, J = 6Hz),
0.9 - 2.6 (13H, m),
0.98 (3H, s), 3.7 - 3.9 (1H, m),
3.89 (3H, s), 4.08 (2H, t, J = 7Hz),
4.72 (2H, s), 5.44 (1H, d, J = 8Hz),
6.22 (1H, d, J = 16Hz),
6.74 (1H, d, J= 8Hz),
7.00 (1H, dd, J = 2, 7Hz),
7.04 (1H, d, J = 2Hz),
7.49 (1H, d, J = 16Hz),
MS [M⁺]: 417

### Example 131 (Esterification and Amidation)

30 mℓ of diisopropylethylamine and 1.0 mℓ of iodomethane were added to a solution of 1.84 g of N-(trans-4-methylcyclohexyl)-4-(carboxymethoxy)-3-methoxycinnamamide (Example 106) in 50 mℓ methylene chloride. The solution was stirred for 16 hours, while it was refluxed. After reaction, the reaction solution was washed three times with 200 mℓ of an aqueous sodium thiosulfate solution and once with an aqueous sodium chloride solution, and was dried over magnesium sulfate. Then, the solvent was removed in vacuo. The product obtained was recrystallized from methylene chloride/ether, yielding 1.38 g of N-(trans-4-methylcyclohexyl)4-(methoxycarbonylmethoxy)-3-methoxycinnamamide (a compound of the present invention) as white crystal, which had the following physiochemical properties:
Proton nuclear magnetic resonance spectrum (δ ppm in CDCℓ₃):
0.90 (3H, d, J = 6Hz),
0.9 - 2.1 (9H, m),
3.6 - 3.8 (1H, m), 3.73 (3H, s),
3.89 (3H, s), 4.70 (2H, s),
5.41 (1H, d, J = 8Hz),
6.23 (1H, d, J = 16Hz),
6.76 (1H, d, J=8Hz),
7.01 (1H, dd, J = 2, 7Hz),
7.05 (1H, d, J = 2Hz),
7.50 (1H, d, J = 16Hz),
MS [M⁺]: 361

### Example 132 (Halogenation)

19.5 g of sodium iodide was added to a solution of 4.57 g of N-(trans-4-methylcyclohexyl)-4-(2-chloroethoxy)-3-methoxycinnamamide (Example 72) in 300 mℓ of methylethylketone. The solution was stirred for 19 hours, while it was refluxed. After reaction, the reaction solution was washed three times with 200 mℓ of an aqueous sodium thiosulfate solution and once with an aqueous sodium chloride solution, and was dried over magnesium sulfate. Then, the solvent was removed in vacuo. The product obtained was recrystallized from methylene chloride/ether, yielding 4.86 g of N-(trans-4-methylcyclohexyl)-4-(2-iodoethoxy)-3-methoxycinnamamide (a compound of the present invention) as white crystal, which had the following physiochemical properties:
Proton nuclear magnetic resonance spectrum (δ ppm in CDCℓ₃):
0.89 (3H, d, J = 6Hz),
0.9 - 2.1 (9H, m),
3.42 (2H, t, J = 7Hz),
3.8 - 3.9 (1H, m),
3.89 (3H, s), 4.28 (2H, t, J = 7Hz),
5.46 (1H, d, J = 8Hz),
6.21 (1H, d, J=16Hz),
6.80 (1H, d, J = 8Hz), 7.03 (1H, s),
7.04 (1H, dd, J = 2, 7Hz),
7.49 (1H, d, J = 16Hz),
MS [M⁺]: 443

### Example 133

| | | |
|---|---|---|
| (i) | Corn starch | 44 g |
| (ii) | Crystalline cellulose | 40 g |
| (iii) | Carboxymethylcellulose calcium | 5 g |
| (iv) | Light anhydrous silicic acid | 0.5 g |
| (v) | Magnesium stearate | 0.5 g |
| (vi) | Compound obtained in Example 2D | 10 g |
| Total | | 100 g |

According to the formulation specified above, the components (i) to (vi) were mixed uniformly. The mixture was compressed and molded by a pelletizer into tablets weighing 200 mg each. Each tablet contains 10 mg of the compound obtained in Example 2D. These tablets are to be administered to an adult a few times a day, 5 to 15 tablets each time.

### Example 134

| | | |
|---|---|---|
| (i) | Crystalline cellulose | 84.5 g |
| (ii) | Magnesium stearate | 0.5 g |
| (iii) | Carboxymethylcellulose calcium | 5 g |
| (iv) | Compound obtained in Example 17 | 10 g |
| Total | | 100 g |

According to the formulation specified above, the components (i) and (iv), and a part of the component (ii) were mixed uniformly. The mixture was compressed and molded, and the molding formed was pulverzed. The component (iii) and the remaining part of the component (ii) were added to the pulverized material, and mixed. The mixture was compressed and molded by the pelletizer into tablets weighing 200 mg each. Each tablet contains 10 mg of the compound obtained in Example 2D. These tablets are to be administered to an adult several times a day, 5 to 15 tablets each time.

### Example 135

| | | |
|---|---|---|
| (i) | Crystalline cellulose | 49.5 g |
| (ii) | 10% ethanol solution of hydroxypropylcellulose | 35 g |
| (iii) | Carboxymethylcellulose calcium | 5 g |
| (iv) | Magnesium stearate | 0.5 g |
| (v) | Compound obtained in Example 30 | 10 g |
| Total | | 100 g |

According to the formulation specified above, the components (i), (ii) and (v) were mixed uniformly. The mixture was kneaded and granulated by a granulator, dried and crushed. Thereafter, the components (iii) and (iv) were mixed with the crushed material. The resultant mixture was compressed and molded by the pelletizer into tablets weighing 200 mg each. Each tablet contains 20 mg of the compound obtained in Example 30. These tablets are to be administered to an adult several times a day, 5 to 15 tablets each time.

### Example 136

| | | |
|---|---|---|
| (i) | Corn starch | 34.5 g |
| (ii) | Magnesium stearate | 50 g |
| (iii) | Carboxymethylcellulose calcium | 5 g |
| (iv) | Light anhydrous silicic acid | 0.5 g |
| (v) | Compound obtained in Example 33 | 10 g |
| Total | | 100 g |

According to the formulation specified above, the components (i)-(v) were mixed uniformly. The mixture was compressed and molded by a compression molder. The molding obtained was pulverized by a pulverizer and sieved, forming granules. Each gram of the granules contains 100 mg of the compound obtained in Example 33. The granules are to be administered to an adult in an amount of 1 to 3 g each day, in several portions.

### Example 137

| | | |
|---|---|---|
| (i) | Crystalline cellulose | 55 g |
| (ii) | 10% methanol solution of hydroxypropylcellulose | 35 g |
| (iii) | Compound obtained in Example 41 | 10 g |
| Total | | 100 g |

According to the formulation specified above, the components (i)-(iii) were mixed uniformly. The mixture was kneaded and granulated by an extruding granulater, dried and sieved, obtaining granules. Each gram of the granules contains 100 mg of the compound obtained in Example 41. The granules are to be administered to an adult in an amount of 1 to 3 g each day, in several portions.

### Example 138

| | | |
|---|---|---|
| (i) | Corn starch | 89.5 g |
| (ii) | Light anhydrous silicic acid | 0.5 g |
| (iii) | Compound obtained in Example 50 | 10 g |
| Total | | 100 g |

According to the formulation specified above, the components (i)-(iii) were mixed uniformly. The mixture was filled in No. 2 capsules, in an amount of 200 mg in each capsule, thereby preparing drug capsules. Each capsule contains 10 mg of the compound obtained in Example 50. The capsules are to be administered to an adult several times a day, 5 to 15 capsules each time.

### Example 139

| | | |
|---|---|---|
| (i) | Soybean oil | 5 g |
| (ii) | Distilled water for injection | 89.5 g |
| (iii) | Soybean phospholipid | 2.5 g |
| (iv) | Glycerin | 2 g |
| (v) | Compound obtained in Example 65 | 1 g |
| Total | | 100 g |

According to the formulation specified above, the component (v) is dissolved in the components (i) and (iii). To the resultant solution, a solution of the components (ii) and (iv) was added, and emulsified, thereby obtaining an injection.

## Claims

1. A compound represented by formula (I) shown below or a pharmaceutically acceptable salt thereof: 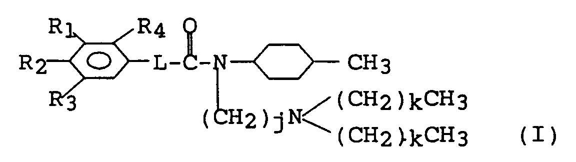 where L represents -(CH=CH)ₖ- or -(CH₂)ₖ-; j represents an integer of 1 to 6; k represents represents an integer of 0 to 5; R₁, R₂, R₃ and R₄ are either identical or different, and each represents a hydrogen atom, an amino group, an acylamino group, a dialkylamino group in which each alkyl moiety has 1 to 5 carbon atoms, a halogen atom, a hydroxyl group, an acetoxy group, an alkoxy group having 1 to 3 carbon atoms, a trifluoromethyl group, a nitro group, tetrahydropyranyloxy group, or a benzyloxy group; and R₁ and R₂ may combine together to form a methylenedioxy group.

2. A compound represented by formula II shown below, or a pharmaceutically acceptable salt thereof: 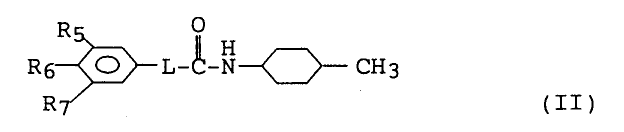 where L represents -(CH=CH)ₖ- or -(CH₂)ₖ-; each k represents an integer of 0 to 5; R₅, R₆ and R₇ are either identical or different, and each represents a hydrogen atom, a hydroxyl group, an acetoxy group, an amino acid ester group in which the amino moiety may be protected by a protective group, an alkoxy group having 1 to 3 carbon atoms, or -O-(CH₂)ⱼ-Y₁; R₅ and R₆ may combine together to form a methylenedioxy group; j represents an integer of 1 to 6; and Y₁ is a halogen atom, an amino group, an amino group substituted with 1 or 2 thioethanol groups, an amino group substituted with 1 or 2 allyl groups, a monoalkylamino group having 1 to 10 carbon atoms, a dialkylamino group in which each alkyl moiety has 1 to 10 carbon atoms, an imidazole group substituted with 1 to 3 substituent groups, a benzimidazole group substituted with 1 to 3 substituent groups, a diazole group, a triazole group, a tetrazole group, a piperidine group, a piperidinopiperidine group, an isonipecotic acid group or an ester thereof with an alkyl group having 1 to 5 carbon atoms, a thiazolidine group, a pyrrolidine group, a morpholine group, a 4-methylpiperazine group, a carboxyl group, a carboxylic acid alkylester group in which the alkyl moiety has 1 to 5 carbon atoms or a carboxamide group.

3. An IV-type allergy suppressive pharmaceutical composition containing, as an effective component, a compound represented by formula (I) or a pharmaceutical acceptable salt thereof: 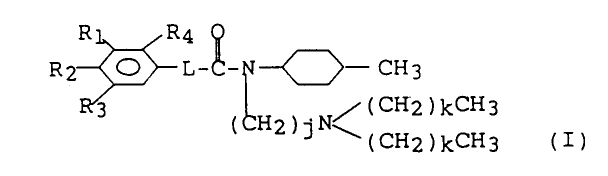 where L represents -(CH=CH)ₖ- or -(CH₂)ₖ-; j represents an integer of 1 to 6; k represents represents an integer of 0 to 5; R₁, R₂, R₃ and R₄ are either identical or different, and each represents a hydrogen atom, an amino group, an acylamino group, a dialkylamino group in which each alkyl moiety has 1 to 5 carbon atoms, a halogen atom, a hydroxyl group, an acetoxy group, an alkoxy group having 1 to 3 carbon atoms, a trifluoromethyl group, a nitro group, tetrahydropyranyloxy group, or a benzyloxy group; and R₁ and R₂ may combine together to form a methylenedioxy group.

4. An IV-type allergy suppressive pharmaceutical composition containing, as an effective component, a compound represented by formula (II) or a pharmaceutical acceptable salt thereof: 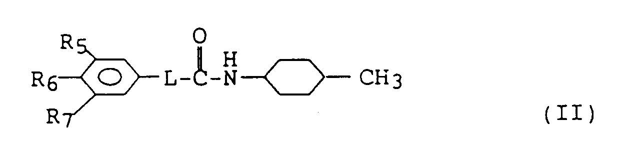 where L represents -(CH=CH)ₖ- or -(CH₂)ₖ-; each k represents an integer of 0 to 5; R₅, R₆ and R₇ are either identical or different, and each represents a hydrogen atom, a hydroxyl group, an acetoxy group, an amino acid ester group in which the amino moiety may be protected by a protective group, an alkoxy group having 1 to 3 carbon atoms, or -O-(CH₂)ⱼ-Y₁; R₅ and R₆ may combine together to form a methylenedioxy group; j represents an integer of 1 to 6; and Y₁ is a halogen atom, an amino group, an amino group substituted with 1 or 2 thioethanol groups, an amino group substituted with 1 or 2 allyl groups, a monoalkylamino group having 1 to 10 carbon atoms, a dialkylamino group in which each alkyl moiety has 1 to 10 carbon atoms, an imidazole group substituted with 1 to 3 substituent groups, a benzimidazole group substituted with 1 to 3 substituent groups, a diazole group, a triazole group, a tetrazole group, a piperidine group, a piperidinopiperidine group, an isonipecotic acid group or an ester thereof with an alkyl group having 1 to 5 carbon atoms, a thiazolidine group, a pyrrolidine group, a morpholine group, a 4-methylpiperazine group, a carboxyl group, a carboxylic acid alkylester group in which the alkyl moiety has 1 to 5 carbon atoms or a carboxamide group.

## Patentansprüche

1. Verbindung der nachstehend gezeigten Formel (I) oder ein pharmazeutisch verträgliches Salz davon: 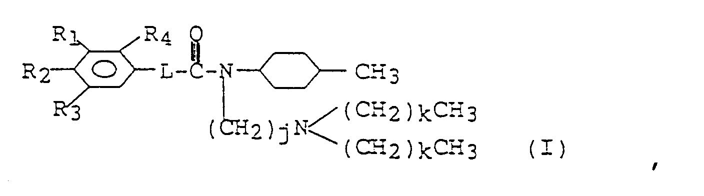 wobei L die Gruppe -(CH=CH)ₖ- oder -(CH₂)ₖ- bezeichnet; j eine ganze Zahl von 1 bis 6 ist; k eine ganze Zahl von 0 bis 5 ist; R₁, R₂, R₃ und R₄ entweder gleich oder verschieden sind und jeweils ein Wasserstoffatom, einen Aminorest, einen Acylaminorest, einen Dialkylaminorest, bei dem jede Alkyleinheit 1 bis 5 Kohlenstoffatome hat, ein Halogenatom, eine Hydroxygruppe, eine Acetoxygruppe, einen Alkoxyrest mit 1 bis 3 Kohlenstoffatomen, eine Trifluormethylgruppe, eine Nitrogruppe, Tetrahydropyranyloxygruppe oder eine Benzyloxygruppe darstellen; und wobei R₁ und R₂ sich verbinden können, um eine Methylendioxygruppe zu bilden.

2. Verbindung der nachstehend gezeigten Formel II oder eine pharmazeutisch verträgliches Salz davon: 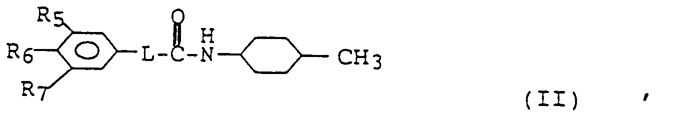 wobei L die Gruppe -(CH=CH)ₖ- oder -(CH₂)ₖ- bezeichnet; jedes k eine ganze Zahl von 0 bis 5 ist; R₅, R₆ und R₇ entweder gleich oder verschieden sind und jeweils ein Wasserstoffatom, eine Hydroxygruppe, eine Acetoxygruppe, einen Ester einer Aminosäure, in dem die Aminoeinheit durch eine Schutzgruppe geschützt sein kann, einen Alkoxyrest mit 1 bis 3 Kohlenstoffatomen, oder -O-(CH₂)ⱼ-Y₁ darstellen; wobei R₅ und R₆ sich verbinden können, um eine Methylendioxygruppe zu bilden; j eine ganze Zahl von 1 bis 6 ist; und Y₁ ein Halogenatom, eine Aminogruppe, eine mit 1 oder 2 Thioethanolgruppen substituierte Aminogruppe, eine mit 1 oder 2 Allylgruppen substituierte Aminogruppe, ein Monoalkylaminorest mit 1 bis 10 Kohlenstoffatomen, ein Dialkylaminorest, in dem jede Alkyleinheit 1 bis 10 Kohlenstoffatome hat, ein mit 1 bis 3 Substituenten substituierter Imidazolrest, ein mit 1 bis 3 Substituenten substituierter Benzimidazolrest, eine Diazolgruppe, eine Triazolgruppe, eine Tetrazolgruppe, eine Piperidingruppe, eine Piperidinopiperidingruppe, ein Isonipecotinsäurerest oder ein Ester davon mit einem Alkylrest mit 1 bis 5 Kohlenstoffatomen, eine Thiazolidingruppe, eine Pyrrolidingruppe, eine Morpholingruppe, eine 4-Methylpiperazingruppe, eine Carboxylgruppe, ein Alkylester mit einer Carboxylgruppe, bei der die Alkyleinheit 1 bis 5 Kohlenstoffatome hat, oder eine Carboxyamidgruppe ist.

3. Allergorepressives Arzneimittel gegen Allergien vom Typ IV, das als wirksamen Bestandteil eine Verbindung der Formel (I) oder ein pharmazeutisch verträgliches Salz davon enthält: 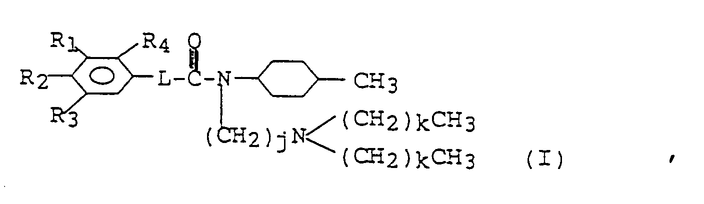 wobei L die Gruppe -(CH=CH)ₖ- oder -(CH₂)ₖ bezeichnet; j eine ganze Zahl von 1 bis 6 ist; k eine ganze Zahl von 0 bis 5 ist; R₁, R₂, R₃ und R₄ entweder gleich oder verschieden sind und jeweils ein Wasserstoffatom, einen Aminorest, einen Acylaminorest, einen Dialkylaminorest, bei dem jede Alkyleinheit 1 bis 5 Kohlenstoffatome hat, ein Halogenatom, eine Hydroxygruppe, eine Acetoxygruppe, einen Alkoxyrest mit 1 bis 3 Kohlenstoffatomen, eine Trifluormethylgruppe, eine Nitrogruppe, eine Tetrahydropyranyloxygruppe oder eine Benzyloxygruppe darstellen; und wobei R₁ und R₂ sich verbinden können, um eine Methylendioxygruppe zu bilden.

4. Allogorepressives Arzneimittel gegen Allergien vom Typ IV, das als wirksamen Bestandteil eine Verbindung der Formel (II) oder ein pharmazeutisch wirksames Salz davon enthält: 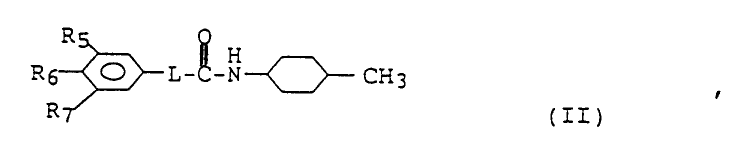 wobei L die Gruppe -(CH=CH)ₖ- oder -(CH₂)ₖ- bezeichnet; jedes k eine ganze Zahl von 0 bis 5 ist; R₅, R₆ und R₇ entweder gleich oder verschieden sind und jeweils ein Wasserstoffatom, eine Hydroxygruppe, eine Acetoxygruppe, einen Ester einer Aminosäure, in dem die Aminoeinheit durch eine Schutzgruppe geschützt sein kann, einen Alkoxyrest mit 1 bis 3 Kohlenstoffatomen, oder -O-(CH₂)ⱼ-Y₁ darstellen; R₅ und R₆ sich verbinden können, um eine Methylendioxygruppe zu bilden; j eine ganze Zahl von 1 bis 6 ist; und Y₁ ein Halogenatom, eine Aminogruppe, eine mit 1 oder 2 Thioethanolgruppen substituierte Aminogruppe, eine mit 1 oder 2 Allylgruppen substituierte Aminogruppe, ein Monoalkylaminorest mit 1 bis 10 Kohlenstoffatomen, ein Dialkylaminorest, in dem jede Alkyleinheit 1 bis 10 Kohlenstoffatome hat, ein mit 1 bis 3 Substituenten substituierter Imidazolrest, ein mit 1 bis 3 Substituenten substituierter Benzimidazolrest, eine Diazolgruppe, eine Triazolgruppe, eine Tetrazolgruppe, eine Piperidingruppe, eine Piperidinopiperidingruppe, ein Isonipecotinsäurerest oder ein Ester davon mit einem Alkylrest mit 1 bis 5 Kohlenstoffatomen, eine Thiazolidingruppe, eine Pyrrolidingruppe, eine Morpholingruppe, eine 4-Methylpiperazingruppe, eine Carboxylgruppe, ein Alkylester mit einer Carboxylgruppe, bei der die Alkyleinheit bis 5 Kohlenstoffatome hat, oder eine Carboxyamidgruppe ist.

## Revendications

1. Composé représenté par la formule (I) décrite ci-dessous ou sel de ce dernier pharmaceutiquement acceptable : 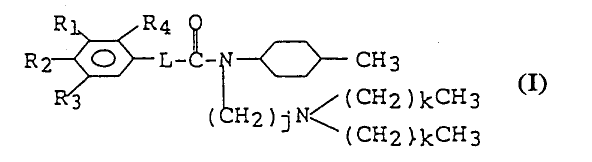 où L représente -(CH=CH)ₖ- ou -(CH₂)ₖ- ; j représente un nombre entier de 1 à 6; k représente un nombre entier de 0 à 5 ; R₁, R₂, R₃ et R₄ sont indentiques ou différents, et chacun représente un atome d'hydrogène, un groupe amino, acylamino, dialkylamino dans lequel chaque partie alkyle comporte de 1 à 5 atomes de carbone, un atome d'halogène, un groupe hydroxyle, acétoxy, alcoxy comportant de 1 à 3 atomes de carbone, trifluorométhyle, nitro, tétrahydropyranyloxy ou benzyloxy ; et R₁ et R₂ peuvent se combiner tous les deux pour former un groupe méthylènedioxy.

2. Composé représenté par la formule (II) décrite ci-dessous ou sel de ce dernier pharmaceutiquement acceptable : 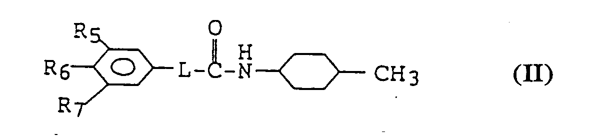 où L représente -(CH=CH)ₖ- ou -(CH₂)ₖ- ; chaque k représente un nombre entier de 0 à 5 ; R₅, R₆ et R₇ sont identiques ou différents, et chacun représente un atome d'hydrogène, un groupe hydroxyle, acétoxy, un groupe dérivé d'un ester d'acide aminé dans lequel la partie aminée peut être protégée par un groupe de protection, un groupe alcoxy qui comporte de 1 à 3 atomes de carbone, ou -O-(CH₂)ⱼ- Y₁ ; R₅ et R₆ peuvent se combiner tous les deux pour former un groupe méthylènedioxy ; j représente un nombre entier de 1 à 6 ; et Y₁ représente un atome d'halogène, un groupe amino substitué par 1 ou 2 groupes dérivés du thio-éthanol, un groupe amino substitué par 1 ou 2 groupes allyle, un groupe monoalkylamino qui comporte de 1 à 10 atomes de carbone, un groupe dialkylamino dans lequel chaque partie alkyle comporte de 1 à 10 atomes de carbone, un groupe dérivé de l'imidazole substitué par 1 à 3 substituants, un groupe dérivé du benzimidazole substitué par 1 à 3 substituants, un groupe dérivé du diazole, du triazole, du tétrazole, de la pipéridine, de la pipéridinopipéridine, de l'acide isonipécotique ou d'un ester de ce dernier avec un groupe alkyle qui comporte de 1 à 5 atomes de carbone, un groupe dérivé de la thiazolidine, de la pyrrolidine, de la morpholine, de la 4-méthylpipérazine, un groupe carboxyle, un groupe dérivé d'un alkylester d'un acide carboxylique dans lequel la partie alkyle comporte de 1 à 5 atomes de carbone ou un groupe carboxamide.

3. Composition pharmaceutique suppressive d'allergies de type IV contenant un composant efficace, un composé représenté par la formule (I) ou un sel de ce dernier pharmaceutiquement acceptable 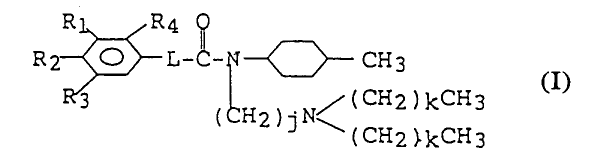 où L représente -(CH=CH)ₖ- ou -(CH₂)ₖ- ; j représente un nombre entier de 1 à 6 ; k représente un nombre entier de 0 à 5 ; R₁, R₂, R₃ et R₄ sont identiques ou différents, et chacun représente un atome d'hydrogène, un groupe amino, acylamino, dialkylamino dans lequel chaque partie alkyle comporte de 1 à 5 atomes de carbone, un atome d'halogène, un groupe hydroxyle, acétoxy, alcoxy comportant de 1 à 3 atomes de carbone, trifluorométhyle, nitro, tétrahydropyranyloxy ou benzyloxy ; et R₁ et R₂ peuvent se combiner tous les deux pour former un groupe méthylènedioxy.

4. Composition pharmaceutique suppressive d'allergies de type IV contenant un composant efficace, un composé représenté par la formule (II) ou un sel de ce dernier pharmaceutiquement acceptable 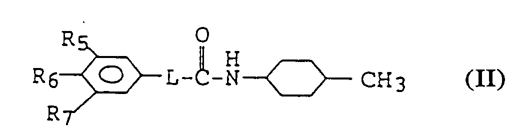 où L représente -(CH=CH)ₖ- ou -(CH₂)ₖ- ; chaque k représente un nombre entier de 0 à 5 ; R₅, R₆ et R₇ sont identiques ou différents, et chacun représente un atome d'hydrogène, un groupe hydroxyle, acétoxy, un groupe dérivé d'un ester d'acide aminé dans lequel la partie aminée peut être protégée par un groupe de protection, un groupe alcoxy qui comporte de 1 à 3 atomes de carbone, ou -O-(CH₂)ⱼ- Y₁ ; R₅ et R₆ peuvent se combiner tous les deux pour former un groupe méthylènedioxy ; j représente un nombre entier de 1 à 6 ; et Y₁ représente un atome d'halogène, un groupe amino substitué par 1 ou 2 groupes dérivés du thio-éthanol, un groupe amino substitué par 1 ou 2 groupes allyle, un groupe monoalkylamino qui comporte de 1 à 10 atomes de carbone, un groupe dialkylamino dans lequel chaque partie alkyle comporte de 1 à 10 atomes de carbone , un groupe dérivé de l'imidazole substitué par 1 à 3 substituants, un groupe dérivé du benzimidazole substitué par 1 à 3 substituants, un groupe dérivé du diazole, du triazole, du tétrazole, de la pipéridine, de la pipéridinopipéridine, de l'acide isonipécotique ou d'un ester de ce dernier avec un groupe alkyle qui comporte de 1 à 5 atomes de carbone, un groupe dérivé de la thiazolidine, de la pyrrolidine, de la morpholine, de la 4-méthylpipérazine, un groupe carboxyle, un groupe dérivé d'un alkylester d'un acide carboxylique dans lequel la partie alkyle comporte de 1 à 5 atomes de carbone ou un groupe carboxamide.
